# EUROPEAN PATENT SPECIFICATION

(11) **EP 1 423 195 B1**
(45) Date of publication and mention of the grant of the patent: **02.03.2016**
(21) Application number: 02791535.4
(22) Date of filing: 14.06.2002
(51) Int. Cl.: B01J 31/00, C07F 15/00, C08F 4/80, B01J 31/22

(54) **HEXACOORDINATED RUTHENIUM OR OSMIUM METAL CARBENE METATHESIS CATALYSTS**
SECHSFACH KOORDINIERTE RUTHENIUM- ODER OSMIUM-METALLCARBEN-METATHESEKATALYSATOREN
CATALYSEURS DE METATHESE DE CARBENE METALLIQUE DE RUTHENIUM OU D'OSMIUM HEXACOORDONNES

(30) Priority: 01.08.2001 US 309806 P; 24.08.2001 US 314978 P; 05.09.2001 US 948115; 14.12.2001 US 17489; 03.05.2002 US 138188; 25.03.2002 US 107531
(43) Date of publication of application: 02.06.2004
(73) Proprietor: CALIFORNIA INSTITUTE OF TECHNOLOGY, Pasadena, CA 91125 (US); Materia, Inc., Pasadena, CA 91107 (US)
(72) Inventor: GRUBBS, Robert, H., South Pasadena, CA (US); SANFORD, Melanie, S., Pasadena, CA 91106 (US); MOORE, Jason L., Huntsville, TX 77340 (US); LOVE, Jennifer, A., Pasadena, CA 91106 (US); TRNKA, Tina M., Pasadena, CA 91101 (US)
(74) Representative: Beck Greener
(86) International application number: PCT/US2002/019167
(87) International publication number: WO 2003/011455

(56) References cited:
- US-A- 6 077 805
- US-B1- 6 310 121
- US-B1- 6 417 363
- US-B1- 6 426 419
- DEL RIO I ET AL: "Ring-Opening Metathesis Polymerization of Norbornene Catalyzed by a Ru(II)-Vinylidene Complex" TETRAHEDRON LETTERS, ELSEVIER SCIENCE PUBLISHERS, AMSTERDAM, NL, vol. 40, no. 7, 12 February 1999 (1999-02-12), pages 1401-1404, XP004154644 ISSN: 0040-4039
- WERNER, HELMUT ET AL: "Vinylidene transition-metal complexes. XXXV. The supporting role of phosphino ester ligands for the synthesis of neutral carbene, vinylidene and allenylidene ruthenium(II) complexes" CHEMISCHE BERICHTE , 128(1), 49-62 CODEN: CHBEAM; ISSN: 0009-2940, 1995, XP002326940
- JAFARPOUR L ET AL: "Development of olefin metathesis catalyst precursors bearing nucleophilic carbene ligands" JOURNAL OF ORGANOMETALLIC CHEMISTRY, ELSEVIER-SEQUOIA S.A. LAUSANNE, CH, vol. 617-618, 15 January 2001 (2001-01-15), pages 17-27, XP004315024 ISSN: 0022-328X

## Description

The U.S. Government has certain rights in this invention pursuant to Grant No. CHE-9809856 awarded by the National Science Foundation.

### BACKGROUND

Metathesis catalysts have been previously described by for example, United States Patents Nos. 5,312,940, 5,342,909, 5,728,917, 5,750,815, 5,710,298, and 5,831,108 and PCT Publications WO 97/20865 and WO 97/29135. These publications describe well-defined single component ruthenium or osmium catalysts that possess several advantageous properties. For example, these catalysts are tolerant to a variety of functional groups and generally are more active than previously known metathesis catalysts. Recently, the inclusion of an N-heterocyclic carbene (NHC) ligand, such as an imidazolidine or triazolylidene ligand as described in U.S. Application No. 09/539,840 (see US-A-2002013473), U.S. Application No 09/576,370 (see US-A-7,329,758) and PCT Publication No. WO 99/51344, in these metal-carbene complexes has been found to improve the already advantageous properties of these catalysts. In an unexpected and surprising result, the shift in structure from the well-established penta-coordinated catalyst structure to the hexacoordinated catalyst structure has been found to significantly improve the properties of the catalyst. For example, these hexacoordinated catalysts of the present invention exhibit increased activity and selectivity not only in ring closing metathesis ("RCM") reactions, but also in other metathesis reactions including cross metathesis ("CM") reactions, reactions of acyclic olefins, and ring opening metathesis polymerization ("ROMP") reactions.

### SUMMARY

The present invention relates to novel hexacoordinated metathesis catalysts and to methods for making and using the same. The inventive catalysts are of the formula 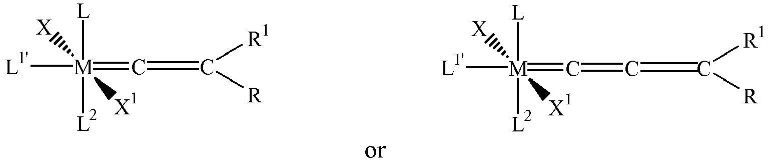 wherein:
M is ruthenium or osmium;
X and X¹ are the same or different and are each independently an anionic ligand;
L, L^{1'} and L² are the same or different and are each independently a neutral electron donor ligand, wherein at least one is an N-heterocyclic carbene (NHC) ligand; and,
R and R¹ are each independently hydrogen or a substituent selected from the group consisting of C₁-C₂₀ alkyl, C₂-C₂₀ alkenyl, C₂-C₂₀ alkynyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl, C₁-C₂₀ alkylsulfinyl, and silyl. Optionally, each of the R or R¹ substituent group may be substituted with one or more moieties selected from the group consisting of C₁-C₁₀ alkyl, C₁-C₁₀ alkoxy, and aryl which in turn may each be further substituted with one or more groups selected from a halogen, a C₁-C₅ alkyl, C₁-C₅ alkoxy, and phenyl. Moreover, any of the catalyst ligands may further include one or more functional groups. Examples of suitable functional groups include but are not limited to: hydroxyl, thiol, thioether, ketone, aldehyde, ester, ether, amine, imine, amide, nitro, carboxylic acid, disulfide, carbonate, isocyanate, carbodiimide, carboalkoxy, carbamate, halogen, alcohol, sulfonic acid, phosphine, imide, acetal, ketal, boronate, cyano, cyanohydrin, hydrazine, enamine, sulfone, sulfide, and sulfenyl.

In preferred embodiments, L² and L^{1'} are pyridine and L is an N-heterocyclic carbene ligand. Examples of N-heterocyclic carbene ligands include: 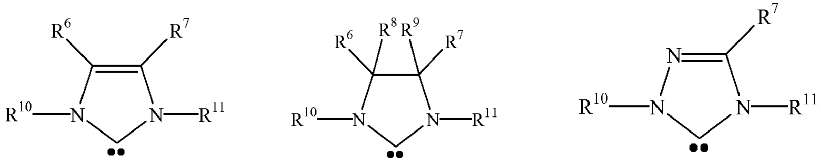 wherein R, R¹ R⁶, R⁷, R⁸, R⁹, R¹⁰ and R¹¹ are each independently hydrogen or a substituent selected from the group consisting of C₁-C₂₀ alkyl, C₂-C₂₀ alkenyl, C₂-C₂₀ alkynyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl, C₁-C₂₀ alkylsulfinyl and silyl. Optionally, each of the R, R¹ R⁶, R⁷, R⁸, R⁹, R¹⁰ and R¹¹ substituent group may be substituted with one or more moieties selected from the group consisting of C₁-C₁₀ alkyl, C₁-C₁₀ alkoxy, and aryl which in turn may each be further substituted with one or more groups selected from a halogen, a C₁-C₅ alkyl, C₁-C₅ alkoxy, and phenyl. Moreover, any of the catalyst ligands may further include one or more functional groups. Examples of suitable functional groups include but are not limited to: hydroxyl, thiol, thioether, ketone, aldehyde, ester, ether, amine, imine, amide, nitro, carboxylic acid, disulfide, carbonate, isocyanate, carbodiimide, carboalkoxy, carbamate, halogen, alcohol, sulfonic acid, phosphine, imide, acetal, ketal, boronate, cyano, cyanohydrin, hydrazine, enamine, sulfone, sulfide, and sulfenyl. The inclusion of an NHC ligand to the hexacoordinated ruthenium or osmium catalysts has been found to dramatically improve the properties of these complexes. Because the NHC-based hexacoordinated complexes are extremely active, the amount of catalysts that is required is significantly reduced.

### DETAILED DESCRIPTION OF THE PREFERRED EMBODIMENTS

The present invention generally relates to ruthenium and osmium carbene catalysts for use in olefin metathesis reactions. More particularly, the present invention relates to hexacoordinated ruthenium and osmium carbene catalysts and to methods for making and using the same. The terms "catalyst" and "complex" herein are used interchangeably.

Unmodified ruthenium and osmium carbene complexes have been described in United States Patents Nos. 5,312,940, 5,342,909, 5,728,917, 5,750,815, and 5,710,298. The ruthenium and osmium carbene complexes disclosed in these patents all possess metal centers that are formally in the +2 oxidation state, have an electron count of 16, and are penta-coordinated. These catalysts are of the general formula A: 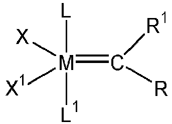 wherein:
M is ruthenium or osmium;
X and X¹ are each independently any anionic ligand;
L and L¹ are each independently any neutral electron donor ligand;
R and R¹ are the same or different and are each independently hydrogen or a substituent selected from the group consisting of C₁-C₂₀ alkyl, C₂-C₂₀ alkenyl, C₂-C₂₀ alkynyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl, C₁-C₂₀ alkylsulfinyl, and silyl. Optionally, each of the R or R¹ substituent group may be substituted with one or more moieties selected from the group consisting of C₁-C₁₀ alkyl, C₁-C₁₀ alkoxy, and aryl which in turn may each be further substituted with one or more groups selected from a halogen, a C₁-C₅ alkyl, C₁-C₅ alkoxy, and phenyl. Moreover, any of the catalyst ligands may further include one or more functional groups. Examples of suitable functional groups include but are not limited to: hydroxyl, thiol, thioether, ketone, aldehyde, ester, ether, amine, imine, amide, nitro, carboxylic acid, disulfide, carbonate, isocyanate, carbodiimide, carboalkoxy, carbamate, halogen, alcohol, sulfonic acid, phosphine, imide, acetal, ketal, boronate, cyano, cyanohydrin, hydrazine, enamine, sulfone, sulfide, and sulfenyl.

Werner et al (Chem. Ber. 1995, 128, 49-62) discloses various ruthenium(II) complexes of the formulae B or C 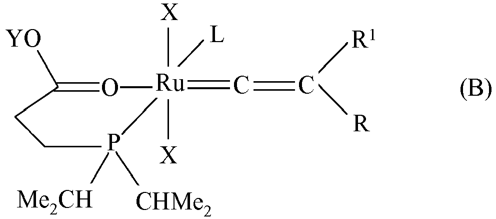 in which
L is P(CH(Me)₂)₂ (CH₂-CO₂Me), P(CH(Me)₂)₂ (CH₂-CO₂Et), or PPh₃,
R is H or Ph,
R¹ is H, Me, CMe₃, Ph, Ph-C≡CH, C(Ph₂)OH, or C(Ph)(o-Tol)OH, or
R and R1 together represent =CPh₂ or =C(Ph)(o-Tol),
X is Br, Cl or I, and
Y is Me or Et; 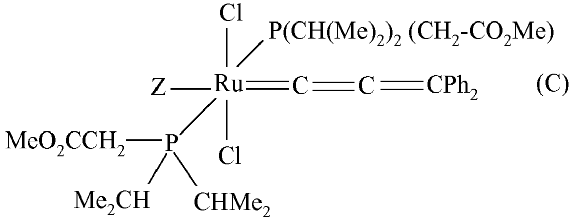 in which Z is CO, CNCMe₃ or pyridine.

del Río et al (Tetrahedron Letters 40 (1999) 1401-1404) discloses ring-opening metathesis polymerization of norbornene catalyzed by the Ru(II)-vinylidene complex [Ru(=C=CHPh)(NN'N)(PPh₃)][BF₄]₂ (1) and refers to investigation of *cis*,*mer*-[Ru(=C=CHPh)Cl₂(NN'N] (2), *mer*-[Ru(=C=CHPh)Cl(NN'N][PPh₃][BF₄] (3), and *mer*-[Ru(=C=CHPh)(OTf)(NN'N][PPh₃][OTf] (3) (OTf = trifluoromethane sulfonate) as catalyst precursors. It is reported that 2 appeared to be inactive.

EP 1130025 discloses catalysts of Formula A *supra* in which M is Os or Ru;
R and R¹ are independently selected from hydrogen, substituted or unsubstituted alkyl, and substituted or unsubstituted aryl; X and X¹ are independently selected from any anionic ligand; and L and L¹ are independently selected from any neutral electron donor.

WO 99/29701 (corresponding to US 6417363) discloses catalysts of the formula D or C 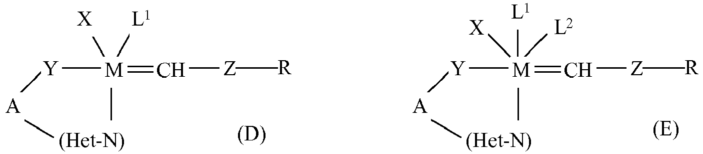 wherein
M is ruthenium or osmium;
L¹ and L² are neutral ligands having electron donor properties;
X is an anionic ligand;
Y is oxygen or sulfur;
A is a direct bond, C₁₋₄ alkylene or C₂₋₄ alkylidene;
Z is a direct bond, oxygen, sulfur or the groups -N(R₁)- or -P(R₂)-wherein R₁ and
R₂ are hydrogen or hydrocarbon radicals;
R is a hydrocarbon radical; and
Het-N is mono- or bi-cyclic, aromatic heterocyclyl having at least one nitrogen atom that is coordinated with M;
and isomers thereof.

The catalysts of the present invention are similar to those of formula A *supra* in that they are Ru or Os complexes; however, in these complexes, the metal is formally in the +2 oxidation state, and has an electron count of 18 and are hexacoordinated. These catalysts are of the general formula: 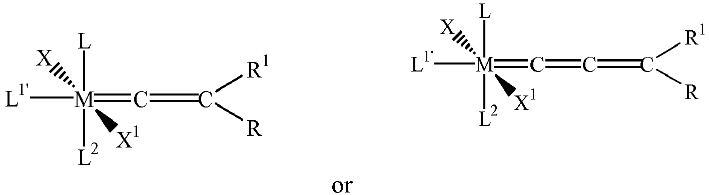 wherein
M is ruthenium or osmium;
X and X¹ are the same or different and are each independently any anionic ligand;
L, L^{1'}, and L² are the same or different and are each independently any neutral electron donor ligand, wherein at least one is an NHC ligand;
R and R¹ are the same or different and are each independently hydrogen or a substituent selected from the group consisting of C₁-C₂₀ alkyl, C₂-C₂₀ alkenyl, C₂-C₂₀ alkynyl, aryl, C₁-C₂₀ carboxylate, C₁-C₂₀ alkoxy, C₂-C₂₀ alkenyloxy, C₀-C₂₀ alkynyloxy, aryloxy, C₂-C₂₀ alkoxycarbonyl, C₁-C₂₀ alkylthio, C₁-C₂₀ alkylsulfonyl, C₁-C₂₀ alkylsulfinyl, and silyl. Optionally, each of the R or R¹ substituent group may be substituted with one or more moieties selected from the group consisting of C₁-C₁₀ alkyl, C₁-C₁₀ alkoxy, and aryl which in turn may each be further substituted with one or more groups selected from a halogen, a C₁-C₅ alkyl, C₁-C₅ alkoxy, and phenyl. Moreover, any of the catalyst ligands may further include one or more functional groups. Examples of suitable functional groups include but are not limited to: hydroxyl, thiol, thioether, ketone, aldehyde, ester, ether, amine, imine, amide, nitro, carboxylic acid, disulfide, carbonate, isocyanate, carbodiimide, carboalkoxy, carbamate, halogen, alcohol, sulfonic acid, phosphine, imide, acetal, ketal, boronate, cyano, cyanohydrin, hydrazine, enamine, sulfone, sulfide, and sulfenyl.

The hexacoordinated complex provides several advantages over the well-known pentacoordinated complexes. For example, the hexacoordinated complexes have greater air stability in the solid state because they are coordinatively saturated. Due to the lability of the additional ligand, e.g. pyridines, the complexes initiate faster than the phosphine based pentacoordinated species. Slow initiation means that only a small amount of complex is actually catalytically active thereby wasting much of the added complex. With faster initiators, catalyst loading is lowered. Further, and without being bound by theory, it is believed that the slower propagation of the hexacoordinated complexes, due to the re-binding of the labile ligands relative to the phosphines, translates to lower polydispersity. Moreover, the coordinatively saturated species crystallize better than their pentacoordinated counterparts. In addition, due to the lability of the ligands in the hexacoordinated complexes (e.g. pyridines and chlorines), these complexes allow access to previously inaccessible complexes and provide with higher purity certain complexes that can be obtained through different routes. For example, the pentacoordinated benzylidene with triphenylphosphine as its phosphine ligand can be prepared in higher yield and with greater purity using the hexacoordinated complex. The pentacoordinated benzylidene with P(*p*-CF₃C₆H₄)₃ as its phosphine ligand is inaccessible through existing routes. Without being bound by theory, it is believe that this is because it would require the substitution of a stronger donor ligand with a weaker donor ligand. Substitution of the anionic ligands of the hexacoordinated complexes is much more rapid than with the corresponding pentacoordinated species (e.g. phosphine bound). Without being bound by theory, it is believed that this results from the requirement of ligand dissociation before anionic ligand substitution. Thus complexes with fast dissociation of their neutral electron donor ligands will undergo faster substitution.

The catalysts of the invention are also useful for ring-opening metathesis polymerization (ROMP), ring-closing metathesis (RCM), acyclic diene metathesis polymerization ("ADMET"), and cross-metathesis. The synthesis and polymerization of olefins via these metathesis reactions can be found in, for example, U.S. Application Nos. 09/891,144 entitled: "Synthesis of Functionalized and Unfunctionalized Olefins, filed June 25, 2001 (see US-A-2002137978), and U.S. Application No. 09/491,800 (see U.S. Patent No. 6306988).

In preferred embodiments of the inventive catalysts, the R substituent is hydrogen and the R¹ substituent is selected from the group consisting of C₁-C₂₀ alkyl, C₂-C₂₀ alkenyl, and aryl. In even more preferred embodiments, the R¹ substituent is phenyl or vinyl, optionally substituted with one or more moieties selected from the group consisting of C₁-C₅ alkyl, C₁-C₅ alkoxy, phenyl, and a functional group. In especially preferred embodiments, R¹ is phenyl or vinyl substituted with one or more moieties selected from the group consisting of chloride, bromide, iodide, fluoride, -NO₂, -NMe₂, methyl, methoxy and phenyl. In the most preferred embodiments, the R¹ substituent is phenyl or - C=C(CH₃)₂.

In preferred embodiments of the inventive catalysts, X and X¹ are each independently hydrogen, halide, or one of the following groups: C₁-C₂₀ alkyl, aryl, C₁-C₂₀ alkoxide, aryloxide, C₃-C₂₀ alkyldiketonate, aryldiketonate, C₁-C₂₀ carboxylate, arylsulfonate, C₁-C₂₀ alkylsulfonate, C₁-C₂₀ alkylthio, C₁-C₂₀ alkylsulfonyl, or C₁-C₂₀ alkylsulfinyl. Optionally, X and X¹ may be substituted with one or more moieties selected from the group consisting of C₁-C₁₀ alkyl, C₁-C₁₀ alkoxy, and aryl which in turn may each be further substituted with one or more groups selected from halogen, C₁-C₅ alkyl, C₁-C₅ alkoxy, and phenyl. In more preferred embodiments, X and X¹ are halide, benzoate, C₁-C₅ carboxylate, C₁-C₅ alkyl, phenoxy, C₁-C₅ alkoxy, C₁-C₅ alkylthio, aryl, and C₁-C₅ alkyl sulfonate. In even more preferred embodiments, X and X¹ are each halide, CF₃CO₂, CH₃CO₂, CFH₂CO₂, (CH₃)₃CO, (CF₃)₂(CH₃)CO, (CF₃)(CH₃)₂CO, PhO, MeO, EtO, tosylate, mesylate, or trifluoromethanesulfonate. In the most preferred embodiments, X and X¹ are each chloride.

L, L^{1'} and L² may be any appropriate monodentate or multidentate neutral electron donor ligands but at least one is an NHC ligand. Multidentate neutral electron donor ligands include bidentate, tridentate, or tetradentate neutral electron donor ligands, for example. In preferred embodiments of the inventive catalysts, L, L^{1'} and L² are each independently selected from the group consisting of phosphine, sulfonated phosphine, phosphite, phosphinite, phosphonite, arsine, stibine, ether, amine, amide, imine, sulfoxide, carboxyl, nitrosyl, pyridine, and thioether, or any derivatives therefrom. At least one L, L^{1'} and L² is an N-heterocyclic carbene ligand. Preferred embodiments include complexes where both L^{1'} and L² are either the same or different NHC ligands.

In preferred embodiments, at least one of L, L¹, L^{1'} and L² is a phosphine of the formula PR³R⁴R⁵, where R³, R⁴, and R⁵ are each independently aryl or C₁-C₁₀ alkyl, particularly primary alkyl, secondary alkyl or cycloalkyl. In the even more preferred embodiments, at least one of L, L¹, L^{1'} and L² is each selected from the group consisting of-P(cyclohexyl)₃, -P(cyclopentyl)₃, -P(isopropyl)₃, and -P(phenyl)₃. At least one of L, L¹, L^{1'} and L² is an NHC ligand. A preferred embodiment include where L is an NHC, and L^{1'} and L² are each heterocyclic ligands optionally aromatic, or together form a bidenatate ligand. Preferably L^{1'} and L² are each independently pyridine or a pyridine derivative.

Examples of NHC ligands include ligands of the general formulas: 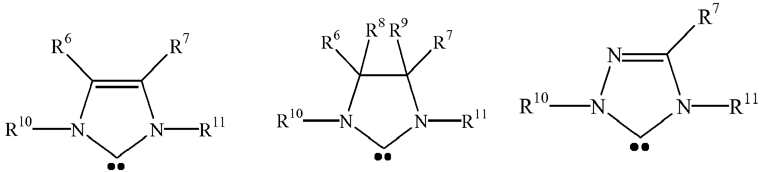 wherein R, R¹, R⁶, R⁷, R⁸, R⁹, R¹⁰, and R¹¹ are each independently hydrogen or a substituent selected from the group consisting of C₁-C₂₀ alkyl, C₂-C₂₀ alkenyl, C₂-C₂₀ alkynyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl, C₁-C₂₀ alkylsulfinyl, and silyl. Optionally, each of the R, R¹ R⁶, R⁷, R⁸, R⁹, R¹⁰, and R¹¹ substituent group may be substituted with one or more moieties selected from the group consisting of C₁-C₁₀ alkyl, C₁-C₁₀ alkoxy, and aryl which in turn may each be further substituted with one or more groups selected from a halogen, a C₁-C₅ alkyl, C₁-C₅ alkoxy, and phenyl. Moreover, any of the catalyst ligands may further include one or more functional groups. Examples of suitable functional groups include but are not limited to: hydroxyl, thiol, thioether, ketone, aldehyde, ester, ether, amine, imine, amide, nitro, carboxylic acid, disulfide, carbonate, isocyanate, carbodiimide, carboalkoxy, carbamate, halogen, alcohol, sulfonic acid, phosphine, imide, acetal, ketal, boronate, cyano, cyanohydrin, hydrazine, enamine, sulfone, sulfide, and sulfenyl.

In preferred embodiments, R⁶, R⁷, R⁸ and R⁹ are independently selected from the group consisting of hydrogen, phenyl, or together form a cycloalkyl or an aryl optionally substituted with one or more moieties selected from the group consisting of C₁-C₁₀ alkyl, C₁-C₁₀ alkoxy, aryl, and a functional group selected from the group consisting of hydroxyl, thiol, thioether, ketone, aldehyde, ester, ether, amine, imine, amide, nitro, carboxylic acid, disulfide, carbonate, isocyanate, carbodiimide, carboalkoxy, carbamate, and halogen; and R¹⁰ and R¹¹ are each is independently C₁-C₁₀ alkyl or aryl optionally substituted with C₁-C₅ alkyl, C₁-C₅ alkoxy, aryl, and a functional group selected from the group consisting of hydroxyl, thiol, thioether, ketone, aldehyde, ester, ether, amine, imine, amide, nitro, carboxylic acid, disulfide, carbonate, isocyanate, carbodiimide, carboalkoxy, carbamate, halogen, alcohol, sulfonic acid, phosphine, imide, acetal, ketal, boronate, cyano, cyanohydrin, hydrazine, enamine, sulfone, sulfide, and sulfenyl.

In more preferred embodiments, R⁶ and R⁷ are both hydrogen or phenyl, or R⁶ and R⁷ together form a cycloalkyl group; R⁸ and R⁹ are hydrogen and R¹⁰ and R¹¹ are each either substituted or unsubstituted aryl. Without being bound by theory, it is believed that bulkier R¹⁰ and R¹¹ groups result in catalysts with improved characteristics such as thermal stability. In especially preferred embodiments, R¹⁰ and R¹¹ are the same and each is independently of the formula: 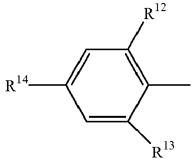 wherein:
R¹², R¹³, and R¹⁴ are each independently hydrogen, C₁-C₁₀ alkyl, C₁-C₁₀ alkoxy, aryl, or a functional group selected from hydroxyl, thiol, thioether, ketone, aldehyde, ester, ether, amine, imine, amide, nitro, carboxylic acid, disulfide, carbonate, isocyanate, carbodiimide, carboalkoxy, carbamate, and halogen. In especially preferred embodiments, R¹², R¹³, and R¹⁴ are each independently selected from the group consisting of hydrogen, methyl, ethyl, propyl, isopropyl, hydroxyl, and halogen. In the most preferred embodiments, R¹², R¹³, and R¹⁴ are the same and are each methyl.

In these complexes, R⁶, R⁷, R⁸, and R⁹ are each independently hydrogen or a substituent selected from the group consisting of C₁-C₂₀ alkyl, C₂-C₂₀ alkenyl, C₂-C₂₀ alkynyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl and C₁-C₂₀ alkylsulfinyl. Imidazolidine ligands are also referred to as imidizol-2-ylidene ligands.

Other examples of neutral electron donor ligands include ligands which are derived, for example, from unsubstituted or substituted heteroarenes such as furan, thiophene, pyrrole, pyridine, bipyridine, picolylimine, gamma-pyran, gamma-thiopyran, phenanthroline, pyrimidine, bipyrimidine, pyrazine, indole, coumarone, thionaphthene, carbazole, dibenzofuran, dibenzothiophene, pyrazole, imidazole, benzimidazole, oxazole, thiazole, dithiazole, isoxazole, isothiazole, quinoline, bisquinoline, isoquinoline, bisisoquinoline, acridine, chromene, phenazine, phenoxazine, phenothiazine, triazine, thianthrene, purine, bisimidazole and bisoxazole.

Examples of substituents are OH, halogen, C(O)ORₛ₁, OC(O)Rₛ₄, C(O)Rₛ₂, nitro, NH₂, cyano, SO₃M¹_{y}, OSO₃M¹_{y}, NR₂₀SO₃M¹_{y}, N=N-Rₛ₂, C₁-C₁₂ alkyl, C₂-C₁₂ alkenyl, C₁-C₁₂ alkoxy, C₃-C₁₂ cycloalkyl, C₃-C₁₂ cycloalkenyl, C₂-C₁₁ heterocycloalkyl, C₂-C₁₁ heterocycloalkenyl, C₆-C₁₀ aryl, C₆-C₁₀ aryloxy, C₅-C₉ heteroaryl, C₅-C₉ heteroaryloxy, C₇-C₁₁ aralkyl, C₇-C₁₁ aralkyloxy, C₆-C₁₀ heteroaralkyl, C₈-C₁₁ aralkenyl, C₇-C₁₀ heteroaralkenyl, monoamino, diamino, sulfonyl, sulfonamide, carbamide, carbamate, sulfohydrazide, carbohydrazide, carbohydroxamic acid residue and aminocarbonylamide, in which Rₛ₁ is hydrogen, M¹_{y}, C₁-C₁₂ alkyl, C₂-C₁₂ alkenyl, C₃-C₁₂ cycloalkyl, C₂-C₁₁ heterocycloalkyl, C₆-C₁₀ aryl, C₅-C₉ heteroaryl, C₇-C₁₁ aralkyl or C₆-C₁₀ heteroaralkyl, Rₛ₄ is hydrogen, C₁-C₁₂ alkyl, C₂-C₁₂ alkenyl, C₃-C₁₂ cycloalkyl, C₂-C₁₁ heterocycloalkyl, C₁₆-C₁₀ aryl, C₅-C₁₉ heteroaryl, C₇-C₁₁ aralkyl or C₆-C₁₀ heteroaralkyl, and Rₛ₂ and Rₛ₂₀ are hydrogen, C₁-C₁₂ alkyl, C₂-C₁₂ alkenyl, C₃-C₁₂ cycloalkyl, C₃-C₁₂ cycloalkenyl, C₂-C₁₁ heterocycloalkyl, C₁-C₁₁ heterocycloalkenyl, C₆-C₁₀ aryl, C₅-C₉ heteroaryl, C₇-C₁₁ aralkyl, C₆-C₁₀ heteroaralkyl, C₈-C₁₁ aralkenyl or C₇-C₁₀ heteroaralkenyl, and alkyl, alkenyl, alkoxy, cycloalkyl, cycloalkenyl, heterocycloalkyl, heterocycloalkenyl, aryl, aryloxy, heteroaryl, heteroaryloxy, aralkyl, aralkyloxy, heteroaralkyl, aralkenyl and heteroaralkenyl in turn are unsubstituted or substituted by one of the above-mentioned substituents; and y is 1 and M¹ is a monovalent metal or y is 1/2 and M¹ is a divalent metal.

In the context of the description of the present invention, the terms metal and corresponding cations refer to an alkali metal, for example Li, Na or K, an alkaline earth metal, for example Mg, Ca or Sr, or Mn, Fe, Zn or Ag, and corresponding cations. Lithium, sodium and potassium ions, with their salts, are preferred. NH₂, monoamino, diamino, carbamide, carbamate, carbohydrazide, sulfonamide, sulfohydrazide and aminocarbonylamide correspond preferably to a group R₈ C(O)(NH)ₚ N(R₉)--,-C(O)(NH)ₚ NR₈R₉, R₈OC(O)(NH)ₚN(R₉)--, R₈R₄₀NC(O)(NH)ₚN(R₉)--, --OC(O)(NH)ₚ NR₈R₉, --N(R₄₀)C(O)(NH)ₚNR₈R₉, R₈S(O)₂(NH)ₚN(R₉)--; --S(O)₂(NH)ₚNR₈R₉; R₈R₄₀NS(O)₂N(R₉)-- or --NR₄₀S(O)₂NR₈R₉, in which R₈, R₉ and R₄₀ independently of one another are hydrogen, OH, C₁-C₁₂ alkyl, C₁-C₁₂ alkenyl, C₃-C₁₂ cycloalkyl, C₃-C₁₂ cycloalkenyl, C₂-C₁₁ heterocycloalkyl, C₂-C₁₁ heterocycloalkenyl, C₆-C₁₀ aryl, C₅-C₉ heteroaryl, C₇-C₁₆ aralkyl, C₈-C₁₆ aralkenyl with C₂-C₆ alkenylene and C₆-C₁₀ aryl, C₆-C₁₅ heteroaralkyl, C₆-C₁₅ heteroaralkenyl, or di-C₆-C₁₀ aryl-C₁-C₆ alkyl, or R_{8'} R_{9'}N, in which R_{8'} and R_{9'} independently of one another are hydrogen, OH, SO₃M_{y}, OSO₃M_{y}, C₁-C₁₂ alkyl, C₃-C₁₂ cycloalkyl, C₂-C₁₁ heterocycloalkyl, C₆-C₁₀ aryl, C₅-C₉ heteroaryl, C₇-C₁₁ aralkyl, C₆-C₁₀ heteroaralkyl, C₈-C₁₆ aralkenyl with C₂-C₆ alkenylene and C₆-C₁₀ aryl, or di-C₆-C₁₀ aryl-C₁-C₆ alkyl, which are unsubstituted or substituted by one or more substituents from the group consisting of OH, halogen, C(O)ORₛ₁, OC(O)Rₛ₄, C(O)Rₛ₂, nitro, NH₂, cyano, SO₃Z_{y}, OSO₃Z_{y}, NR₂₀SO₃Z_{y}, C₁-C₁₂ alkyl, C₂-C₁₂ alkenyl, C₁-C₁₂ alkoxy, C₃-C₁₂ cycloalkyl, C₃-C₁₂ cycloalkenyl, C₂-C₁₁ heterocycloalkyl, C₂-C₁₁ heterocycloalkenyl, C₆-C₁₀ aryl, C₆-C₁₀ aryloxy, C₅-C₉ heteroaryl, C₅-C₉ heteroaryloxy, C₇-C₁₁ aralkyl, C₇-C₁₁ aralkyloxy, C₆-C₁₀ heteroaralkyl, C₈-C₁₁ aralkenyl, C₇-C₁₀ heteroaralkenyl, monoamino, diamino, sulfonyl, sulfonamide, carbamide, carbamate, sulfohydrazide, carbohydrazide, carbohydroxamic acid residue and aminocarbonylamide, in which Rₛ₁ is hydrogen, Z_{y}, C₁-C₁₂ alkyl, C₂-C₁₂ alkenyl, C₃-C₁₂ cycloalkyl, C₂-C₁₁ heterocycloalkyl, C₆-C₁₀ aryl, C₅-C₉ heteroaryl, C₇-C₁₁ aralkyl or C₆-C₁₀ heteroaralkyl, Rₛ₄ is hydrogen, C₁-C₁₂ alkyl, C₂-C₁₂ alkenyl, C₃-C₁₂ cycloalkyl, C₂-C₁₁ heterocycloalkyl, C₆-C₁₀ aryl, C₅-C₉ heteroaryl, C₇-C₁₁ aralkyl or C₆-C₁₀ heteroaralkyl, and Rₛ₂ is hydrogen, C₁-C₁₂ alkyl, C₂-C₁₂ alkenyl, C₃-C₁₂ cycloalkyl, C₃-C₁₂ cycloalkenyl, C₂-C₁₁ heterocycloalkyl, C₂-C₁₁ heterocycloalkenyl, C₆-C₁₀ aryl, C₅-C₉ heteroaryl, C₇-C₁₁ aralkyl, C₆-C₁₀ heteroaralkyl, C₈-C₁₁ aralkenyl or C₇-C₁₀ heteroaralkenyl, and alkyl, alkenyl, alkoxy, cycloalkyl, cycloalkenyl, heterocycloalkyl, heterocycloalkenyl, aryl, aryloxy, heteroaryl, heteroaryloxy, aralkyl, aralkyloxy, heteroaralkyl, aralkenyl and heteroaralkenyl in turn are unsubstituted or substituted by one of the above-mentioned substituents; p is 0 or 1 and y is 1 and Z is a monovalent metal or y is 1/2 and Z is a divalent metal; or R₈ and R₉ or R_{8'} and R_{9'} or R₈ and R₄₀ in the case of --NR₈R₉ or --NR_{8'}R_{9'} or R₈R₄₀N-- together are tetramethylene, pentamethylene, --(CH₂)₂ --O--(CH₂)₂ --, --(CH₂)₂--S--(CH₂)₂-- or --(CH₂)₂--NR₇-(CH₂)₂--, and R₇ is H, C₁-C₆ alkyl, C₇-C₁₁ aralkyl, C(O)Rₛ₂ or sulfonyl.

The sulfonyl substituent is, for example, of the formula R₁₀--SO₂-- in which R₁₀ is C₁-C₁₂ alkyl, C₃-C₁₂ cycloalkyl, C₂-C₁₁ heterocycloalkyl, C₆-C₁₀ aryl, C₅-C₉ heteroaryl, C₇-C₁₁ aralkyl or C₆-C₁₀ heteroaralkyl which are unsubstituted or substituted by one or more substituents selected from the group consisting of OH, halogen, C(O)ORₛ₁. OC(O)Rₛ₄, C(O)Rₛ₂, nitro, NH₂, cyano, SO₃Z_{y}, OSO₃Z_{y}, NR₂₀SO₃Z_{y}, C₁-C₁₂ alkyl, C₂-C₁₂ alkenyl, C₁-C₁₂ alkoxy, C₃-C₁₂ cycloalkyl, C₃-C₁₂ cycloalkenyl, C₂-C₁₁ heterocycloalkyl, C₂-C₁₁ heterocycloalkenyl, C₆-C₁₀ aryl, C₆-C₁₀ aryloxy, C₅-C₉ heteroaryl, C₅-C₉ heteroaryloxy, C₇-C₁₁ aralkyl, C₆-C₁₀ heteroaralkyl, C₈-C₁₁ aralkenyl, C₇-C₁₀ heteroaralkenyl, monoamino, diamino, sulfonyl, sulfonamide, carbamide, carbamate, sulfonhydrazide, carbohydrazide, carbohydroxamic acid residue and aminocarbonylamide, in which Rₛ₁ is hydrogen, Z_{y}, C₁-C₁₂ alkyl, C₂-C₁₂ alkenyl, C₃-C₁₂ cycloalkyl, C₂-C₁₁ heterocycloalkyl, C₆-C₁₀ aryl, C₅-C₉ heteroaryl, C₇-C₁₁ aralkyl or C₆-C₁₀ heteroaralkyl, Rₛ₄ is hydrogen, C₁-C₁₂ alkyl, C₂-C₁₂ alkenyl, C₃-C₁₂ cycloalkyl, C₂-C₁₁ heterocycloalkyl, C₆-C₁₀ aryl, C₅-C₉ heteroaryl, C₇-C₁₁ aralkyl or C₆-C₁₀ heteroaralkyl, and Rₛ₂ and R₂₀ are hydrogen, C₁-C₁₂ alkyl, C₂-C₁₂ alkenyl, C₃-C₁₂ cycloalkyl, C₃-C₁₂ cycloalkenyl, C₂-C₁₁ heterocycloalkyl, C₂-C₁₁ heterocycloalkenyl, C₆-C₁₀ aryl, C₅-C₉ heteroaryl, C₇-C₁₁ aralkyl, C₆-C₁₀ heteroaralkyl, C₈-C₁₁ aralkenyl or C₇-C₁₀ heteroaralkenyl, and alkyl, alkenyl, alkoxy, cycloalkyl, cycloalkenyl, heterocycloalkyl, heterocycloalkenyl, aryl, aryloxy, heteroaryl, heteroaryloxy, aralkyl, heteroaralkyl, aralkenyl and heteroaralkenyl in turn are unsubstituted or substituted by one of the above-mentioned substituents; and y is 1 and Z is a monovalent metal or y is 1/2 and Z is a divalent metal.

Preferred neutral electron donor ligands are derived, for example, from heteroarenes of the group

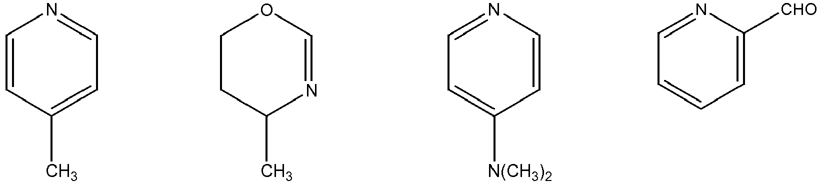

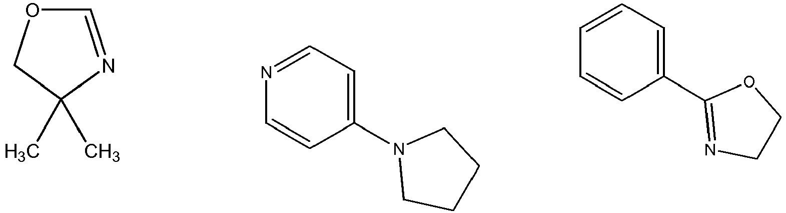

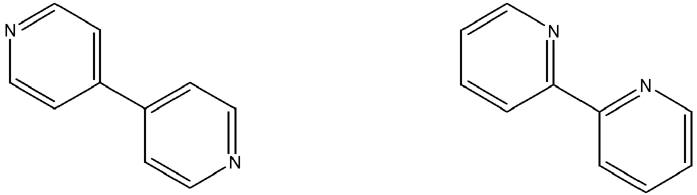

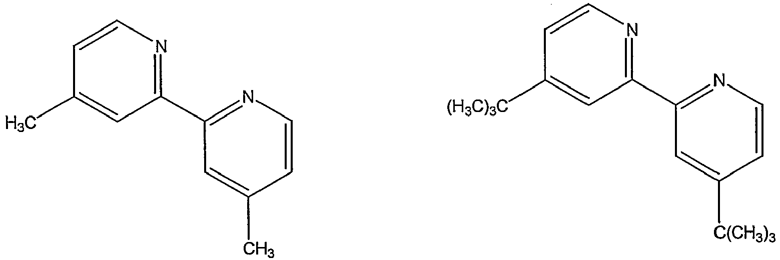

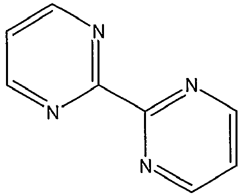

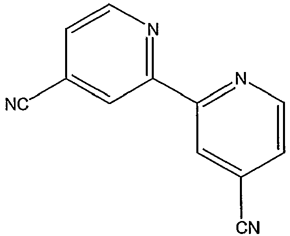

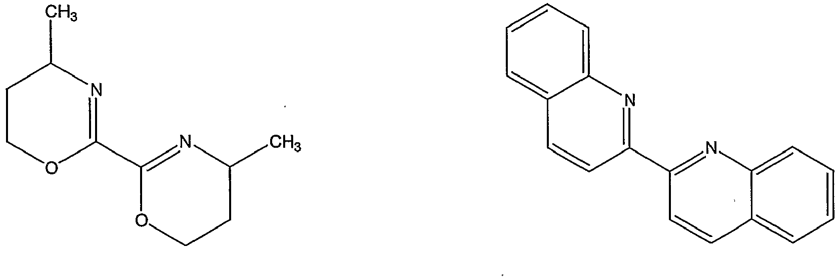

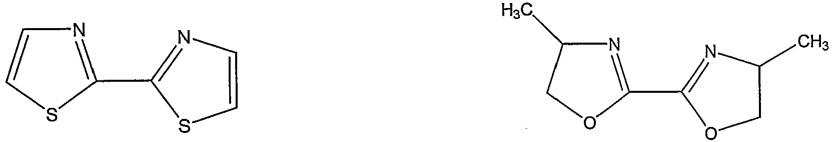

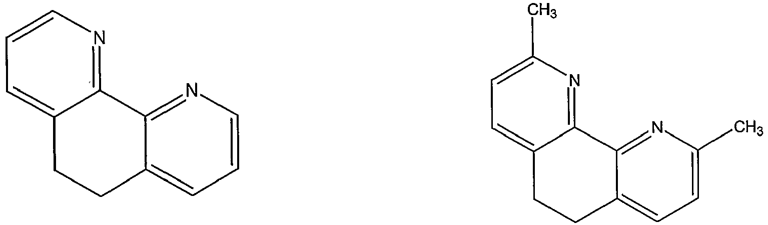

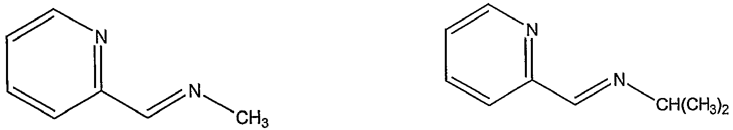

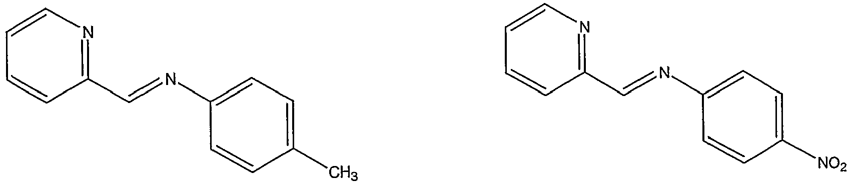

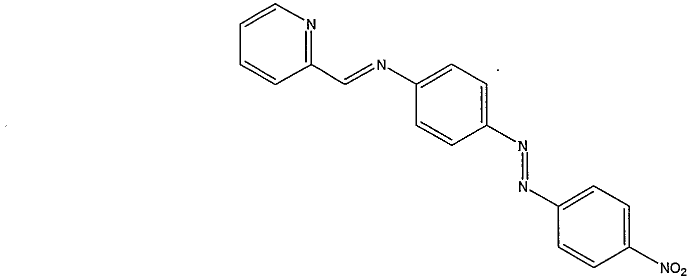

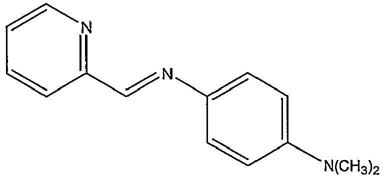

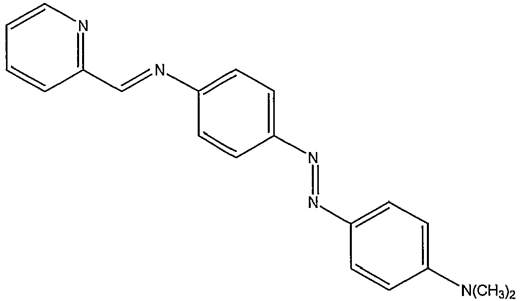

A more preferred group of compounds is formed when L² and L^{1'} independently of one another are pyridyl which is unsubstituted or substituted by one or more substituents from the group consisting of C₁-C₁₂ alkyl, C₂-C₁₁ heterocycloalkyl, C₅-C₉ heteroaryl, halogen, monoamino, diamino and -C(O)H. Examples are

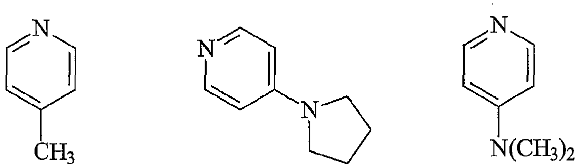

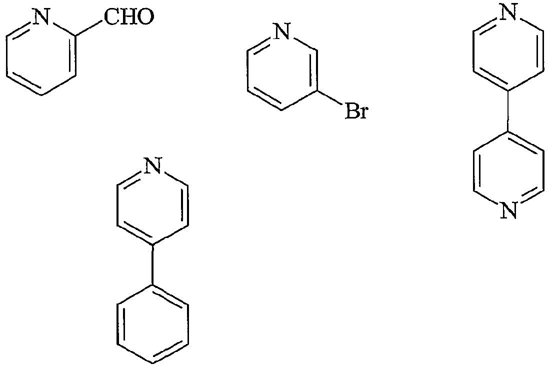

Another preferred group of compounds is formed when L² and L^{1'} together are bipyridyl, phenanthrolinyl, bithiazolyl, bipyrimidinyl or picolylimine which are unsubstituted or substituted by one or more substituents from the group consisting of C₁ -C₁₂ alkyl, C₆-C₁₀ aryl and cyano, the substituents alkyl and aryl being in turn unsubstituted or substituted by one or more substituents from the group consisting of C₁-C₁₂ alkyl, nitro, monoamino, diamino and nitro- or diamino-substituted --N=.N-C₆-C₁₀ aryl. Examples are:

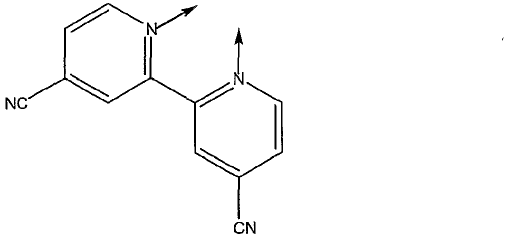

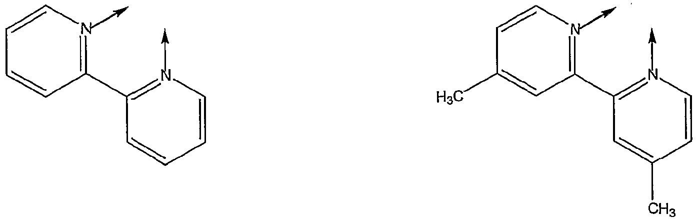

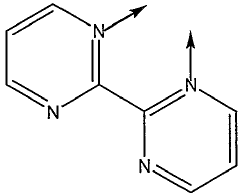

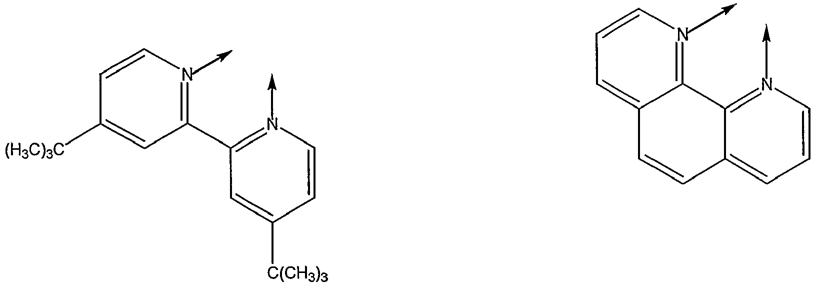

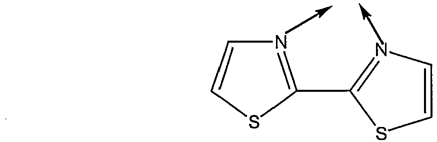

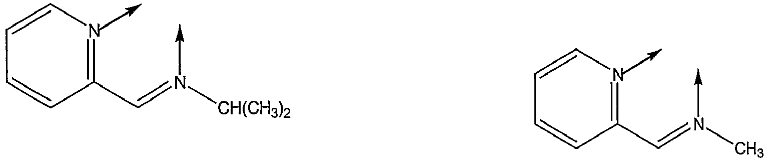

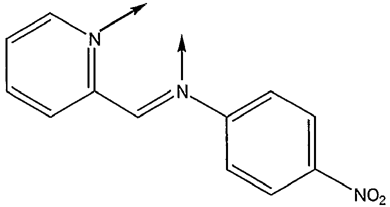

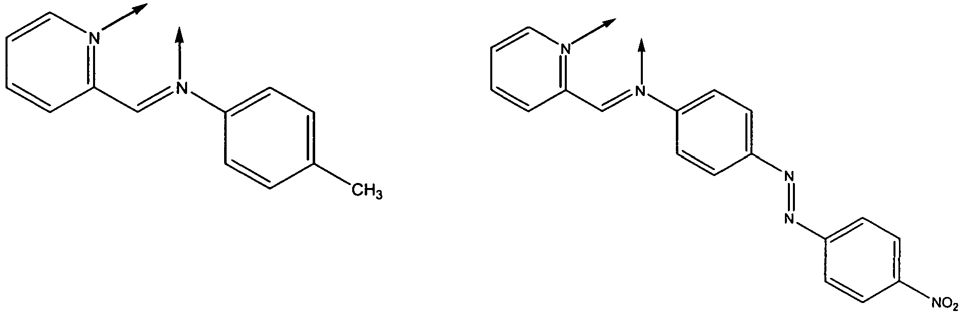

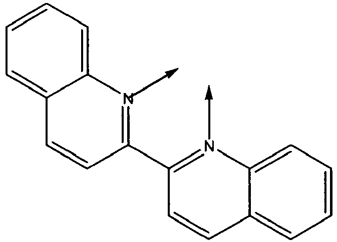

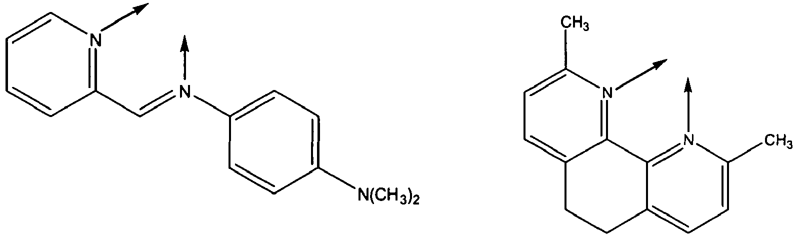

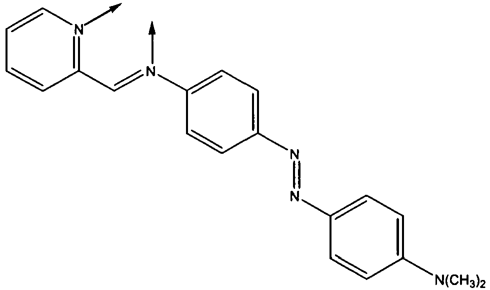

Even more preferably, L² and L^{1'} are each independently selected from the group consisting of: 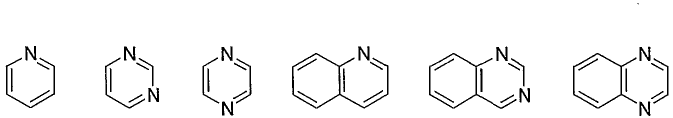 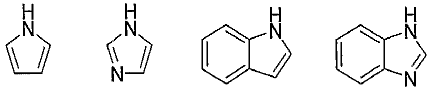 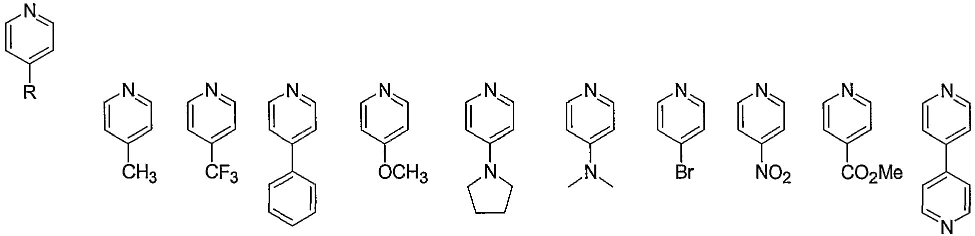 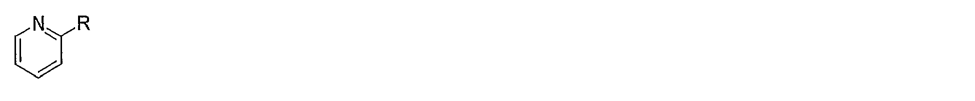 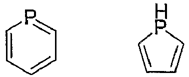 wherein R is selected from the group consisting of hydrogen or a substituent selected from the group consisting of C₁-C₂₀ alkyl, C₂-C₂₀ alkenyl, C₂-C₂₀ alkynyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl, C₁-C₂₀ alkylsulfinyl, and silyl. Optionally, the R group may be substituted with one or more moieties selected from the group consisting of C₁-C₁₀ alkyl, C₁-C₁₀ alkoxy, and aryl which in turn may each be further substituted with one or more groups selected from a halogen, a C₁-C₅ alkyl, C₁-C₅ alkoxy, and phenyl. Moreover, any of the heterocycles may further include one or more functional groups. Examples of suitable functional groups include but are not limited to: hydroxyl, thiol, thioether, ketone, aldehyde, ester, ether, amine, imine, amide, nitro, carboxylic acid, disulfide, carbonate, isocyanate, carbodiimide, carboalkoxy, carbamate, halogen, alcohol, sulfonic acid, phosphine, imide, acetal, ketal, boronate, cyano, cyanohydrin, hydrazine, enamine, sulfone, sulfide, and sulfenyl. Preferably R is selected from the group consisting of C₁-C₂₀ alkyl, aryl, ether, amine, halide, nitro, ester and pyridyl.

Preferably complexes **1a-4b** and **44-48** are used to make the following cumulated complexes **49-83** and cumulated complexes analogous to the following non-cumulated complexes **5 to 29** (complexes 49-63, 69, 70, & 72-77 are not complexes of the invention): 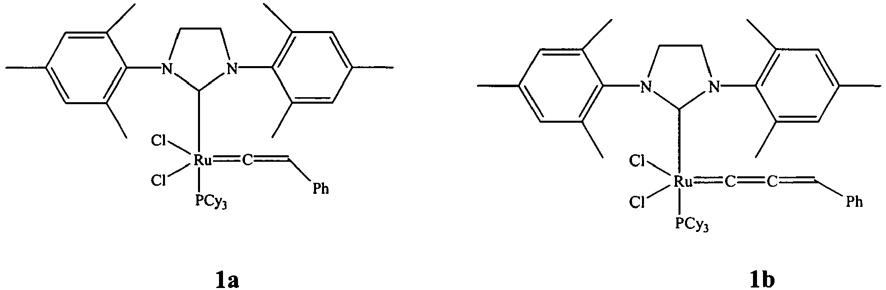 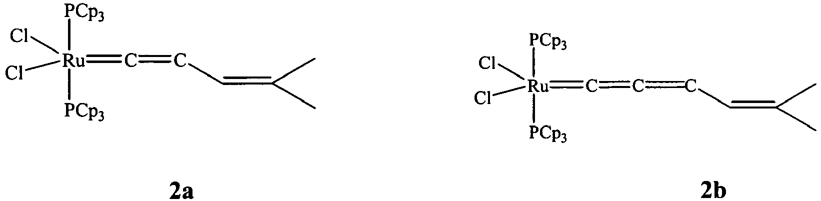 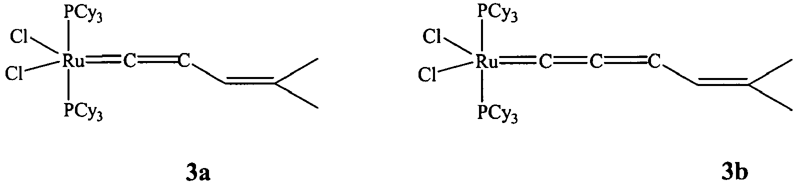 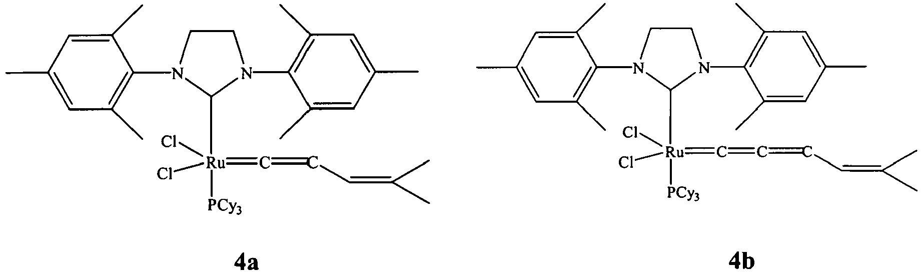 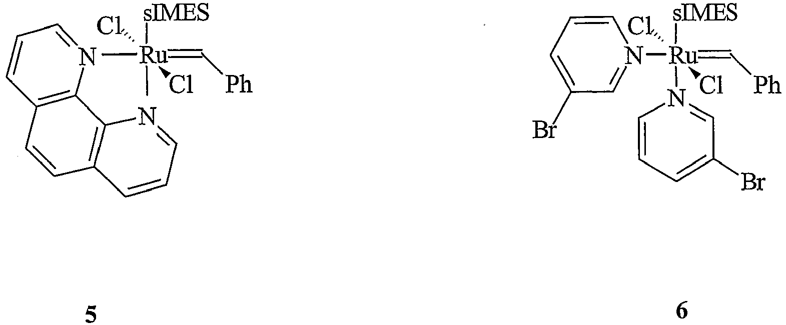 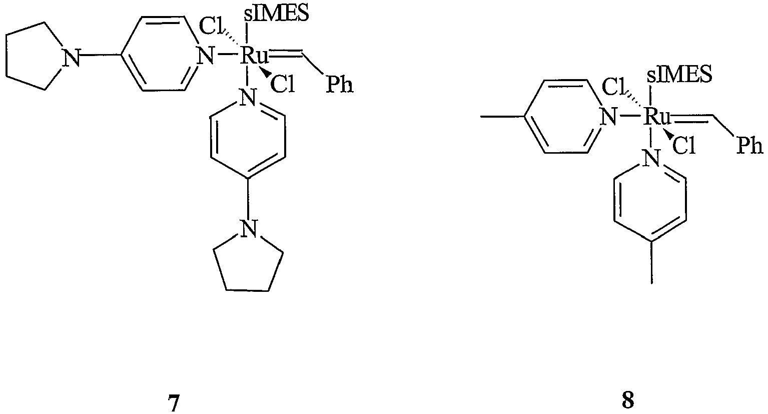 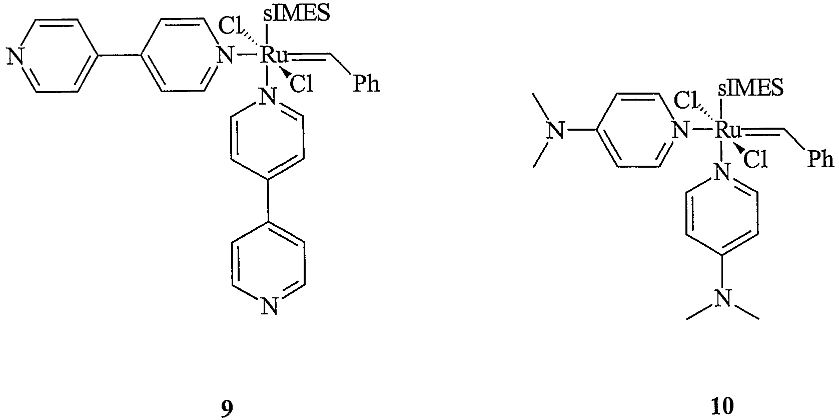 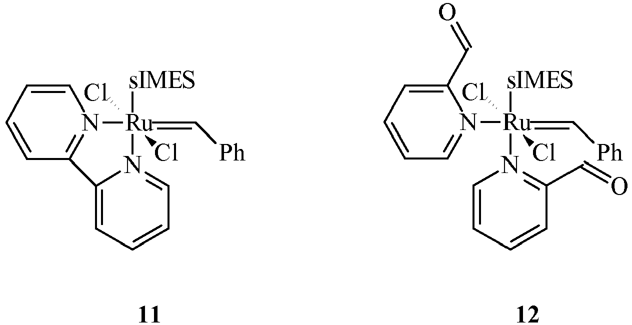 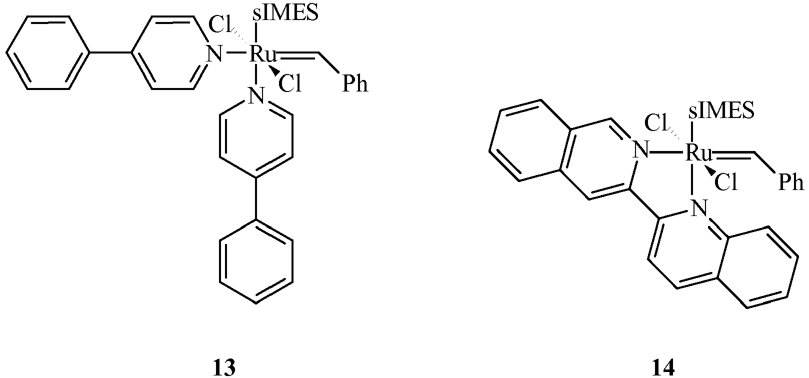 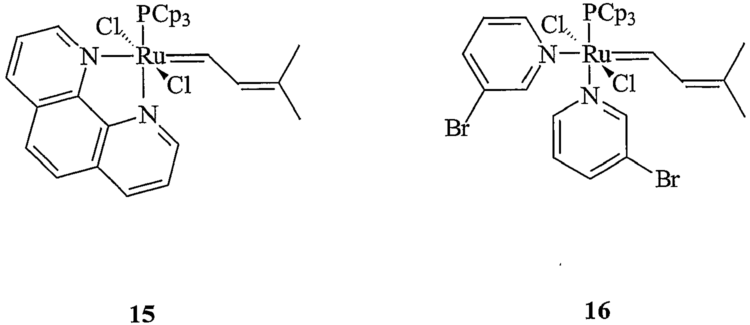 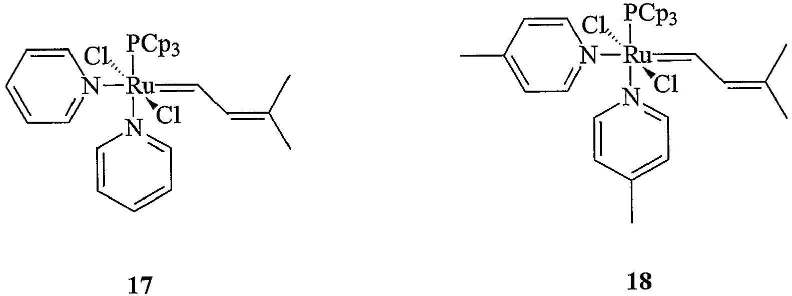 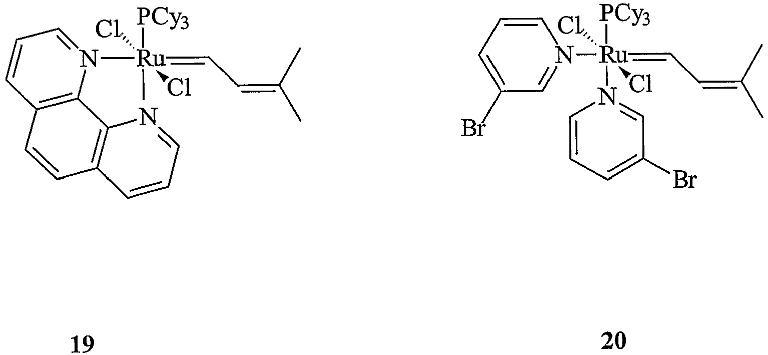 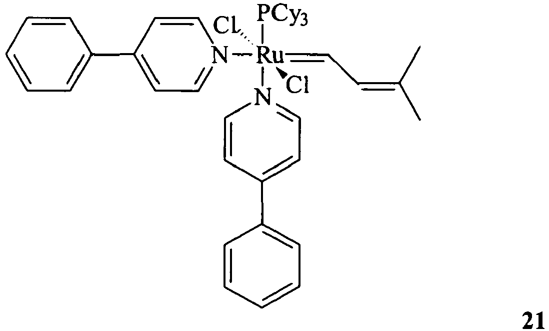 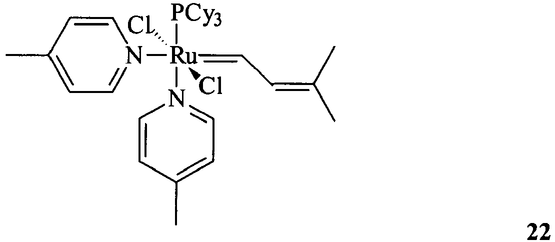 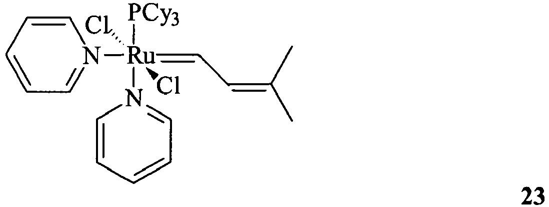 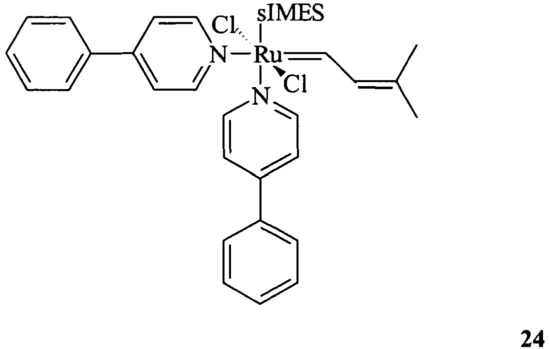 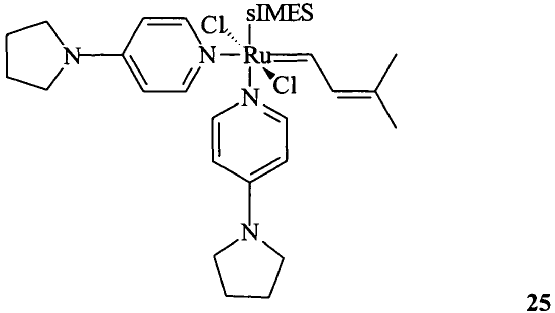 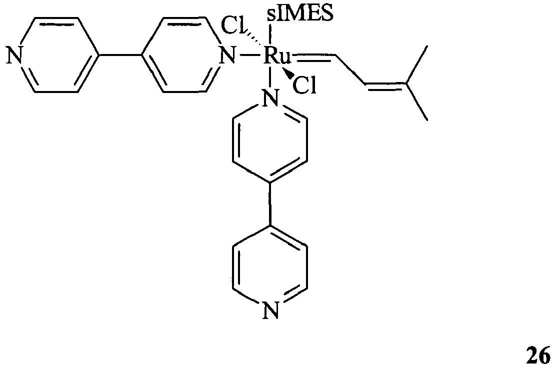 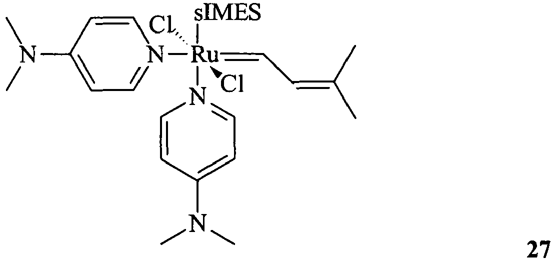 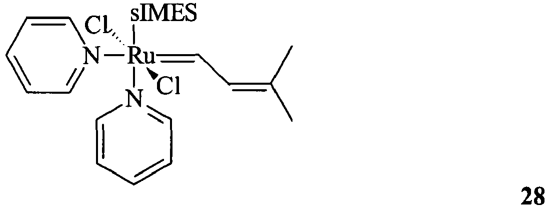 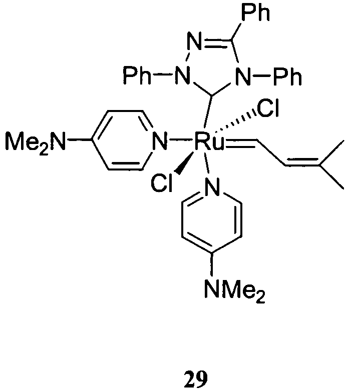 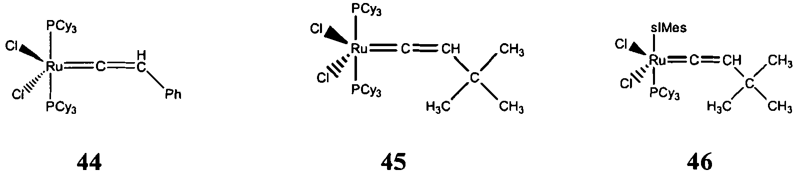 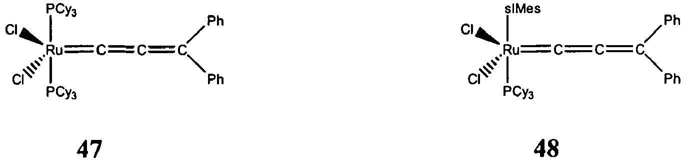 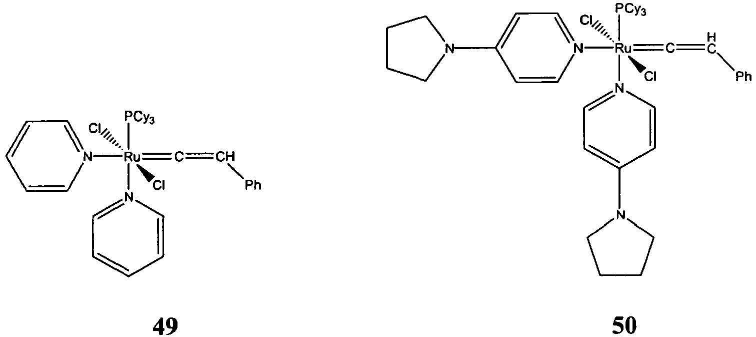 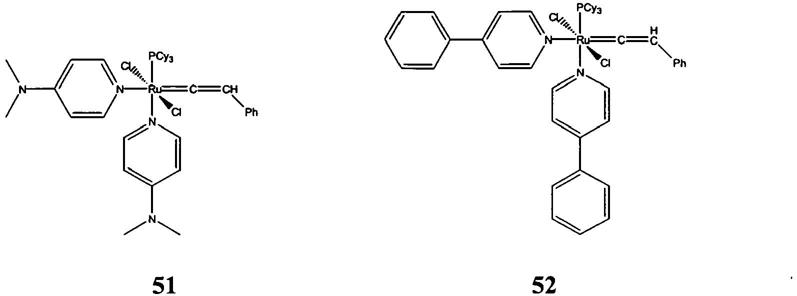 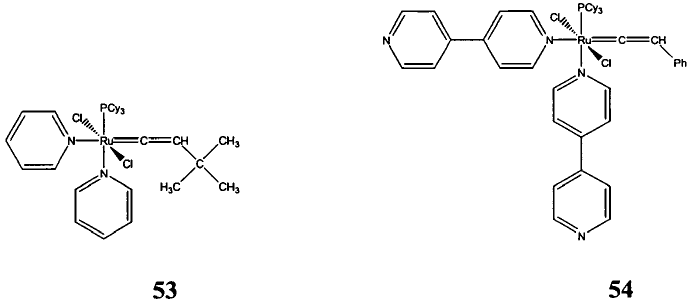 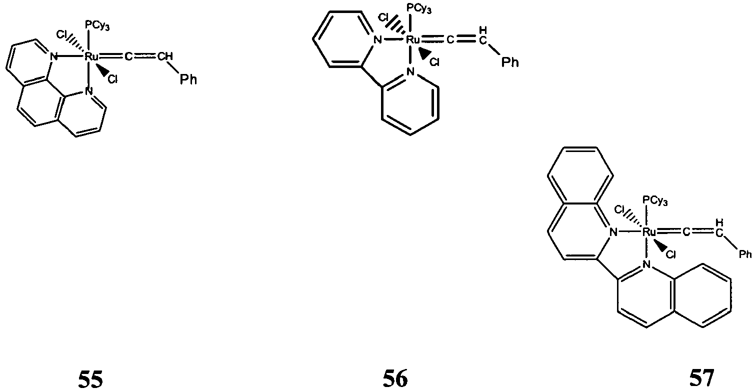 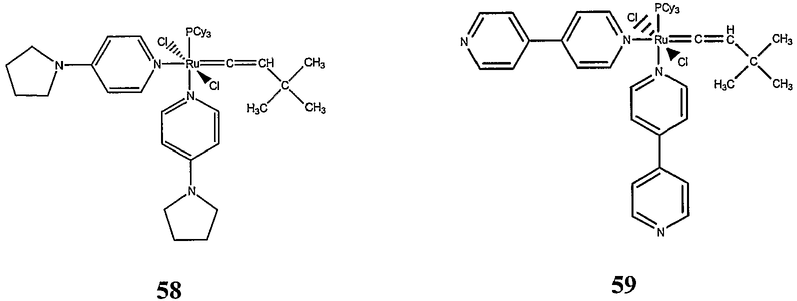 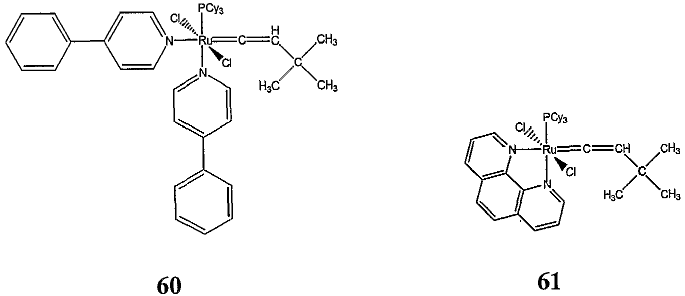 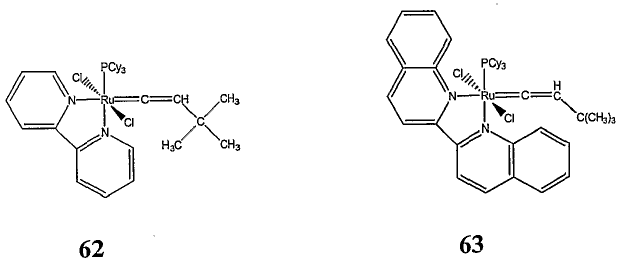 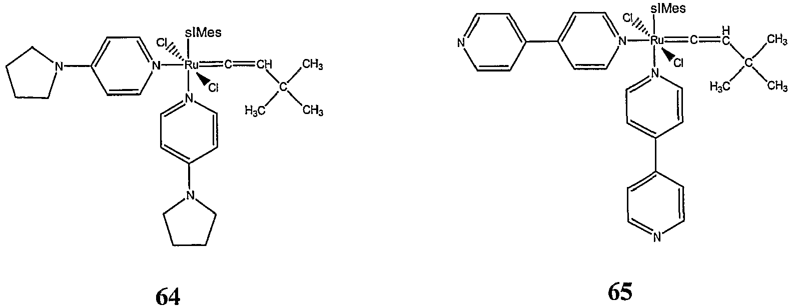 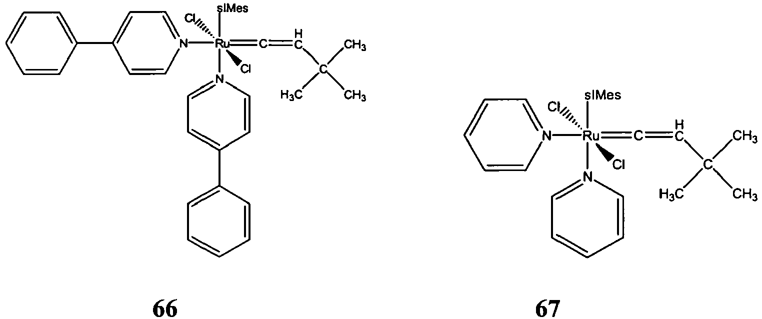 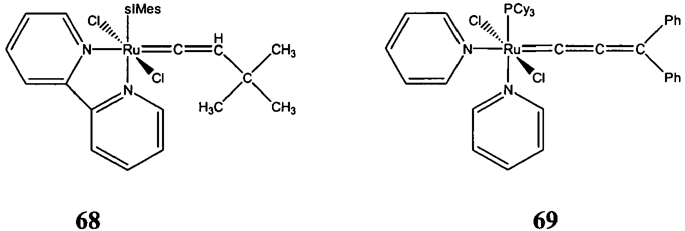 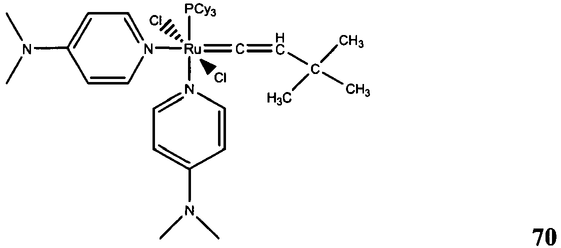 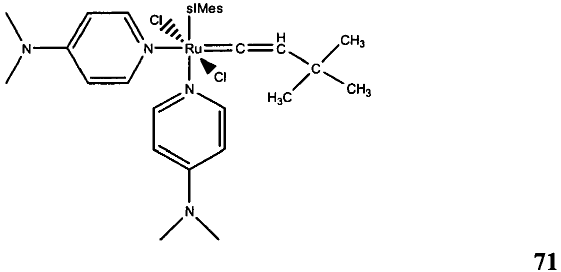 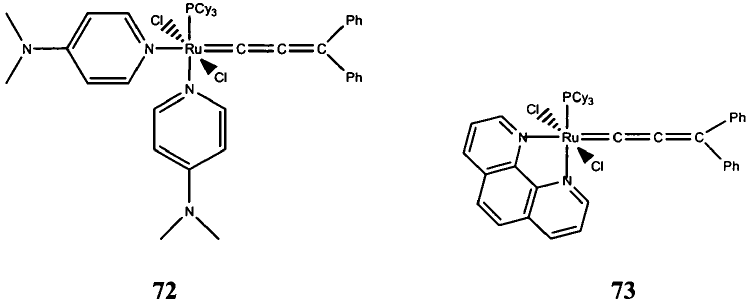 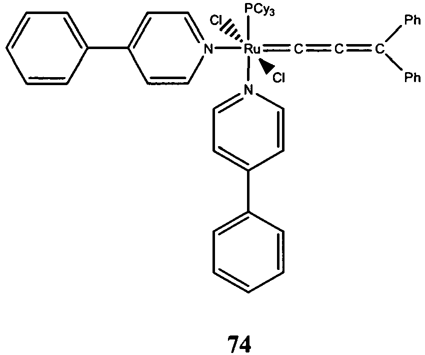 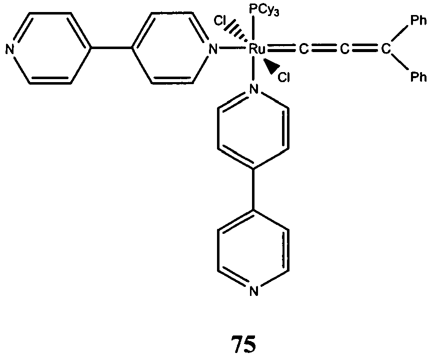 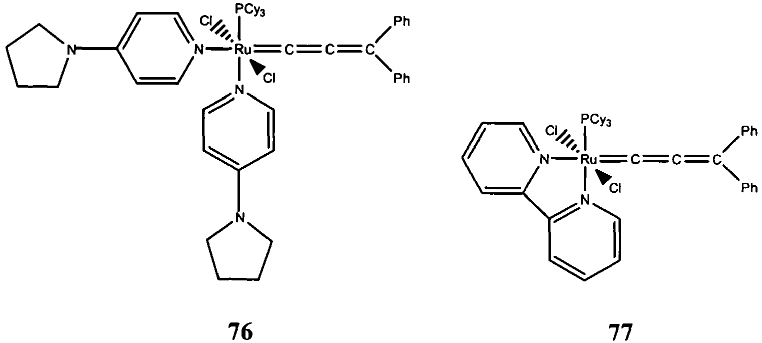 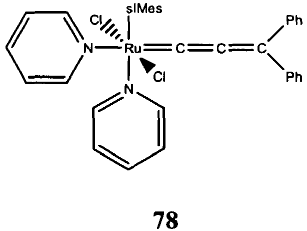 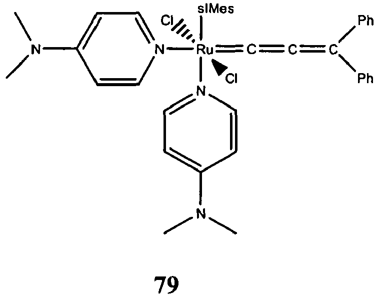 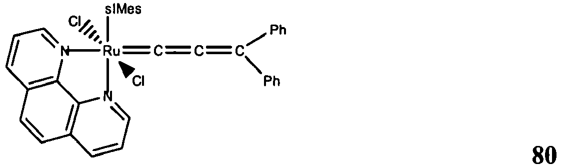 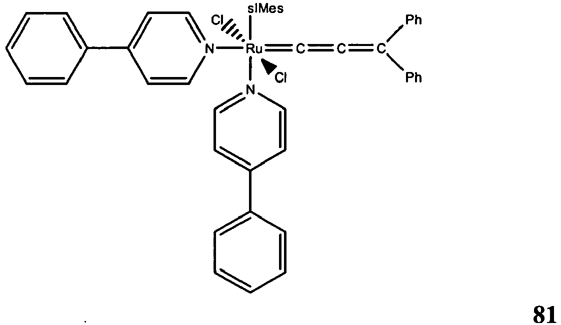 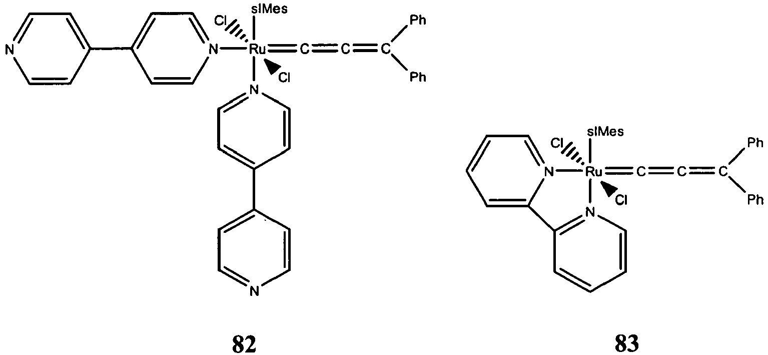 wherein sIMES or IMesH₂ is

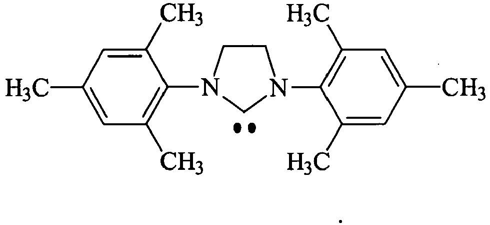

Most preferably, L is an NHC, preferably an imidazolidine ligand, and L² and L^{1'} are pyridines.

The complexes can also be of the formulae: 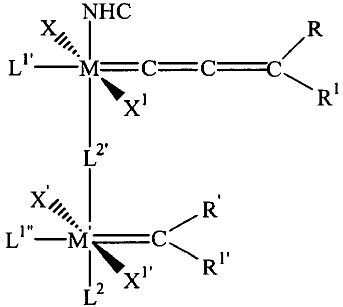 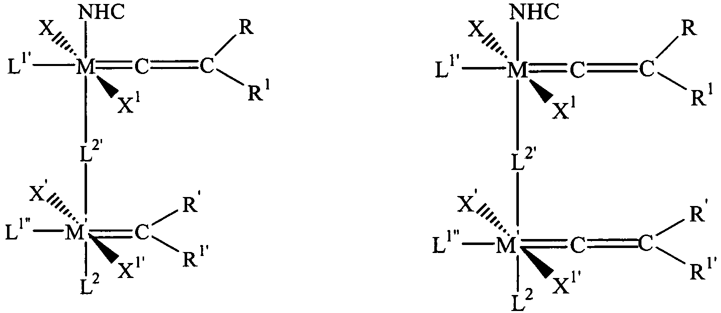 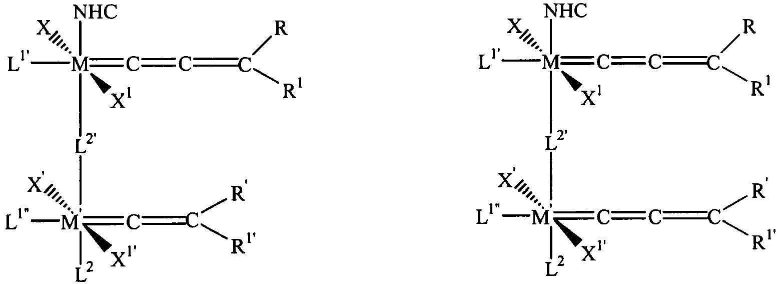 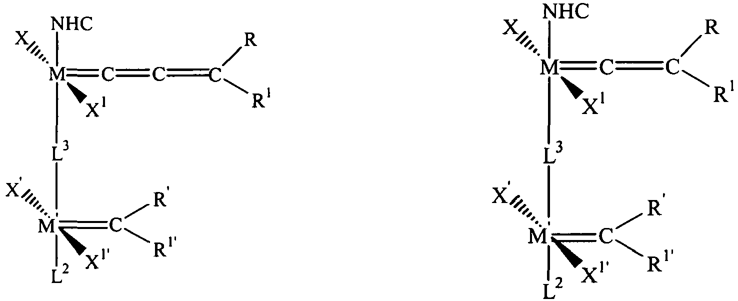 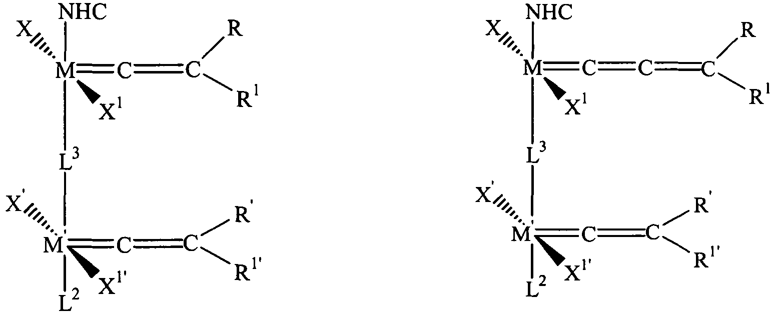 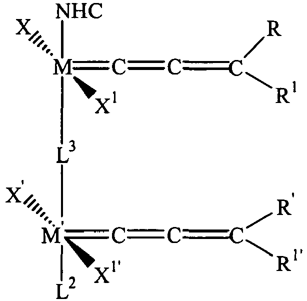 wherein M and M' are independently selected from the group consisting of ruthenium and osmium, X, X¹, L², L^{1'}, R and R¹ are as previously defined, X^{'} and X^{1'} are substituted or unsubstituted and are independently selected from the group from which X and X¹ are selected, R^{'} and R^{1'} are substituted or unsubstituted and are independently selected from the group from which R and R¹ are selected, L^{1"} is selected from the group from which L^{1'} is selected, L^{2'} is any bidentate, neutral electron donor ligand, and L³ is any tetradentate, neutral electron donor ligand.

Using cumulated pentacordinated complexes, for example those seen in complexes 1-4 , in the inventive process will produce inventive cumulated hexacoordinated complexes. For example, the cumulated complexes corresponding to complex 5 is as follows

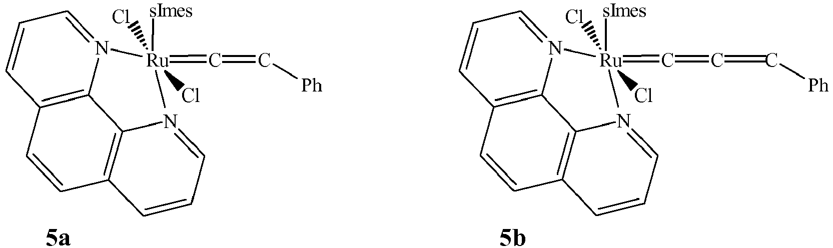

In all of the above carbene complexes, at least one of L, L^{1'}, L², X, X¹, R and R¹, may be linked to at least one other of L, L^{1'}, L², X, X¹, R and R¹ to form a bidentate or multidentate ligand array.

### Synthesis:

In general, hexacoordinated metal carbene complexes catalysts can be made by contacting excess neutral electron donor ligand, such as a pyridine, with the previously described penta-coordinated cumulated analogues
wherein the third neutral electron donor ligand attaches to the metal center. Scheme 1 shows the general synthesis reaction for forming the analogous hexacoordinated metal carbene complexes: 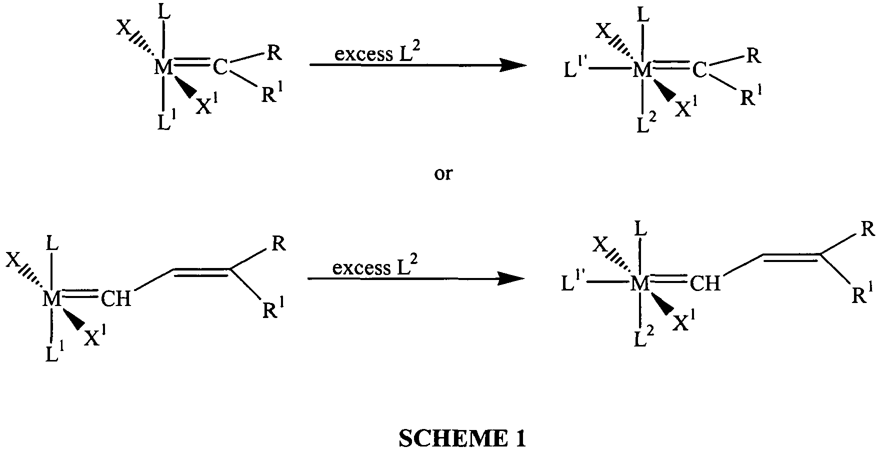 wherein:
M, X, X¹, L, L¹ L^{1'}, L², R and R¹ are as previously defined.

The analogous synthesis of a preferred embodiment is shown in Scheme 2:

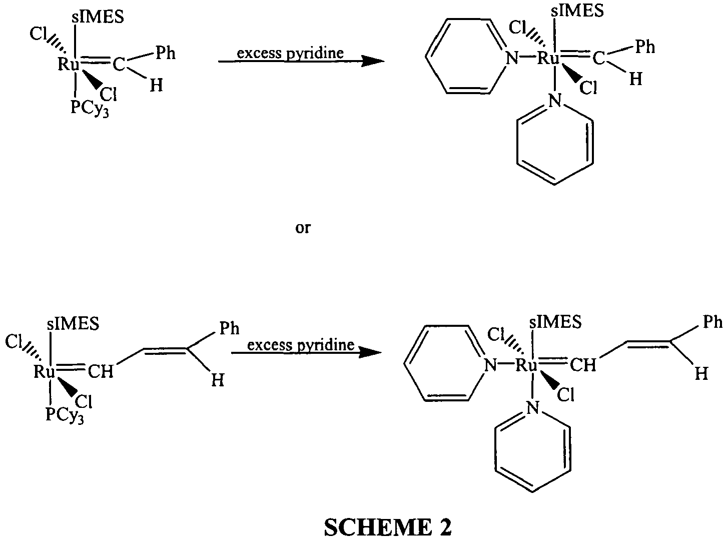

As shown by Schemes 1 and 2, in the presence of excess ligand L², the pentacoordinated complex loses the L¹ ligand and ligands L² and L^{1'} attach to the metal center. Ligands L² and L^{1'} may be the same compound, for example, pyridines (when excess pyridine is used), or may together form a bidentate ligand. Alternatively, L¹ and L^{1'} may be the same, in which case, the pentacoordinated compound does not necessarily lose the L¹ ligand in the presence of excess L².

The synthesis of preferred embodiments of the vinylidenes can be seen in Scheme 3 (product not in accordance with the invention):

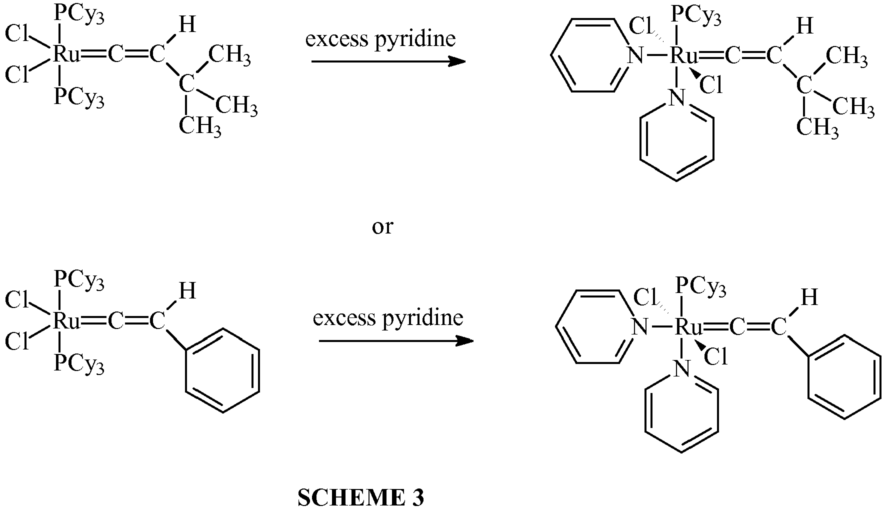

Other preferred compounds synthesized by method include where L² and L^{1'} form a bidentate ligand: 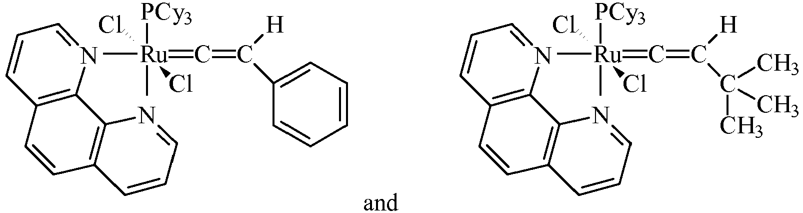 (products not in accordance with the invention)

The inventive hexacoordinated catalyst complexes provide synthetic utility and utility in catalytic reactions. Without being bound by theory, these complexes contain substitutionally labile ligands, for example, pyridine and chloride ligands, and serve as a versatile starting material for the synthesis of new ruthenium metal carbene complexes. The chloride ligands are more labile than in the corresponding pentacoordinated phosphine-based complexes. As stated above, X and X¹ are any anionic ligand. Preferably X and X¹ are selected from the group consisting of chloride, bromide, iodide, tris(pyrazolyl)borate (Tp), alkoxide, amide, and thiolate. The pyridine ligands are more labile than the phosphines in the corresponding pentacoordinated phosphine-based complexes. Again, as stated above, L, L¹, L^{1'}, and L² can be any neutral electron donor ligands, including the NHC ligand. Depending on the size of the ligand, one or two neutral ligands (in addition to the NHC) may bind to the metal center.

Interestingly, hexacoordinated catalyst complexes may be used in both metathesis reactions or the formation of an NHC ligand based complex. As shown in Scheme 4, the hexacoordinated complex can lose a neutral electron donor ligand to produce the pentacoordinated catalyst complex. The reaction may also proceed the other way to produce a hexacoordinated complex in the presence of excess L².

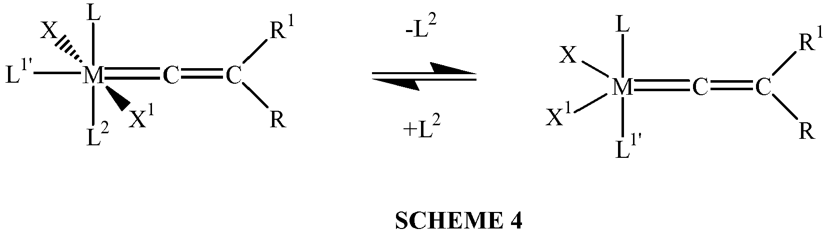

The pentacoordinated complex may also lose the L¹ ligand to form the metathesis active tetracoordinated species (Scheme 5):

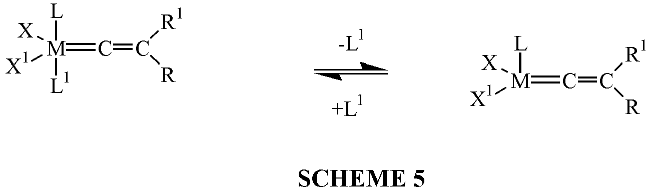

As shown in Scheme 5, the L¹ ligand may also attach to a tetracoordinated species to form the pentacoordinated complex.

The tetracoordinated species may then initiate polymerization when in the presence of an olefin, as shown in Scheme 6, or may form the NHC-ligand based pentacoordinated complex when in the presence of a protected NHC-ligand (Scheme 7):

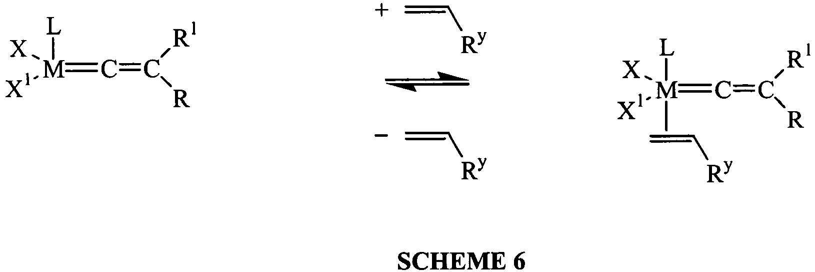

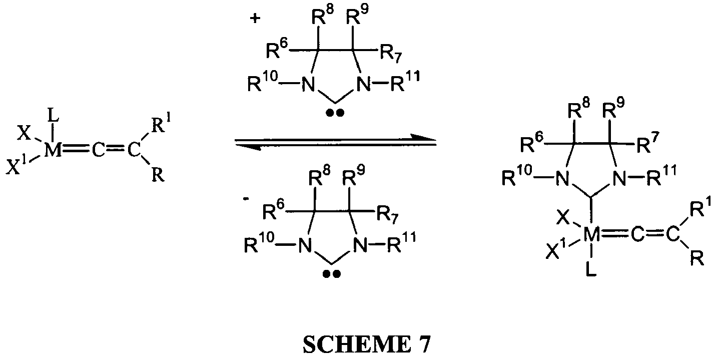

The following structure NHC-A-B indicates generally the protected form of a N-Heterocyclic Carbene (NHC).

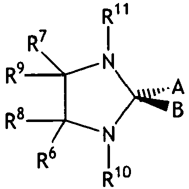

It is also envisioned that the protected NHC-A-B may be of an unsaturated variety, such as

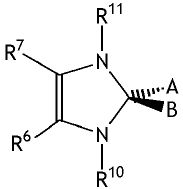

In the above structures, A is preferably H, Si, Sn, Li, Na, MgX³ and acyl, wherein X³ is any halogen, and B may be selected from the group consisting of CCl₃; CH₂SO₂Ph; C₆F₅; OR²¹; and N(R²²)(R²³), wherein R²¹ is selected from the group consisting of Me, C₂H₅, i-C₃H₇, CH₂CMe₃, CMe₃, C₆H₁₁ (cyclohexyl), CH₂Ph, CH₂norbornyl, CH₂norbornenyl, C₆H₅, 2,4,6-(CH₃)₃C₆H₂ (mesityl), 2,6-i-Pr₂C₆H₂, 4-Me-C₆H₄ (tolyl), 4-Cl-C₆H₄; and wherein R²² and R²³ are independently selected from the group consisting of Me, C₂H₅, i-C₃H₇, CH₂CMe₃, CMe₃, C₆H₁₁ (cyclohexyl), CH₂Ph, CH₂norbornyl, CH₂norbornenyl, C₆H₅, 2,4,6-(CH₃)₃C₆H₂ (mesityl), 2,6-i-Pr₂C₆H₂, 4-Me-C₆H₄ (tolyl), 4-Cl-C₆H₄. This approach relates to the thermal generation of a NHC ligand from a stable (protected) NHC derivative with a release of a quantity of A-B. One of the more preferred methods to generate a reactive NHC ligand is to employ a stable carbene precursor where the A-B compound is also a reactive NHC ligand. A detailed description of the protected NHC and related methods of synthesis and use can be seen in U.S. Patent Application Nos. 10/107,531 (see WO 03/097562; European patent Application No. 02807449) and No. 10/138,188 (see US-A-20030083445). The following structure for the sImesHCCl₃ shows a preferred embodiment of a protected NHC ligand for use with the hexacoordinated complexes:

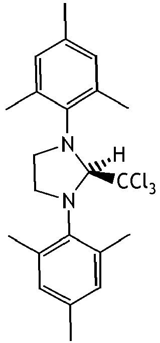

The NHC ligand based pentacoordinated complex may then lose the L ligand to form the metathesis active tetracoordinated species and proceed to initiate the polymerization reaction in the presence of an olefin (Scheme 8):

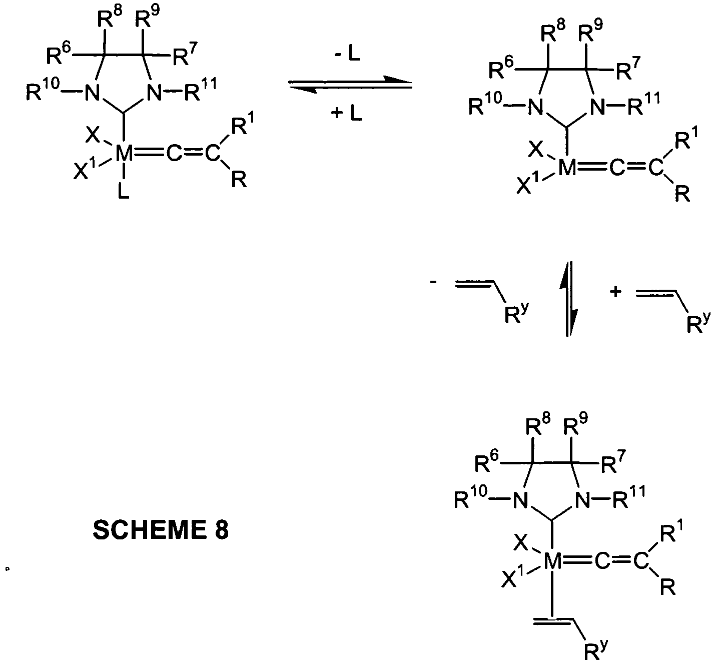

It should also be noted that the hexacoordinated complex can undergo a ligand exchange such that the NHC replaces another neutral electron donor ligand resulting in an NHC ligand based hexacoordinated complex (Scheme 9):

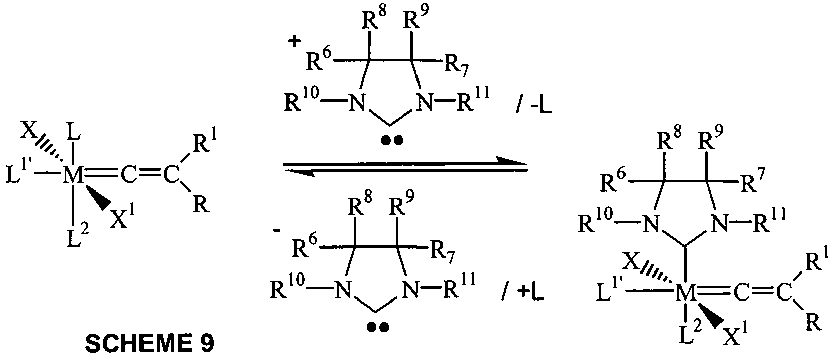

In all the above schemes and complexes M, X, X¹, L, L¹, L^{1'}, L², R, R¹, R⁶, R⁷, R⁸, R⁹, R¹⁰, R¹¹, and R^{y} are as previously defined.

### Olefin Metathesis:

The inventive complexes are useful in olefin metathesis reactions, particularly for polymerization reactions. These catalysts can be used in various metathesis reactions, including but not limited to, ring-opening metathesis polymerization of strained and unstrained cyclic olefins, ring-closing metathesis of acyclic dienes, acyclic diene metathesis polymerization ("ADMET"), self- and cross-metathesis reactions, alkyne polymerization, carbonyl olefination, depolymerization of unsaturated polymers, synthesis of telechelic polymers, and olefin synthesis.

The most preferred olefin monomer for use in the invention is substituted or unsubstituted dicyclopentadiene (DCPD). Various DCPD suppliers and purities may be used such as Lyondell 108 (94.6% purity), Veliscol™ UHP (99+% purity), B.F. Goodrich Ultrene^{®} (97% and 99% purities), and Hitachi (99+% purity). Other preferred olefin monomers include other cyclopentadiene oligomers including trimers, tetramers, and pentamers,; cyclooctadiene (COD; DuPont); cyclooctene (COE, Alfa Aesar); cyclohexenylnorbornene (Shell); norbornene (Aldrich); norbornene dicarboxylic anhydride (nadic anhydride); norbornadiene (Elf Atochem); and substituted norbornenes including butyl norbornene, hexyl norbornene, octyl norbornene, and decyl norbornene,. Preferably, the olefinic moieties include mono-or disubstituted olefins and cycloolefins containing between 3 and 200 carbons. Most preferably, metathesis-active olefinic moieties include substituted or unsubstituted cyclic or multicyclic olefins, for example, cyclopropenes, cyclobutenes, cycloheptenes, cyclooctenes, [2.2.1]bicycloheptenes, [2.2.2]bicyclooctenes, benzocyclobutenes, cyclopentenes, cyclopentadiene oligomers including trimers, tetramers, and pentamers,; cyclohexenes. It is also understood that such compositions include frameworks in which one or more of the carbon atoms carry substituents derived from radical fragments including halogens, pseudohalogens, alkyl, aryl, acyl, carboxyl, alkoxy, alkyl- and arylthiolate, amino, and aminoalkyl, , or in which one or more carbon atoms have been replaced by, for example, silicon, oxygen, sulfur, nitrogen, phosphorus, antimony, or boron. For example, the olefin may be substituted with one or more groups such as thiol, thioether, ketone, aldehyde, ester, ether, amine, amide, nitro, carboxylic acid, disulfide, carbonate, isocyanate, phosphate, phosphite, sulfate, sulfite, sulfonyl, carbodiimide, carboalkoxy, carbamate, halogen, or pseudohalogen. Similarly, the olefin may be substituted with one or more groups such as C₁-C₂₀ alkyl, aryl, acyl, C₁-C₂₀ alkoxide, aryloxide, C₃-C₂₀ alkyldiketonate, aryldiketonate, C₁-C₂₀ carboxylate, arylsulfonate, C₁-C₂₀ alkylsulfonate, C₁-C₂₀ alkylthio, arylthio, C₁-C₂₀ alkylsulfonyl, and C₁-C₂₀ alkylsulfinyl, C₁-C₂₀ alkylphosphate, arylphosphate, wherein the moiety may be substituted or unsubstituted.

Examples of preferred polymerizable norbornene-type monomers include but are not limited to, norbornene (bicyclo[2.2.1]hept-2-ene), 5-methyl-2-norbornene, ethylnorbornene, propylnorbornene, isopropylnorbornene, butylnorbornene, isobutylnorbornene, pentylnorbornene, hexylnorbornene, heptylnorbornene, octylnorbornene, decylnorbornene, dodecylnorbornene, octadecylnorbornene, p-tolylnorbornene, methylidene norbornene, phenylnorbornene, ethylidenenorbornene, vinylnorbornene, exo-dicyclopentadiene, endo-dicyclopentadiene, tetracyclododecene, methyltetracyclododecene, tetracyclododecadiene, dimethyltetracyclododecene, ethyltetracyclododecene, ethylidenyl tetracyclododecene, phenyltetracyclodecene, symmetrical and unsymmetrical trimers and tetramers of cyclopentadiene, 5,6-dimethylnorbornene, propenylnorbornene, 5,8-methylene-5a,8a-dihydrofluorene, cyclohexenylnorbornene, dimethanohexahydronaphthalene, endo,exo-5,6-dimethoxynorbornene, endo,endo-5,6-dimethoxynorbornene, 2,3-dimethoxy-norbornadiene, 5,6-bis(chloromethyl)bicyclo[2.2.1]hept-2-ene, 5-tris(ethoxy)-silylnorbornene, 2-dimethylsilylbicyclo[2.2.1]hepta-2,5-diene, 2,3-bistrifluoromethylbicyclo[2.2.1]hepta-2,5-diene, 5-fluoro-5-pentafluoroethyl-6,6-bis(trifluoromethyl)-bicyclo[2.2.1]hept-2-ene, 5,6-difluoro-5-heptatafluoroisopropyl-6-trifluoromethyl)-bicyclo[2.2.1]hept-2-ene, 2,3,3,4,4,5,5,6-octafluorotricyclo[5.2.1.0]dec-8-ene, and 5-trifluoromethylbicyclo[2.2.1]hept-2-ene, 5,6-dimethyl-2-norbornene, 5-a-naphthyl-2-norbornene, 5,5-dimethyl-2-norbornene, 1,4,4a,9,9a,10-hexahydro-9,10[1',2']-benzeno-1,4-methanoanthracene. indanylnorbornene (i.e., 1,4,4,9-tetrahydro-1,4-methanofluorene, the reaction product of CPD and indene), 6,7,10,10-tetrahydro-7,10-methanofluoranthene (i.e., the reaction product of CPD with acenaphthalene), 1,4,4,9,9,10-hexahydro-9,10[1',2']-benzeno-1,4-methanoanthracene, endo,endo-5,6-dimethyl-2-norbornene, endo,exo-5,6-dimethyl-2-norbornene, exo,exo-5,6-dimethyl-2-norbornene, 1,4,4,5,6,9,10,13,14,14-decahydro-1,4-methanobenzocyclododecene (i.e., reaction product of CPD and 1,5,9-cyclododecatriene), 2,3,3,4,7,7-hexahydro-4,7-methano-1H-indene (i.e., reaction product of CPD and cyclopentene), 1,4,4,5,6,7,8,8-octahydro-1,4-methanonaphthalene (i.e., reaction product of CPD and cyclohexene), 1,4,4,5,6,7,8,9,10,10-decahydro-1,4-methanobenzocyclooctene (i.e., reaction product of CPD and cyclooctene), and 1,2,3,3,3,4,7,7,8,8,decahydro-4,7-methanocyclopent[a]indene.

These olefin monomers may be used alone or mixed with each other in various combinations to adjust the properties of the olefin monomer composition. For example, mixtures of cyclopentadiene dimer and trimers offer a reduced melting point and yield cured olefin copolymers with increased mechanical strength and stiffness relative to pure poly-DCPD. As another example, incorporation of COD, norbornene, or alkyl norbornene co-monomers tend to yield cured olefin copolymers that are relatively soft and rubbery. The resulting polyolefin compositions formed from the metathesis reactions are amenable to thermosetting and are tolerant of additives, stabilizers, rate modifiers, hardness and/or toughness modifiers, fillers and fibers including, but not limited to, carbon, glass, aramid (e.g., Kevlar^{®} and Twaron^{®}), polyethylene (e.g., Spectra^{®} and Dyneema^{®}), polyparaphenylene benzobisoxazole (e.g., Zylon^{®}), polybenzamidazole (PBI), and hybrids thereof as well as other polymer fibers.

The metathesis reactions may optionally include formulation auxiliaries. Known auxiliaries include antistatics, antioxidants (primary antioxidants, secondary antioxidants, or mixtures thereof), ceramics, light stabilizers, plasticizers, dyes, pigments, fillers, reinforcing fibers, lubricants, adhesion promoters, viscosity-increasing agents, and demolding enhancers. Illustrative examples of fillers for improving the optical physical, mechanical, and electrical properties include glass and quartz in the form of powders, beads, and fibers, metal and semi-metal oxides, carbonates (e.g. MgCO₃, CaCO₃), dolomite, metal sulfates (e.g. gypsum and barite), natural and synthetic silicates (e.g. zeolites, wollastonite, and feldspars), carbon fibers, and plastics fibers or powders.

The UV and oxidative resistance of the polyolefin compositions resulting from the metathesis reactions using the inventive carbene complex may be enhanced by the addition of various stabilizing additives such as primary antioxidants (e.g., sterically hindered phenols), secondary antioxidants (e.g., organophosphites and thioesters), light stabilizers (e.g., hindered amine light stabilizers or HALS), and UV light absorbers (e.g., hydroxy benzophenone absorbers and hydroxyphenylbenzotriazole absorbers,), as described in U.S. Application No. 09/498,120, filed February 4, 2000 (see U.S. Patent No. 6583236).

Exemplary primary antioxidants include, for example, 4,4'-methylenebis (2,6-di-tertiarybutylphenol) (Ethanox 702^{®}; Albemarle Corporation), 1,3,5-trimethyl-2,4,6-tris (3,5-di-tert-butyl-4-hydroxybenzyl) benzene (Ethanox 330^{®}; Albermarle Corporation), octadecyl-3-(3',5'-di-tert-butyl-4'-hydroxyphenyl) propionate (Irganox 1076^{®}; Ciba-Geigy), and pentaerythritol tetrakis(3-(3,5-di-tert-butyl-4-hydroxyphenyl)propionate)(Irganox^{®} 1010; Ciba-Geigy). Exemplary secondary antioxidants include tris(2,4-ditert-butylphenyl)phosphite (Irgafos^{®} 168; Ciba-Geigy), and 1:11(3,6,9-trioxaudecyl)-bis(dodecylthio)propionate (Wingstay^{®} SN-1; Goodyear),. Exemplary light stabilizers and absorbers include bis(1,2,2,6,6-pentamethyl-4-piperidinyl)-[[3,5-bis(1,1-dimethylethyl)-4-hydroxyphenyl]methyl]butylmalonate (Tinuvin^{®} 144 HALS; Ciba-Geigy), 2-(2H-benzotriazol-2-yl)-4,6-ditertpentylphenol (Tinuvin^{®} 328 absorber; Ciba-Geigy), 2,4-di-tert-butyl-6-(5-chlorobenzotriazol-2-yl)phenyl (Tinuvin^{®} 327 absorber; Ciba-Geigy), and 2-hydroxy-4-(octyloxy)benzophenone (Chimassorb^{®} 81 absorber; Ciba-Geigy),.

In addition, a suitable rate modifier such as, for example, triphenylphosphine (TPP), tricyclopentylphosphine, tricyclohexylphosphine, triisopropylphosphine, trialkylphosphites, triarylphosphites, mixed phosphites, or other Lewis base, as described in U.S. Patent No. 5,939,504 and U.S. Application No. 09/130,586 (see U.S. Patent No. 6310121) may be added to the olefin monomer to retard or accelerate the rate of polymerization as required.

The resulting polyolefin compositions, and parts or articles of manufacture prepared therefrom, may be processed in a variety of ways including, for example, Reaction Injection Molding (RIM), Resin Transfer Molding (RTM) and vacuum-assisted variants such as VARTM (Vacuum-Assisted RTM) and SCRIMP (Seemann Composite Resin Infusion Molding Process), open casting, rotational molding, centrifugal casting, filament winding, and mechanical machining. These processing compositions are well known in the art. Various molding and processing techniques are described, for example, in PCT Publication WO 97/20865, and U.S. Provisional Patent Application No. 60/360,755, filed March 1, 2002 and entitled "Polymer Processing Methods and Techniques Using Pentacoordinated or Hexacoordinated Ruthenium or Osmium Metathesis Catalysts".

The metathesis reactions may occur in the presence or absence of a solvent. Examples of solvents that can be used in the polymerization reaction include organic, protic, or aqueous solvents, which are preferably inert under the polymerization conditions. Examples of such solvents include aromatic hydrocarbons, chlorinated hydrocarbons, ethers, aliphatic hydrocarbons, alcohols, water, or mixtures thereof. Preferred solvents include benzene, toluene, p-xylene, methylene chloride, dichloroethane, dichlorobenzene, chlorobenzene, tetrahydrofuran, diethylether, pentane, methanol, ethanol, water or mixtures thereof. More preferably, the solvent is benzene, toluene, p-xylene, methylene chloride, dichloroethane, dichlorobenzene, chlorobenzene, tetrahydrofuran, diethylether, pentane, methanol, ethanol, or mixtures thereof. Most preferably, the solvent is toluene, or a mixture of benzene and methylene chloride. The solubility of the polymer formed in the polymerization reaction will depend on the choice of solvent and the molecular weight of the polymer obtained.

The inventive complexes have a well-defined ligand environment that enables flexibility in modifying and fine-tuning the activity level, stability, solubility and ease of recovery of these catalysts. The solubility of the carbene compounds may be controlled by proper selection of either hydrophobic or hydrophilic ligands as is well known in the art. The desired solubility of the catalyst will largely be determined by the solubility of the reaction substrates and reaction products.

The inventive metal carbene complexes have shown a high rate of initiation allowing for most, if not all, of the complex added to the reaction to be consumed. Thus, less catalyst is wasted in the metathesis reaction. In contrast, the previous pentacoordinated initiators had a higher amount of extractibles (i.e. unpolymerized monomer) remaining after the reaction concluded. The rate of propagation is also slowed by the presence of the two pyridine ligands. The high rate of initiation and low rate of propagation yields polymers with narrow polydispersities relative to those achieved with the earlier pentacoordinated complexes. Moreover, it was determined that heat increases the rate of the initiation. Thermal initiation of the pentacoordinated complexes can be seen in U.S. Patent No. 6,107,420. In general, the initiation and/or rate of the metathesis polymerization using the inventive catalysts is controlled by a method comprising contacting the inventive catalyst with an olefin and heating the reaction mixture. In a surprising and unexpected result, the Tₘₐₓ for the thermal initiation of the inventive catalyst is significantly higher than the Tₘₐₓ for the previous pentacoordinated catalysts. Without being bound by theory, this is significant in that in a reaction using a metathesis catalyst, if the part or article being prepared is a type of filled system (e.g., a system containing reinforcing fillers, fibers, beads, etc.), the filling material may act as a heat sink. With the previous pentacoordinated catalysts, post-curing was sometimes necessary due to the effect of the heat sink resulting from a filled system. ROMP polymerization in the presence of peroxide cross linking agents using pentacoordinated catalysts is discussed in U.S. Patent No. 5,728,785. In contrast, the reactions using the inventive hexacoordinated catalysts generate significantly more internal heat. This high Tₘₐₓ reduces the need for post cure. Additionally, even if peroxides or radicals are added to promote crosslinking, the degree of crosslinking in the part that uses the radical mechanism is increased in comparison to a part prepared using the previous pentacoordinated metathesis catalysts. Moreover, the half-life is dependent on the maximum temperature. Using the inventive catalysts, the half life is reduced substantially, and therefore less catalyst is needed, providing a significant commercial advantage. Without being bound by theory, the higher Tₘₐₓ indicates that in a ROMP reaction, more rings are opened, and there is a better degree of cure. With a higher Tₘₐₓ, the extractibles are almost to zero, indicating that almost every molecule that can be reacted is reacted. For example, the vinylidenes are advantageous in that they are more stable at higher temperatures than the alkylidenes. When the protected NHC (e.g., a saturated Imes ligand as described in U.S. Provisional Patent Application Nos. 60/288,680 (corresponding in part to WO 2002/020535) and 60/278,311 (corresponding in part to WO 2002/020535) is added to the reaction mixture, a dramatic increase in peak exotherm is seen. Additionally, the time to reach the peak is significantly reduced. A high peak exotherm means more catalyst is available for polymerization, indicating that the extractibles are close to zero. Accordingly, the inventive catalysts have better conversion, better properties, even in the presence of fillers and additives.

For the purposes of clarity, the specific details of the invention will be illustrated with reference to especially preferred embodiments. However, it should be appreciated that these embodiments and examples are for the purposes of illustration only and are not intended to limit the scope of the invention.

### EXAMPLES

### General Procedures

Manipulation of organometallic compounds was performed using standard Schlenk techniques under an atmosphere of dry argon or in a nitrogen-filled Vacuum Atmospheres drybox (O₂ < 2ppm). NMR spectra were recorded on a Varian Inova™ (499.85 MHz for ¹H; 202.34 MHz for ³¹P; 125.69 MHz for ¹³C) or a Varian Mercury™ 300 (299.817 for ¹H; 121.39 MHz for ³¹P; 74.45 MHz for ¹³C). ³¹P NMR spectra were referenced using H₃PO₄ (ò = 0 ppm) as an external standard. UV-vis spectra were recorded on an HP 8452A diode-array spectrophotometer.

### Materials and Methods

Pentane, toluene, benzene, and benzene-*d*₆ were dried by passage through solvent purification columns. Pyridine was dried by vacuum transfer from CaH₂. All phosphines as well as KTp were obtained from commercial sources and used as received. Ruthenium complexes **1-4** and **44-48** were prepared according to literature procedures.

### Synthesis of (PCy₃)(C₅H₅N)₂(Cl)₂Ru=C=CHPh

Complex **44** (2.0 grams) was dissolved in toluene (10 mL), and pyridine (0.9 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (PCy₃)(C₅H₅N)₂(Cl)₂Ru=C=CHPh **49** as an orange powder (1.5 gram, 88% yield).

**Example (1):** A 75 gram mass of DCPD (containing about 24% trimerized DCPD) was polymerized using (PCy₃)(C₅H₅N)₂(Cl)₂Ru=C=CHPh = 0.0377 grams in the presence of sIMesHCCl₃ = 0.0450 grams at a DCPD:RuisIMesHCCl₃ ratio of (about 10,000:1:2) at a starting temperature of about 88.2 °C. **Result:** Time to reach maximum temperature (Tₘₐₓ) = 205 seconds. Tₘₐₓ = 249.7 °C. Glass transition temperature measured by thermal mechanical analysis (TMA) = 164.77 °C.

### Synthesis of (PCy₃)(C₁₁H₉N)₂(Cl)₂Ru=C=CHPh

Complex **44** (2.0 grams) was dissolved in toluene (10 mL), and 4-phenylpyridine (1.2 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (PCy₃)(C₁₁H₉N)₂(Cl)₂Ru=C=CHPh **52** as an orange powder (0.9 gram, 43% yield).

**Example (1)**: A 75 gram mass of DCPD (containing about 24% trimerized DCPD) was polymerized using (PCy₃)(C₁₁H₉N)₂(Cl)₂Ru=C=CHPh = 0.0451 grams in the presence of sIMesHCCl₃ = 0.0450 grams at a DCPD:Ru:sIMesHCCl₃ ratio of (about 10,000:1:2) at a starting temperature of about 82.9 °C. **Result:** Time to reach maximum temperature (Tₘₐₓ) = 148 seconds. Tₘₐₓ = 242.1 °C. Glass transition temperature measured by thermal mechanical analysis (TMA) = 158.28 °C.

### Example (2):

A 75 g mass of hexylnorbornene, was polymerized using
(PCy₃)(C₁₁H₉N)₂(Cl)₂Ru=C=CHPh = 0.0246 g in the presence of s-ImesHCCl₃ = 0.0240 g at a HₓN:Ru:s-ImesHCCl₃ reactant ratio of (15,000:1:2) at a starting temperature of about 81.7 °C. **Result:** Time to reach maximum temperature (Tₘₐₓ) = 224 seconds. Tₘₐₓ = 193.9 °C.

### Example (3)

A 75 g mass of a monomer mixture, prepared by mixing together 37.5 g of DCPD (containing 24 wt% trimerized DCPD) and 37.5 g of hexylnorbornene, was polymerized using (PCy₃)(C₁₁H₉N)₂(Cl)₂Ru=C=CHPh = 0.0275 g in the presence of s-ImesHCCl₃ = 0.0269 g at a DCPD:Ru:s-ImesHCCl₃ reactant ratio of (15,000:1:2) and HₓN:Ru:s-ImesHCCl₃ reactant ratio of (15,000:1:2), by heating the mixture to a starting temperature of about 82.1 °C. **Result:** Time to reach maximum temperature (Tₘₐₓ) = 180 seconds. Tₘₐₓ = 217.3 °C.

### Synthesis of (PCy₃)(C₅H₅N)₂(Cl)₂Ru=C=CH-C(CH₃)₃

Complex **45** (2.0 grams) was dissolved in toluene (10 mL), and pyridine (0.9 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (PCy₃)(C₅H₅N)₂(Cl)₂Ru=C=CH-C(CH₃)₃ **53** as an orange powder (1.5 gram, 88% yield).

**Example (1):** A 75 gram mass of DCPD (containing about 24% trimerized DCPD) was polymerized using (PCy₃)(C₅H₅N)₂(Cl)₂Ru=C=CH-C(CH₃)₃ = 0.0368 grams in the presence of sIMesHCCl₃ = 0.0446 grams at a DCPD:Ru:sIMesHCCl₃ ratio of (about 10,000:1:2) at a starting temperature of about 82.2 °C. **Result:** Time to reach maximum temperature (Tₘₐₓ) = 76 seconds. Tₘₐₓ = 239.7 °C. Glass transition temperature measured by thermal mechanical analysis (TMA) = 178.83 °C.

**Example (2):** A 75 g mass of hexylnorbornene, was polymerized using (PCy₃)(C₅H₅N)₂(Cl)₂Ru=C=CH-C(CH₃)₃ = 0.0149 g in the presence of s-ImesHCCl₃ = 0.0092 g at a HₓN:Ru:s-ImesHCCl₃ reactant ratio of (20,000:1:1) at a starting temperature of 80.9 °C. **Result:** Time to reach maximum temperature (Tₘₐₓ) = 154 seconds. Tₘₐₓ = 194.5 °C.

**Example (3):** A 75 g mass of a monomer mixture, prepared by mixing together 37.5 g of DCPD (containing 24 wt% trimerized DCPD) and 37.5 g of hexylnorbornene, was polymerized using (PCy₃)(C₅H₅N)₂(Cl)₂Ru=C=CH-C(CH₃)₃ = 0.0163 g in the presence of s-ImesHCCl₃ = 0.0100 g at a DCPD:Ru:s-ImesHCCl₃ reactant ratio of (20,000:1:2) and HₓN:Ru:s-ImesHCCl₃ reactant ratio of (20,000:1:2), by heating the mixture to a starting temperature of about 81.2 °C. **Result:** Time to reach maximum temperature (Tₘₐₓ) = 169 seconds. Tₘₐₓ = 221.3 °C.

### Synthesis of (PCy₃)(C₁₀H₈N₂)₂(Cl)₂Ru=C=CHPh

Complex **44** (2.0 grams) was dissolved in toluene (10 mL), and 4,4'-bipyridine (1.5 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (PCy₃)(C₁₀H₈N₂)₂(Cl)₂Ru=C=CHPh **54** as an orange powder (1.9 gram, 90% yield).

**Example (1):** A 75 gram mass of DCPD (containing about 24% trimerized DCPD) was polymerized using (PCy₃)(C₁₀H₈N₂)₂(Cl)₂Ru=C=CHPh = 0.0462 grams in the presence of sIMesHCCl₃ = 0.0448 grams at a DCPD:Ru:sIMesHCCl₃ ratio of (about 10,000:1:2) at a starting temperature of about 82.3 °C. **Result:** Time to reach maximum temperature (Tₘₐₓ) = 244 seconds. Tₘₐₓ = 230.0 °C. Glass transition temperature measured by thermal mechanical analysis (TMA) = 126.38 °C.

### Synthesis of (PCy₃)(C₁₀H₈N₂)₂(Cl)₂Ru=C=CH-C(CH₃)₃

Complex **45** (2.0 grams) was dissolved in toluene (10 mL), and 4,4'-bipyridine (1.5 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (PCy₃)(C₁₀H₈N₂)₂(Cl)₂Ru=C=CH-C(CH₃)₃ **59** as a brown powder (1.7 gram, 81% yield).

**Example (1):** A 75 gram mass of DCPD (containing about 24% trimerized DCPD) was polymerized using (PCy₃)(C₁₀H₈N₂)₂(Cl)₂Ru=C=CH-C(CH₃)₃ = 0.0447 grams in the presence of sIMesHCCl₃ = 0.0445 grams at a DCPD:Ru:sIMesHCCl₃ ratio of (about 10,000:1:2) at a starting temperature of about 80.8 °C. **Result:** Time to reach maximum temperature (Tₘₐₓ) = 189 seconds. Tₘₐₓ = 208.4 °C. Glass transition temperature measured by thermal mechanical analysis (TMA) = 95.70 °C.

### Synthesis of (PCy₃)(C₁₁H₉N)₂(Cl)₂Ru=C=CH-C(CH₃)₃

Complex **45** (2.0 grams) was dissolved in toluene (10 mL), and 4-phenylpyridine (1.6 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (PCy₃)(C₁₁H₉N)₂(Cl)₂Ru=C=CH-C(CH₃)₃ **60** as a brown powder (1.7 gram, 81% yield).
¹H NMR (300 MHz, C₆D₆): δ = 6.89-10.08 (multiple peaks, 18H), 4.17 (d, 1H, J = 4 Hz, vinylidene), 1.25-2.74 (multiple peaks, 33H), 1.31 (s, 9H) ppm.

**Example (1):** A 75 gram mass of DCPD (containing about 24% trimerized DCPD) was polymerized using (PCy₃)(C₁₁H₉N)₂(Cl)₂Ru=C=CH-C(CH₃)₃ = 0.0445 grams in the presence of sIMesHCCl₃ = 0.0449 grams at a DCPD:Ru:sIMesHCCl₃ ratio of (about 10,000:1:2) at a starting temperature of about 81.1 °C. **Result:** Time to reach maximum temperature (Tₘₐₓ) = 126 seconds. Tₘₐₓ = 246.2 °C. Glass transition temperature measured by thermal mechanical analysis (TMA) = 175.35 °C.

### Synthesis of (IMesH₂)(C₉H₁₂N₂)₂(Cl)₂Ru=C=CH-C(CH₃)₃

Complex **46** (2.0 grams) was dissolved in toluene (10 mL), and 4-pyrrolidinopyridine (1.4 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₉H₁₂N₂)₂(Cl)₂Ru=C=CH-C(CH₃)₃ 64 as a gray powder (0.7 gram, 35% yield).

**Example (1):** A 75 gram mass of DCPD (containing about 24% trimerized DCPD) was polymerized using (IMesH₂)(C₉H₁₂N₂)₂(Cl)₂Ru=C=CH-C(CH₃)₃ = 0.0456 grams at a DCPD:Ru ratio of (about 10,000:1) at a starting temperature of about 80.7 °C. **Result:** Time to reach maximum temperature (Tₘₐₓ) = 143 seconds. Tₘₐₓ = 170.5 °C.

### Synthesis of (IMesH₂)(C₁₀H₈N₂)₂(Cl)₂Ru=C=CH-C(CH₃)₃

Complex **46** (2.0 grams) was dissolved in toluene (10 mL), and 4,4'-bipyridine (1.5 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₁₀H₈N₂)₂(Cl)₂Ru=C=CH-C(CH₃)₃ **65** as a dark purple powder (2.0 gram, 95% yield).

**Example (1):** A 75 g mass of hexylnorbornene was polymerized using (IMesH₂)(C₁₀H₈N₂)₂(Cl)₂Ru=C=CH-C(CH₃)₃ = 0.0488 g at a HₓN:Ru reactant ratio of (7,500:1) at a starting temperature of about 80.6 °C. **Result:** Time to reach maximum temperature (Tₘₐₓ) = 183 seconds. Tₘₐₓ = 191.7 °C.

**Example (2):** A 75 g mass of a monomer mixture, prepared by mixing together 37.5 g of DCPD (containing 24 wt% trimerized DCPD) and 37.5 g of hexylnorbornene, was polymerized using (IMesH₂)(C₁₀H₈N₂)₂(Cl)₂Ru=C=CH-C(CH₃)₃ = 0.0549 g at a DCPD:Ru reactant ratio of (7,500:1) and HₓN:Ru reactant ratio of (7,500:1), by heating the mixture to a starting temperature of about 80.3 °C. **Result:** Time to reach maximum temperature (Tₘₐₓ) = 138 seconds. Tₘₐₓ = 181.9 °C.

### Synthesis of (IMesH₂)(C₁₁H₉N)₂(Cl)₂Ru=C=CH-C(CH₃)₃

Complex **46** (2.0 grams) was dissolved in toluene (10 mL), and 4-phenylpyridine (1.5 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₁₁H₉N)₂(Cl)₂Ru=C=CH-C(CH₃)₃ **66** as a light brown powder (0.6 gram, 29% yield).

### Synthesis of (IMesH₂)(C₅H₅N)₂(Cl)₂Ru=C=CH-C(CH₃)₃

Complex **46** (2.0 grams) was dissolved in toluene (10 mL), and pyridine (0.8 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₅H₅N)₂(Cl)₂Ru=C=CH-C(CH₃)₃ **67** as a yellow powder (0.9 gram, 53% yield).

### Synthesis of (IMesH₂)(C₁₀H₈N₂)(Cl)₂Ru=C=CH-C(CH₃)₃

Complex **46** (2.0 grams) was dissolved in toluene (10 mL), and 2,2'-bipyridine (0.8 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₁₀H₈N₂)(Cl)₂Ru=C=CH-C(CH₃)₃ **68** as a brown powder (0.9 gram, 53% yield).

### Synthesis of (PCy₃)(C₇H₁₀N₂)₂(Cl)₂Ru=C=CH-C(CH₃)₃

Complex **45** (2.0 grams) was dissolved in toluene (10 mL), and 4-dimethylaminopyridine (1.2 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (PCy₃)(C₇H₁₀N₂)₂(Cl)₂Ru=C=CH-C(CH₃)₃ **70** as pink powder (1.6 gram, 84% yield).
¹H NMR (300 MHz, C₆D₆): δ = 5.89-9.66 (multiple peaks, 8H), 4.14 (d, J = 4 Hz, vinylidene), 1.31-2.78 (multiple peaks, 45H), 1.40 (s, 9H) ppm.

**Example (1):** A 75 gram mass of DCPD (containing about 24% trimerized DCPD) was polymerized using (PCy₃)(C₇H₁₀N₂)₂(Cl)₂Ru=C=CH-C(CH₃)₃ = 0.0411 grams in the presence of sIMesHCCl₃ = 0.0450 grams at a DCPD:Ru:sIMesHCCl₃ ratio of (about 10,000:1:2) at a starting temperature of about 81.9 °C. **Result:** Time to reach maximum temperature (Tₘₐₓ) = 161 seconds. Tₘₐₓ = 246.5 °C. Glass transition temperature measured by thermal mechanical analysis (TMA) = 169.56 °C.

### Synthesis of (IMesH₂)(C₇H₁₀N₂)₂(Cl)₂Ru=C=CH-C(CH₃)₃

Complex **46** (2.0 grams) was dissolved in toluene (10 mL), and 4-dimethylaminopyridine (1.2 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₇H₁₀N₂)₂(Cl)₂Ru=C=CH-C(CH₃)₃ **71** as a gray powder (0.9 gram, 47% yield).

### Synthesis of (PCy₃)(C₁₁H₉N)₂(Cl)₂Ru=C=C=C(Ph)₂

Complex **47** (2.0 grams) was dissolved in toluene (10 mL), and 4-phenylpyridine (1.4 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (PCy₃)(C₁₁H₉N)₂(Cl)₂Ru=C=C=C(Ph)₂ **74** as a dark purple powder (1.5 gram, 71% yield).

**Example (1):** A 75 gram mass of DCPD (containing about 24% trimerized DCPD) was polymerized using (PCy₃)(C₁₁H₉N)₂(Cl)₂Ru=C=C=C(Ph)₂ = 0.0499 grams in the presence of sIMesHCCl₃ = 0.0447 grams at a DCPD:Ru:sIMesHCCl₃ ratio of (about 10,000:1:2) at a starting temperature of about 83.8 °C. **Result:** Time to reach maximum temperature (Tₘₐₓ) = 288 seconds. Tₘₐₓ = 238.7 °C. Glass transition temperature measured by thermal mechanical analysis (TMA) = 124.72 °C.

### Example (2)

A 75 g mass of hexylnorbornene was polymerized using
(PCy₃)(C₁₁H₉N)₂(Cl)₂Ru=C=C=C(Ph)₂ = 0.0536 g in the presence of s-ImesHCCl₃ = 0.0478 g at a HₓN:Ru:s-ImesHCCl₃ reactant ratio of (7,500:1:2) at a starting temperature of 80.3 °C. **Result:** Time to reach maximum temperature (Tₘₐₓ) = 230 seconds. Tₘₐₓ = 195.6 °C.

**Example (3):** A 75 g mass of a monomer mixture, prepared by mixing together 37.5 g of DCPD (containing 24 wt% trimerized DCPD) and 37.5 g of hexylnorbornene, was polymerized using (PCy₃)(C₁₁H₉N)₂(Cl)₂Ru=C=C=C(Ph)₂ = 0.0599 g in the presence of s-ImesHCCl₃ = 0.0536 g at a DCPD:Ru:s-ImesHCCl₃ reactant ratio of (7,500:1:2) and HₓN:Ru:s-ImesHCCl₃ reactant ratio of (7,500:1:2), by heating the mixture to a starting temperature of about 82.4 °C. **Result:** Time to reach maximum temperature (Tₘₐₓ) = 178 seconds. Tₘₐₓ = 220.8 °C.

### Synthesis of (PCy₃)(C₁₀H₈N₂)₂(Cl)₂Ru=C=C=C(Ph)₂

Complex **47** (2.0 grams) was dissolved in toluene (10 mL), and 4,4'-bipyridine (1.4 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (PCy₃)(C₁₀H₈N₂)₂(Cl)₂Ru=C=C=C(Ph)₂ **75** as a red-brown powder (2.0 gram, 95% yield).

**Example (1):** A 75 gram mass of DCPD (containing about 24% trimerized DCPD) was polymerized using (PCy₃)(C₁₀H₈N₂)₂(Cl)₂Ru=C=C=C(Ph)₂ = 0.0500 grams in the presence of sIMesHCCl₃ = 0.0448 grams at a DCPD:Ru:sIMesHCCl₃ ratio of (about 10,000:1:2) at a starting temperature of about 84.6 °C. **Result:** Time to reach maximum temperature (Tₘₐₓ) = 190 seconds. Tₘₐₓ = 224.7 °C. Glass transition temperature measured by thermal mechanical analysis (TMA) = 105.52 °C.

### Synthesis of (IMesH₂)(C₅H₅N)₂(Cl)₂Ru=C=C=C(Ph)₂

Complex **48** (2.0 grams) was dissolved in toluene (10 mL), and pyridine (0.7 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₅H₅N)₂(Cl)₂Ru=C=C=C(Ph)₂ **78** as a red-brown powder (0.9 gram, 53% yield).
¹H NMR (300 MHz, C₆D₆): δ = 6.52-8.09 (multiple peaks, 20H), 4.00 (s, 4H, sIMes)1.00-2.28 (multiple peaks, 18H) ppm.

### Synthesis of (IMesH₂)(C₇H₁₀N₂)₂(Cl)₂Ru=C=C=C(Ph)₂

Complex **48** (2.0 grams) was dissolved in toluene (10 mL), and 4-dimethylaminopyridine (1.0 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₇H₁₀N₂)₂(Cl)₂Ru=C=C=C(Ph)₂ **79** as a red-brown powder (1.0 gram, 53% yield).

### Synthesis of (IMesH₂)(C₁₂H₈N₂)(Cl)₂Ru=C=C=C(Ph)₂

Complex **48** (2.0 grams) was dissolved in toluene (10 mL), and 1,10-phenanthroline (0.8 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₁₂H₈N₂)(Cl)₂Ru=C=C=C(Ph)₂ **80** as a red powder (0.6 gram, 33% yield).

### Synthesis of (IMesH₂)(C₁₁H₉N)₂(Cl)₂Ru=C=C=C(Ph)₂

Complex **48** (2.0 grams) was dissolved in toluene (10 mL), and 4-phenylpyridine (1.3 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₁₁H₉N)₂(Cl)₂Ru=C=C=C(Ph)₂ **81** as a brown powder (1.1 gram, 52% yield).

**Example (1):** A 75 gram mass of DCPD (containing about 24% trimerized DCPD) was polymerized using (IMesH₂)(C₁₁H₉N)₂(Cl)₂Ru=C=C=C(Ph)₂ = 0.0515 grams at a DCPD:Ru ratio of (about 10,000:1) at a starting temperature of about 80.9 °C. **Result:** Time to reach maximum temperature (Tₘₐₓ) = 275 seconds. Tₘₐₓ = 118.2 °C.

### Synthesis of (IMesH₂)(C₁₀H₈N₂)₂(Cl)₂Ru=C=C=C(Ph)₂

Complex **48** (2.0 grams) was dissolved in toluene (10 mL), and 4,4'-bipyridine (1.3 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₁₀H₈N₂)₂(Cl)₂Ru=C=C=C(Ph)₂ **82** as a brown powder (1.9 gram, 90% yield).

**Example (1):** A 75 gram mass of DCPD (containing about 24% trimerized DCPD) was polymerized using (IMesH₂)(C₁₀H₈N₂)₂(Cl)₂Ru=C=C=C(Ph)₂ = 0.0512 grams at a DCPD:Ru ratio of (about 10,000:1) at a starting temperature of about 80.1 °C. **Result:** Time to reach maximum temperature (Tₘₐₓ) = 144 seconds. Tₘₐₓ = 138.8 °C.

**Example (2):** A 75 g mass of hexylnorbornene was polymerized using (IMesH₂)(C₁₀H₈N₂)₂(Cl)₂Ru=C=C=C(Ph)₂ = 0.0552 g at a HₓN:Ru reactant ratio of (7,500:1) at a starting temperature of about 80.3 °C. **Result:** Time to reach maximum temperature (Tₘₐₓ) = 578 seconds. Tₘₐₓ = 138.5 °C.

**Example (3):** A 75 g mass of a monomer mixture, prepared by mixing together 37.5 g of DCPD (containing 24 wt% trimerized DCPD) and 37.5 g of hexylnorbornene, was polymerized using (IMesH₂)(C₁₀H₈N₂)₂(Cl)₂Ru=C=C=C(Ph)₂ = 0.0617 g at a DCPD:Ru reactant ratio of (7,500:1) and HₓN:Ru reactant ratio of (7,500:1), by heating the mixture to a starting temperature of about 80.6 °C. **Result:** Time to reach maximum temperature (Tₘₐₓ) = 259 seconds. Tₘₐₓ = 135.7 °C.

### Synthesis of (IMesH₂)(C₁₀H₈N₂)(Cl)₂Ru=C=C=C(Ph)₂

Complex **48** (2.0 grams) was dissolved in toluene (10 mL), and 2,2'-bipyridine (0.7 grams) was added. The reaction flask was purged with argon and the reaction mixture was stirred for approximately 12 hours at 20 °C to 25 °C. After approximately 12 hours the reaction mixture was transferred into 75 mL of cold (about 0 °C) pentane. The pentane mixture was filtered, washed with 4 x 20 mL of cold pentane, and dried under vacuum to afford (IMesH₂)(C₁₀H₈N₂)(Cl)₂Ru=C=C=C(Ph)₂ **83** as a red-brown powder (1.3 gram, 76% yield).
¹H NMR (300 MHz, C₆D₆): δ = 6.60-7.85 (multiple peaks, 18H), 4.00 (s, 4H, sIMes)1.08-2.60 (multiple peaks, 18H) ppm.

## Claims

1. A compound of the formula: 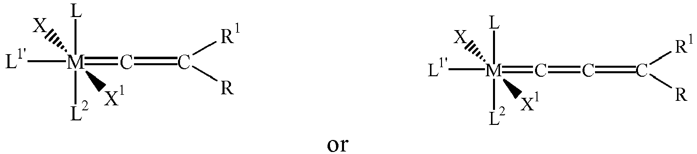 wherein
M is ruthenium or osmium;
X and X¹ are the same or different and are each independently any anionic ligand;
L, L^{1'}, and L² are the same or different and are each independently any neutral electron donor ligand, wherein at least one is an N-heterocyclic carbene ligand;
R and R¹ are the same or different and are each independently hydrogen or a substituted or unsubstituted substituent selected from C₁-C₂₀ alkyl, C₂-C₂₀ alkenyl, C₂-C₂₀ alkynyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl, C₁-C₂₀ alkylsulfinyl, and silyl.

2. A compound of Claim 1, wherein at least one of the R and R¹ substituent group is substituted with one or more moieties selected from C¹-C¹⁰ alkyl, C¹-C¹⁰ alkoxy, and aryl, and wherein the moiety is substituted or unsubstituted.

3. A compound of Claim 2, wherein the moiety is substituted with one or more groups selected from halogen, a C₁-C₅ alkyl, C₁-C₅ alkoxy, and phenyl.

4. A compound of of claim 1, wherein R is hydrogen and R¹ is selected from C₁-C₂₀ alkyl, C₂-C₂₀ alkenyl, and aryl.

5. A compound of Claim 4, wherein R¹ is phenyl or vinyl.

6. A compound of of claim 1 wherein X and X¹ are each independently hydrogen, halide, or selected from C₁-C₂₀ alkyl, aryl, C₁-C₂₀ alkoxide, aryloxide, C₃-C₂₀ alkyldiketonate, aryldiketonate, C₁-C₂₀ carboxylate, arylsulfonate, C₁-C₂₀ alkylsulfonate, C₁-C₂₀ alkylthio, C₁-C₂₀ alkylsulfonyl, or C₁-C₂₀ alkylsulfinyl, wherein X and X¹ is each independently substituted or unsubstituted.

7. A compound of Claim 6, wherein at least one of X and X¹ is substituted with one or more moieties selected from C₁-C₁₀ alkyl, C₁-C₁₀ alkoxy, and aryl, wherein the moiety is substituted or unsubstituted.

8. A compound of Claim 7, wherein the moiety is substituted with one or more groups selected from halogen, C₁-C₅ alkyl, C₁-C₅ alkoxy, and phenyl.

9. A compound of Claim 6, wherein X and X¹ are each independently selected from halide, benzoate, C₁-C₅ carboxylate, C₁-C₅ alkyl, phenoxy, C₁-C₅ alkoxy, C₁-C₅ alkylthio, aryl, and C₁-C₅ alkyl sulfonate.

10. A compound of Claim 9, wherein X and X¹ are each independently selected from halide, CF₃CO₂, CH₃CO₂, CFH₂CO₂, (CH₃)₃CO, (CF₃)₂(CH₃)CO, (CF₃)(CH₃)₂CO, PhO, MeO, EtO, tosylate, mesylate, or trifluoromethanesulfonate.

11. A compound of claim 1, wherein, subject to the requirement of Claim 1, L, L^{1'} and L² are each independently selected from a monodentate, bidentate or tetradentate neutral electron donor ligand.

12. A compound of Claim 11, wherein, subject to the requirement of Claim 1, L, L^{1'} and L² are each independently selected from phosphine, sulfonated phosphine, phosphite, phosphinite, phosphonite, arsine, stibine, ether, amine, amide, imine, sulfoxide, carboxyl, nitrosyl, pyridine, and thioether, and derivatives therefrom and N-heterocyclic carbene ligands.

13. A compound of Claim 1, wherein both L^{1'} and L² are either the same or different N-heterocyclic carbene ligands.

14. A compound of Claim 1, wherein L is an N-heterocyclic carbene ligand and L^{1'} and L² are each heterocyclic ligands.

15. A compound of Claim 14, wherein at least one of L^{1'} and L² is aromatic.

16. A compound of Claim 14, wherein L^{1'} and L² together form a bidentate ligand.

17. A compound of claim 1, wherein the N-heterocyclic carbene ligand is selected from: 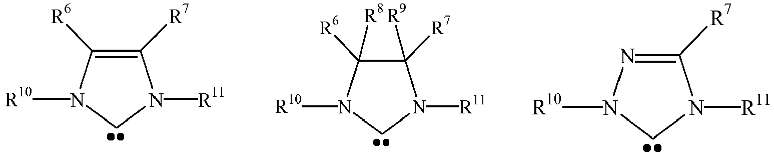 wherein R, R¹, R⁶, R⁷, R⁸, R⁹, R¹⁰, and R¹¹ are each independently hydrogen or a substituted or unsubstituted substituent selected from C₁-C₂₀ alkyl, C₂-C₂₀ alkenyl, C₂-C₂₀ alkynyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl, C₁-C₂₀ alkylsulfinyl and silyl.

18. A compound of any one of Claims 1 wherein, subject to the requirement of Claim 1, at least one of L^{1'} and L² is a unsubstituted or substituted heteroarene selected from furan, thiophene, pyrrole, pyridine, bipyridine, picolylimine, gamma-pyran, gamma-thiopyran, phenanthroline, pyrimidine, bipyrimidine, pyrazine, indole, coumarone, thionaphthene, carbazole, dibenzofuran, dibenzothiophene, pyrazole, imidazole, benzimidazole, oxazole, thiazole, dithiazole, isoxazole, isothiazole, quinoline, bisquinoline, isoquinoline, bisisoquinoline, acridine, chromene, phenazine, phenoxazine, phenothiazine, triazine, thianthrene, purine, bisimidazole and bisoxazole.

19. A compound of Claim 18, wherein at least one of L^{1'} and L² is a substituted or unsubstituted pyridine or a substituted or unsubstituted pyridine derivative.

20. A compound of Claim 18, wherein L is an imidazolidine ligand and L^{1'} and L² are both pyridine.

21. A compound of Claim 18, wherein the substituted or unsubstituted heteroarene is selected from: 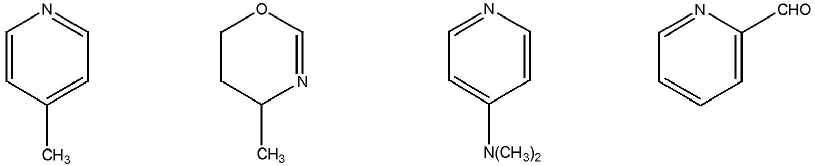 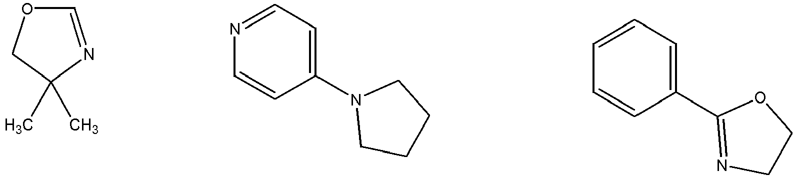 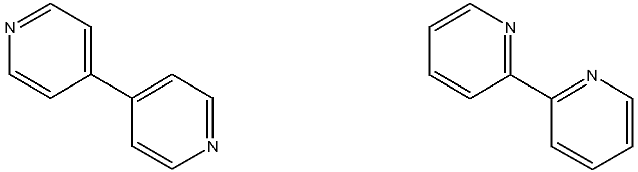 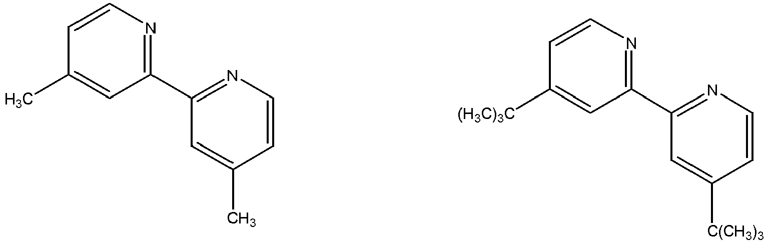 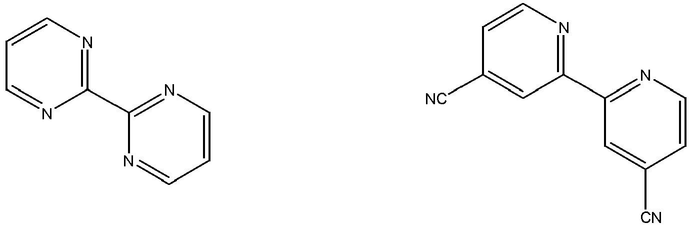 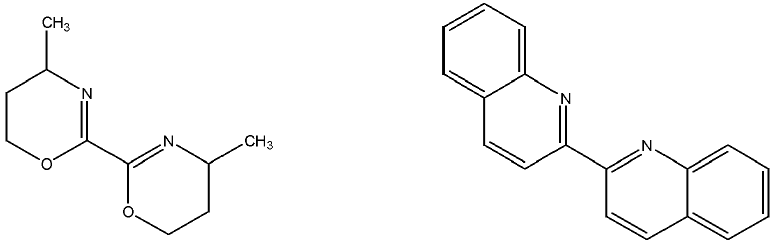 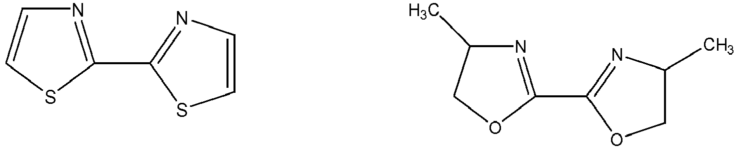 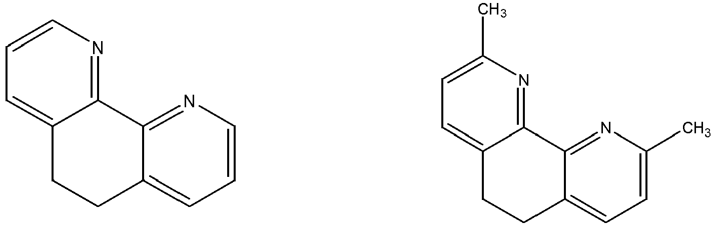 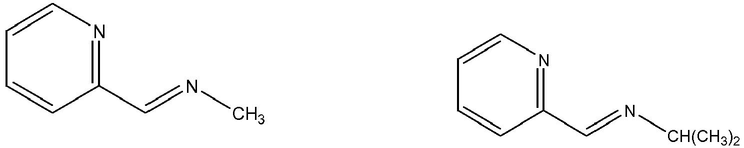 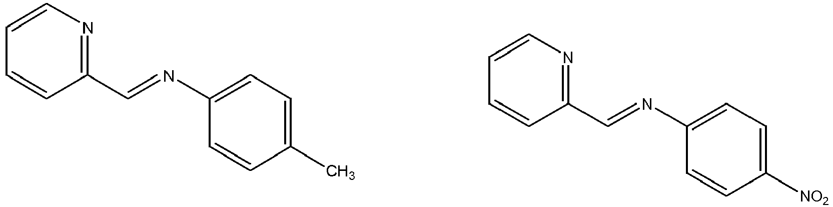 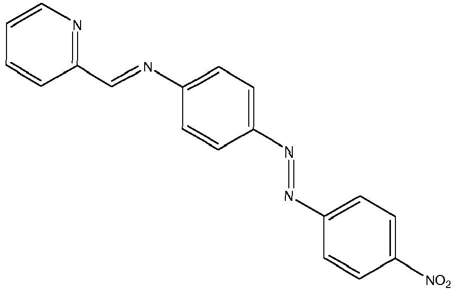 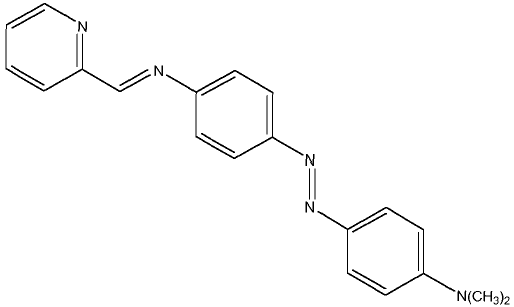 and

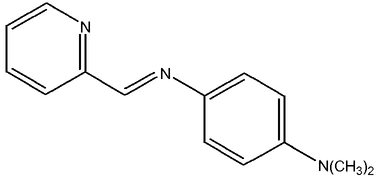

22. A compound of any one of claim 1 wherein, subject to the requirement of Claim 1, at least one of L^{1'} and L² is a unsubstituted or substituted heterocycle selected from: 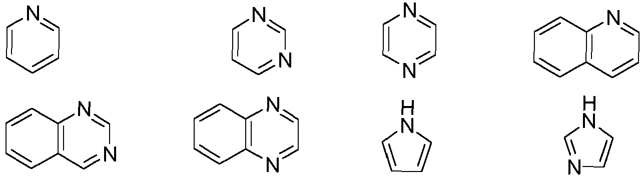 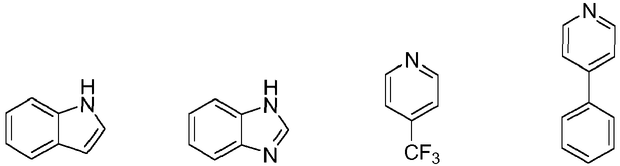 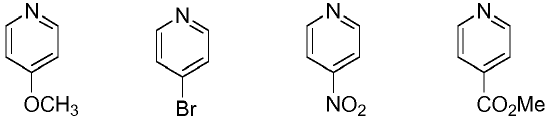 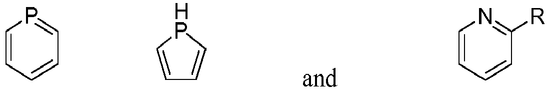 wherein R is selected from C₁-C₂₀ alkyl, aryl, ether, amide, halide, nitro, ester, pyridyl.

23. A compound of Claim 1, wherein
M is ruthenium;
X and X¹ are each independently selected from halide, CF₃CO₂, CH₃CO₂, CFH₂CO₂, (CH₃)₃CO, (CF₃)₂(CH₃)CO, (CF₃)(CH₃)₂CO, PhO, MeO, EtO, tosylate, mesylate, or trifluoromethanesulfonate;
L is a N-heterocyclic carbene ligand;
L^{1'} and L² are the same or different and are each a substituted or unsubstituted heteroarene, and wherein L¹ and L^{1'} may be joined.
R is hydrogen and R¹ is selected from C₁-C₂₀ alkyl, C₂-C₂₀ alkenyl, and aryl.

24. A compound of Claim 23, wherein X and X¹ are each Cl, L is (IMesH₂), L^{1'} and L² are each independently a pyridine or pyridine derivative; R is hydrogen and R¹ is phenyl or vinyl.

25. A compound of Claim 1 selected from: 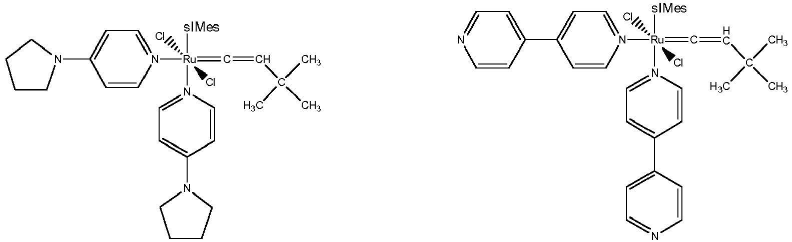 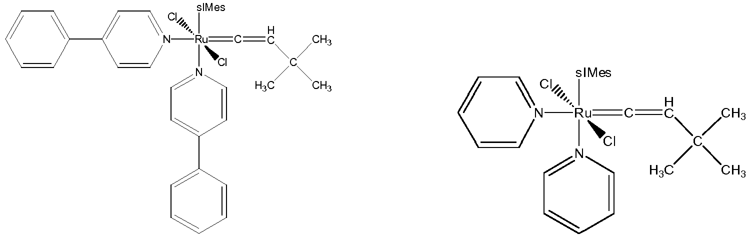 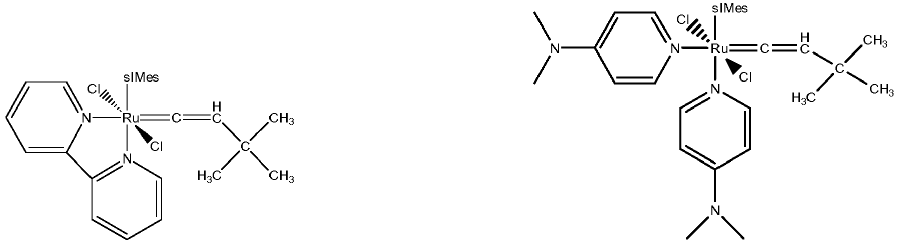 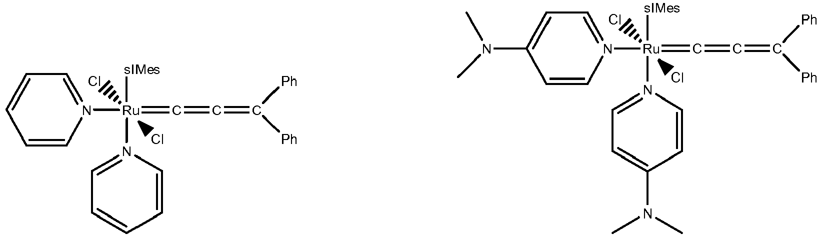 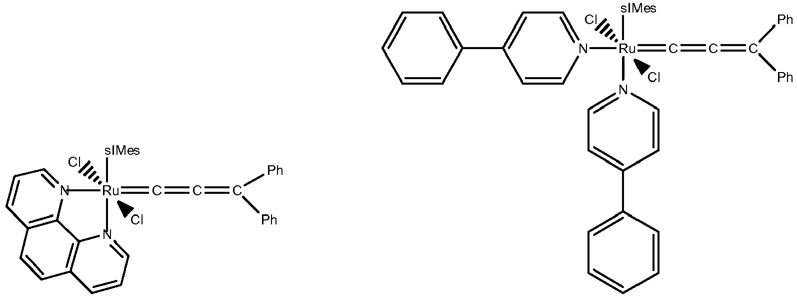 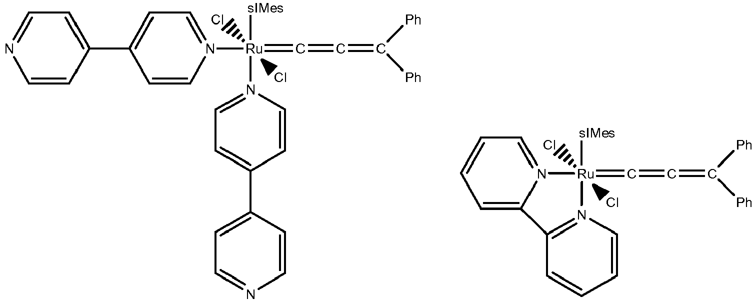 wherein sIMES or IMesH₂ is

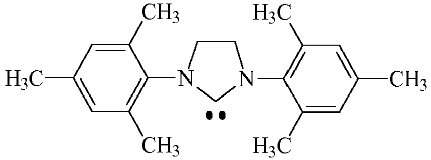

26. A compound of Claim 1, wherein
M is ruthenium;
X and X¹ are each independently selected from halide, CF₃CO₂, CH₃CO₂, CFH₂CO₂, (CH₃)₃CO, (CF₃)₂(CH₃)CO, (CF₃)(CH₃)₂CO, PhO, MeO, EtO, tosylate, mesylate, or trifluoromethanesulfonate;
L is an N-heterocyclic carbene ligand;
L¹ and L² are the same or different and are each a substituted or unsubstituted heteroarene;
R is hydrogen and R¹ is selected from C₁-C₂₀ alkyl, C₂-C₂₀ alkenyl, and aryl.

27. A compound of Claim 26, wherein X and X¹ are each Cl, L is (IMesH₂), L^{1'} and L² are each independently a pyridine or pyridine derivative; R is hydrogen and R¹ is phenyl or vinyl.

28. A compound of Claim 1, wherein
X and X¹ are each independently selected from halide, CF₃CO₂, CH₃CO₂, CFH₂CO₂, (CH₃)₃CO, (CF₃)₂(CH₃)CO, (CF₃)(CH₃)₂CO, PhO, MeO, EtO, tosylate, mesylate, or trifluoromethanesulfonate;
L is a N-heterocyclic carbene ligand;
L^{1'} and L² are the same or different and are each a substituted or unsubstituted heteroarene;
R is hydrogen and R¹ is selected from C₁-C₂₀ alkyl, C₂-C₂₀ alkenyl, and aryl.

29. A method for the metathesis of a cyclic or acyclic olefin comprising contacting the olefin with a compound as defined in any one of the preceding claims.

30. A method for the ring-opening metathesis polymerization of a cyclic olefin comprising contacting the olefin with a compound as defined in Claim 28.

31. A method for the synthesis of a ruthenium or osmium hexacoordinated metathesis catalyst of Claim 1 comprising contacting a ruthenium or osmium pentacoordinated metal carbene metathesis catalyst with a neutral electron donor ligand.

32. A method for the synthesis of a ruthenium or osmium hexacoordinated metathesis catalyst of Claim 1 comprising ligand exchange in which the N-heterocyclic carbene ligand replaces another neutral donor ligand in a corresponding ruthenium or osmium hexacoordinated complex.

33. A method for polymerizing an olefin comprising contacting a compound of the formula: 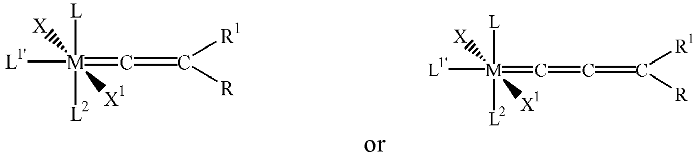 with a compound of the formula: 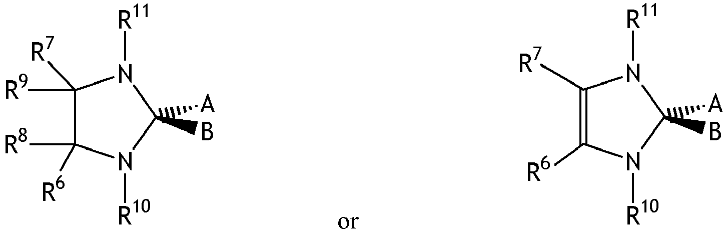 and a cyclic or acyclic olefin;
wherein
M is ruthenium or osmium;
X and X¹ are the same or different and are each independently any anionic ligand;
L, L^{1'}, and L² are the same or different and are each independently any neutral electron donor ligand;
R, R¹, R⁶, R⁷, R⁸, R⁹, R¹⁰ and R¹¹ are the same or different and are each independently hydrogen or a substituted or unsubstituted substituent selected from C₁-C₂₀ alkyl, C₂-C₂₀ alkenyl, C₂-C₂₀ alkynyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl and C₁-C₂₀ alkylsulfinyl and silyl; and
A is hydrogen, Si, Sn, Li, Na, MgX³ and acyl, wherein X³ is any halogen and
B is selected from CCl₃; CH₂SO₂Ph; C₆F₅; OR²¹; and N(R²²)(R²³), wherein R²¹ is selected from Me, C₂H₅, i-C₃H₇, CH₂CMe₃, CMe₃, C₆H₁₁ (cyclohexyl), CH₂Ph, CH₂norbornyl, CH₂norbornenyl, C₆H₅, 2,4,6-(CH₃)₃C₆H₂ (mesityl), 2,6-i-Pr₂C₆H₂, 4-Me-C₆H₄ (tolyl), 4-Cl-C₆H₄; and wherein R²² and R²³ are each independently selected from Me, C₂H₅, i-C₃H₇, CH₂CMe₃, CMe₃, C₆H₁₁ (cyclohexyl), CH₂Ph, CH₂norbornyl, CH₂norbornenyl, C₆H₅, 2,4,6-(CH₃)₃C₆H₂ (mesityl), 2,6-i-Pr₂C₆H₂, 4-Me-C₆H₄ (tolyl), and 4-Cl-C₆H₄.

34. A method of Claim 33, wherein
M is ruthenium;
X and X¹ are each independently selected from halide, CF₃CO₂, CH₃CO₂, CFH₂CO₂, (CH₃)₃CO, (CF₃)₂(CH₃)CO, (CF₃)(CH₃)₂CO, PhO, MeO, EtO, tosylate, mesylate, or trifluoromethanesulfonate;
L is any neutral electron donor ligand;
L^{1'} and L² are the same or different and are each a substituted or unsubstituted heteroarene;
R is hydrogen and R¹, R⁶, R⁷, R⁸, R⁹, R¹⁰, R¹¹ are each independently selected from hydrogen, C₁-C₂₀ alkyl, C₂-C₂₀ alkenyl, and aryl;
A is hydrogen and
B is CCl₃.

35. A method of Claim 33 wherein the olefin is a substituted or unsubstituted norbornene or a norbornene-type monomer or derivative therefrom.

36. A method of Claim 33 wherein the olefin is substituted or unsubstituted dicyclopentadiene.

37. A method of Claim 33 wherein the olefin is a mixture of one or more substituted or unsubstituted norbornenes or norbornene-type monomers or derivatives therefrom.

## Patentansprüche

1. Verbindung der Formel: 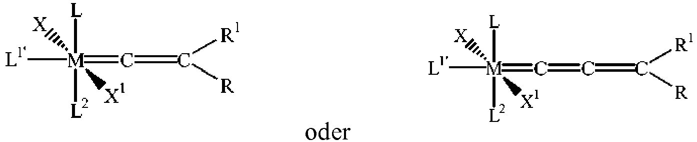 wobei
M für Ruthenium oder Osmium steht;
X und X¹ gleich oder verschieden sind und jeweils unabhängig voneinander für einen beliebigen anionischen Liganden stehen;
L, L^{1'} und L² gleich oder verschieden sind und jeweils unabhängig voneinander für einen beliebigen neutralen Elektronendonorliganden stehen, wobei wenigstens eines ein N-heterocyclischer Carbenligand ist;
R und R¹ gleich oder verschieden sind und jeweils unabhängig voneinander für Wasserstoff oder einen substituierten oder unsubstituierten Substituenten, der aus C₁-C₂₀-Alkyl, C₂-C₂₀-Alkenyl, C₂-C₂₀-Alkinyl, Aryl, C₁-C₂₀-Carboxylat, C₁-C₂₀-Alkoxy, C₂-C₂₀-Alkenyloxy, C₂-C₂₀-Alkinyloxy, Aryloxy, C₂-C₂₀-Alkoxycarbonyl, C₁-C₂₀-Alkylthio, C₁-C₂₀-Alkylsulfonyl, C₁-C₂₀ -Alkylsulfinyl und Silyl ausgewählt ist, stehen.

2. Verbindung nach Anspruch 1, wobei wenigstens eine der R- und R¹-Substituentengruppen durch eine oder mehrere Einheiten, die aus C₁-C₁₀-Alkyl, C₁-C₁₀-Alkoxy und Aryl ausgewählt sind, substituiert ist und wobei die Einheit substituiert oder unsubstituiert ist.

3. Verbindung nach Anspruch 2, wobei die Einheit durch eine oder mehrere Gruppen, die aus Halogen, einem C₁-C₅-Alkyl, C₁-C₅-Alkoxy und Phenyl ausgewählt sind, substituiert ist.

4. Verbindung nach Anspruch 1, wobei R für Wasserstoff steht und R¹ aus C₁-C₂₀-Alkyl, C₂-C₂₀-Alkenyl und Aryl ausgewählt ist.

5. Verbindung nach Anspruch 4, wobei R¹ für Phenyl oder Vinyl steht.

6. Verbindung nach Anspruch 1, wobei X und X¹ jeweils unabhängig voneinander für Wasserstoff oder Halogenid stehen oder aus C₁-C₂₀-Alkyl, Aryl, C₁-C₂₀-Alkoxid, Aryloxid, C₃-C₂₀-Alkyldiketonat, Aryldiketonat, C₁-C₂₀-Carboxylat, Arylsulfonat, C₁-C₂₀-Alkylsulfonat, C₁-C₂₀-Alkylthio, C₁-C₂₀-Alkylsulfonyl oder C₁-C₂₀-Alkylsulfinyl ausgewählt sind, wobei X und X¹ jeweils unabhängig voneinander substituiert oder unsubstituiert sind.

7. Verbindung nach Anspruch 6, wobei wenigstens eines von X und X¹ durch eine oder mehrere Einheiten, die aus C₁-C₁₀-Alkyl, C₁-C₁₀-Alkoxy und Aryl ausgewählt sind, substituiert ist, wobei die Einheit substituiert oder unsubstituiert ist.

8. Verbindung nach Anspruch 7, wobei die Einheit durch eine oder mehrere Gruppen, die aus Halogen, C₁-C₅-Alkyl, C₁-C₅-Alkoxy und Phenyl ausgewählt sind, substituiert ist.

9. Verbindung nach Anspruch 6, wobei X und X¹ jeweils unabhängig voneinander aus Halogenid, Benzoat, C₁-C₅Carboxylat, C₁-C₅-Alkyl, Phenoxy, C₁-C₅-Alkoxy, C₁-C₅-Alkylthio, Aryl und C₁-C₅-Alkylsulfonat ausgewählt sind.

10. Verbindung nach Anspruch 9, wobei X und X¹ jeweils unabhängig voneinander aus Halogenid, CF₃CO₂, CH₃CO₂, CFH₂CO₂, (CH₃)₃CO, (CF₃)₂(CH₃)CO, (CF₃)(CH₃)₂CO, PhO, MeO, EtO, Tosylat, Mesylat oder Trifluormethansulfonat ausgewählt sind.

11. Verbindung nach Anspruch 1, wobei unter der Maßgabe von Anspruch 1 L, L^{1'} und L² jeweils unabhängig voneinander aus einem monodentaten, bidentaten oder tetradentaten neutralen Elektronendonorliganden ausgewählt sind.

12. Verbindung nach Anspruch 11, wobei unter der Maßgabe von Anspruch 1 L, L^{1'} und L² jeweils unabhängig voneinander aus Phosphin, sulfoniertem Phosphin, Phosphit, Phosphinit, Phosphonit, Arsin, Stibin, Ether, Amin, Amid, Imin, Sulfoxid, Carboxyl, Nitrosyl, Pyridin und Thioether sowie Derivaten davon und N-heterocyclischen Carbenliganden ausgewählt sind.

13. Verbindung nach Anspruch 1, wobei sowohl L^{1'} als auch L² entweder für den gleichen oder für verschiedene N-heterocyclische Carbenliganden stehen.

14. Verbindung nach Anspruch 1, wobei L für einen N-heterocyclischen Carbenliganden steht und L^{1'} und L² jeweils für heterocyclische Liganden stehen.

15. Verbindung nach Anspruch 14, wobei wenigstens eines von L¹ und L² aromatisch ist.

16. Verbindung nach Anspruch 14, wobei L^{1'} und L² zusammen einen bidentaten Liganden bilden.

17. Verbindung nach Anspruch 1, wobei der N-heterocyclische Carbenligand ausgewählt ist aus: 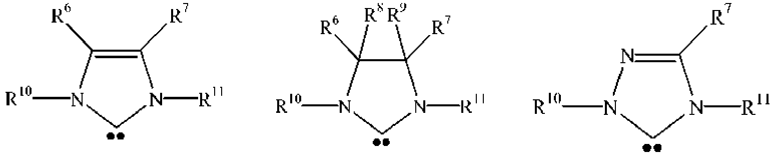 wobei R, R¹, R⁶, R⁷, R⁸, R⁹, R¹⁰ und R¹¹ jeweils unabhängig voneinander für Wasserstoff oder einen substituierten oder unsubstituierten Substituenten, der aus C₁-C₂₀-Alkyl, C₂-C₂₀-Alkenyl, C₂-C₂₀-Alkinyl, Aryl, C₁-C₂₀-Carboxylat, C₁-C₂₀-Alkoxy, C₂-C₂₀-Alkenyloxy, C₂-C₂₀-Alkinyloxy, Aryloxy, C₂-C₂₀-Alkoxycarbonyl, C₁-C₂₀-Alkylthio, C₁-C₂₀-Alkylsulfonyl, C₁-C₂₀-Alkylsulfinyl und Silyl ausgewählt ist, stehen.

18. Verbindung nach Anspruch 1, wobei unter der Maßgabe von Anspruch 1 wenigstens eines von L^{1'} und L² für ein unsubstituiertes oder substituiertes Heteroaren steht, das aus Furan, Thiophen, Pyrrol, Pyridin, Bipyridin, Picolylimin, gamma-Pyran, gamma-Thiopyran, Phenanthrolin, Pyrimidin, Bipyrimidin, Pyrazin, Indol, Cumaron, Thionaphthen, Carbazol, Dibenzofuran, Dibenzothiophen, Pyrazol, Imidazol, Benzimidazol, Oxazol, Thiazol, Dithiazol, Isoxazol, Isothiazol, Chinolin, Bischinolin, Isochinolin, Bisisochinolin, Acridin, Chromen, Phenazin, Phenoxazin, Phenothiazin, Triazin, Thianthren, Purin, Bisimidazol und Bisoxazol ausgewählt ist.

19. Verbindung nach Anspruch 18, wobei wenigstens eines von L^{1'} und L² für ein substituiertes oder unsubstituiertes Pyridin oder ein substituiertes oder unsubstituiertes Pyridinderivat steht.

20. Verbindung nach Anspruch 18, wobei L für einen Imidazolidinliganden steht und L^{1'} und L² jeweils für Pyridin stehen.

21. Verbindung nach Anspruch 18, wobei das substituierte oder unsubstituierte Heteroaren ausgewählt ist aus: 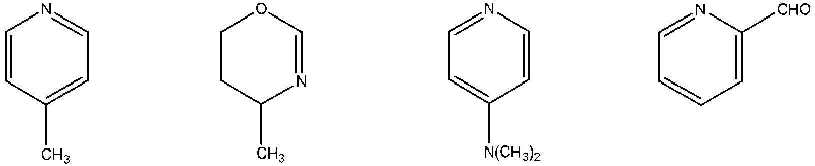 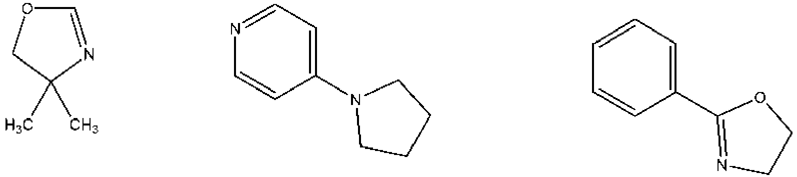 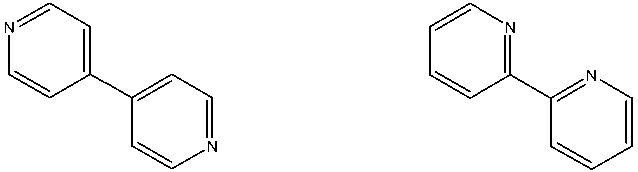 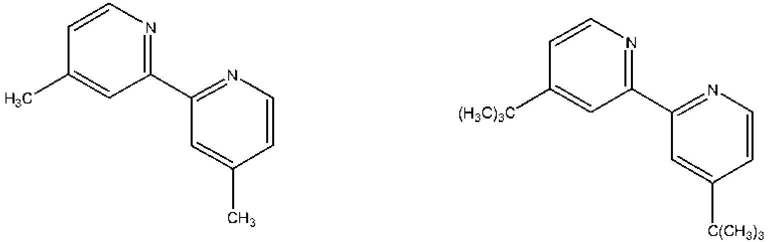 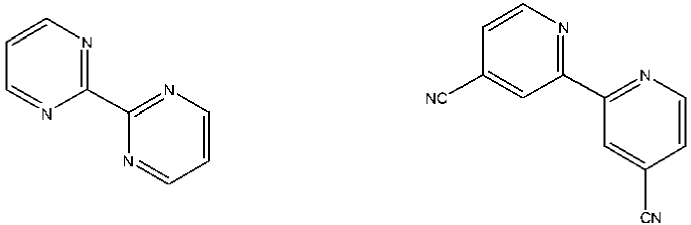 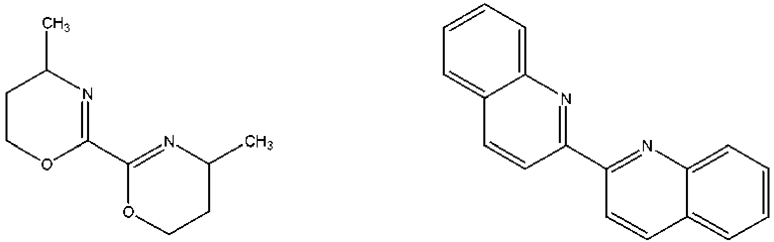 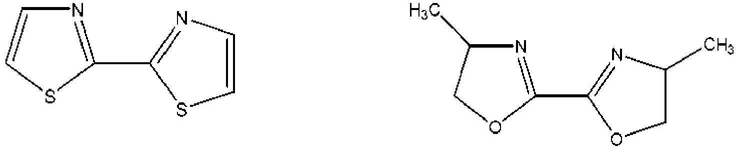 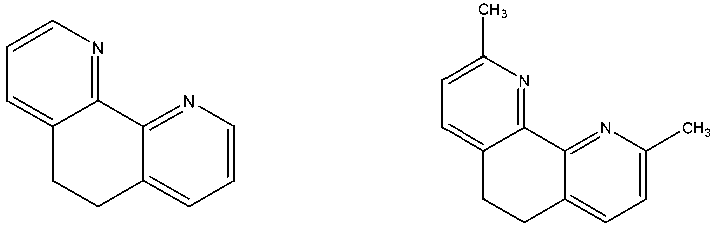 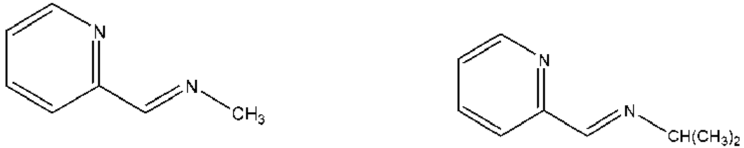 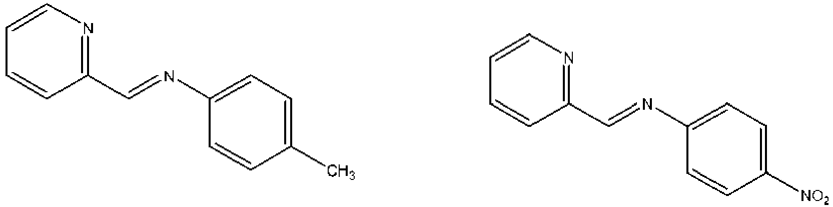 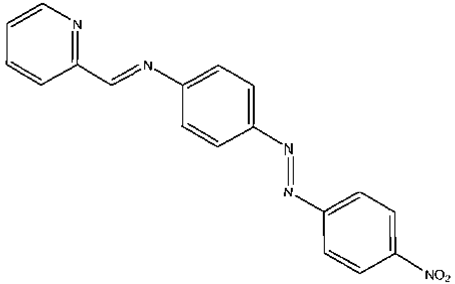 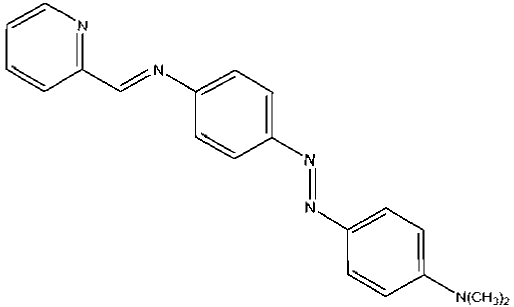 und

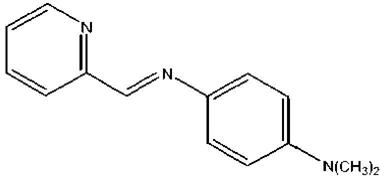

22. Verbindung nach Anspruch 1, wobei unter der Maßgabe von Anspruch 1 wenigstens eines von L^{1'} und L² für einen unsubstituierten oder substituierten Heterocyclus steht, der ausgewählt ist aus: 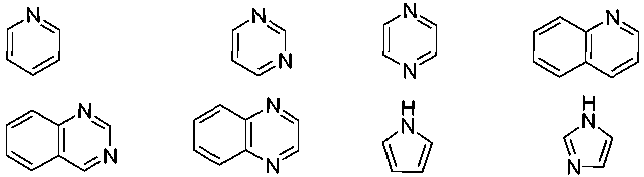 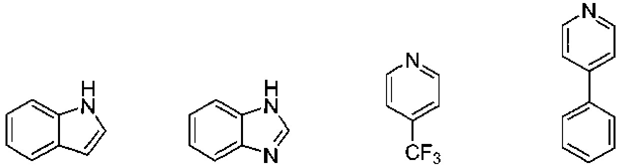 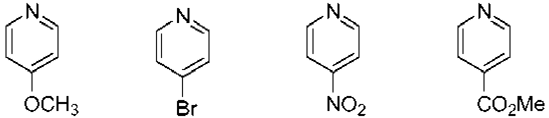 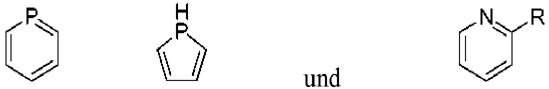 wobei R aus C₁-C₂₀-Alkyl, Aryl, Ether, Amid, Halogenid, Nitro, Ester und Pyridyl ausgewählt ist.

23. Verbindung nach Anspruch 1, wobei
M für Ruthenium steht;
X und X¹ jeweils unabhängig voneinander aus Halogenid, CF₃CO₂, CH₃CO₂, CFH₂CO₂, (CH₃)₃CO, (CF₃)₂(CH₃)CO, (CF₃)(CH₃)₂CO, PhO, MeO, EtO, Tosylat, Mesylat oder Trifluormethansulfonat ausgewählt sind;
L für einen N-heterocyclischen Carbenliganden steht;
L^{1'} und L² gleich oder verschieden sind und jeweils für ein substituiertes oder unsubstituiertes Heteroaren stehen und wobei L^{1'} und L^{1'} verbunden sein können.
R für Wasserstoff steht und R¹ aus C₁-C₂₀-Alkyl, C₂-C₂₀-Alkenyl und Aryl ausgewählt ist.

24. Verbindung nach Anspruch 23, wobei X und X¹ jeweils für Cl stehen, L für (IMesH₂) steht, L^{1'} und L² jeweils unabhängig voneinander für ein Pyridin oder Pyridinderivat stehen; R für Wasserstoff steht und R¹ für Phenyl oder Vinyl steht.

25. Verbindung nach Anspruch 1, ausgewählt aus: 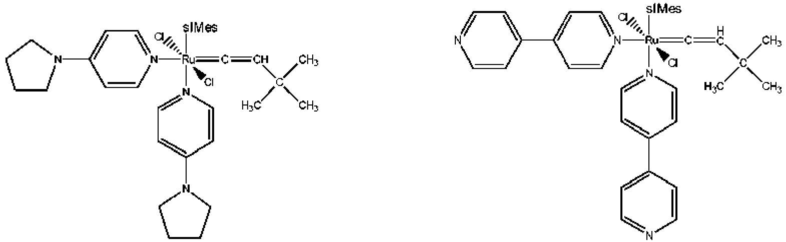 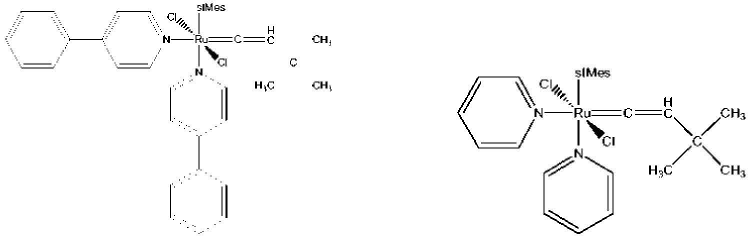 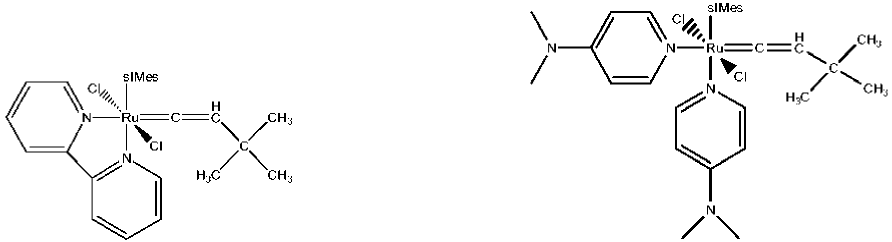 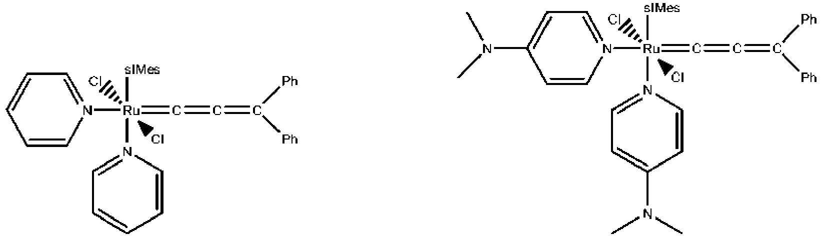 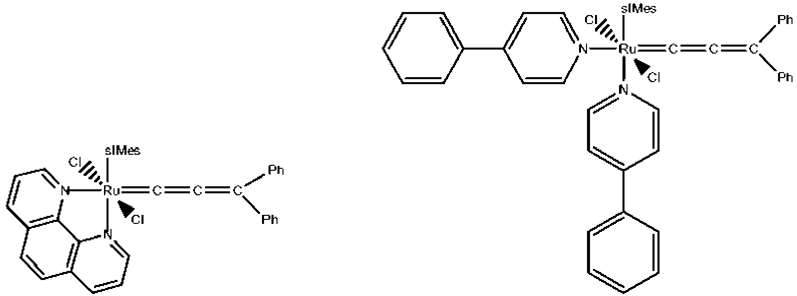 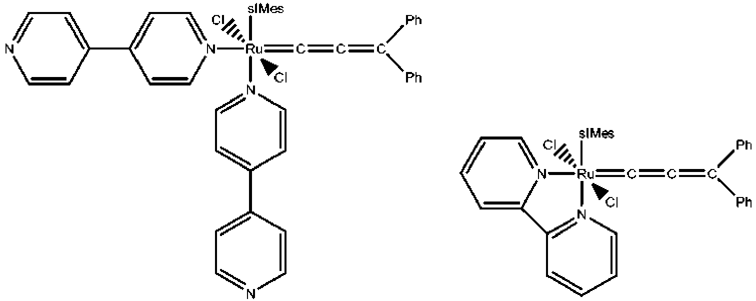 wobei sIMES oder IMesH₂ für Folgendes stehen:

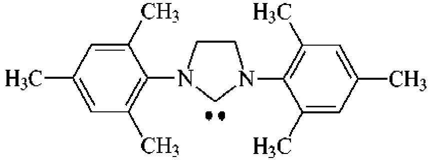

26. Verbindung nach Anspruch 1, wobei
M für Ruthenium steht;
X und X¹ jeweils unabhängig voneinander aus Halogenid, CF₃CO₂, CH₃CO₂, CFH₂CO₂, (CH₃)₃CO, (CF₃)₂(CH₃)CO, (CF₃)(CH₃)₂CO, PhO, MeO, EtO, Tosylat, Mesylat oder Trifluormethansulfonat ausgewählt sind;
L für einen N-heterocyclischen Carbenliganden steht;
L^{1'} und L² gleich oder verschieden sind und jeweils für ein substituiertes oder unsubstituiertes Heteroaren stehen;
R für Wasserstoff steht und R¹ aus C₁-C₂₀-Alkyl, C₂-C₂₀-Alkenyl und Aryl ausgewählt ist.

27. Verbindung nach Anspruch 26, wobei X und X¹ jeweils für Cl stehen, L für (IMesH₂) steht, L^{1'} und L² jeweils unabhängig voneinander für ein Pyridin oder Pyridinderivat stehen; R für Wasserstoff steht und R¹ für Phenyl oder Vinyl steht.

28. Verbindung nach Anspruch 1, wobei
X und X¹ jeweils unabhängig voneinander aus Halogenid, CF₃CO₂, CH₃CO₂, CFH₂CO₂, (CH₃)₃CO, (CF₃)₂(CH₃)CO, (CF₃)(CH₃)₂CO, PhO, MeO, EtO, Tosylat, Mesylat oder Trifluormethansulfonat ausgewählt sind;
L für einen N-heterocyclischen Carbenliganden steht;
L^{1'} und L² gleich oder verschieden sind und jeweils für ein substituiertes oder unsubstituiertes Heteroaren stehen;
R für Wasserstoff steht und R¹ aus C₁-C₂₀-Alkyl, C₂-C₂₀-Alkenyl und Aryl ausgewählt ist.

29. Verfahren zur Metathese eines cyclischen oder acyclischen Olefins, bei dem man das Olefin mit einer Verbindung nach einem der vorhergehenden Ansprüche in Kontakt bringt.

30. Verfahren zur ringöffnenden metathetischen Polymerisation eines cyclischen Olefins, bei dem man das Olefin mit einer Verbindung nach Anspruch 28 in Kontakt bringt.

31. Verfahren zur Synthese eines sechsfach koordinierten Ruthenium- oder Osmium-Metathesekatalysators nach Anspruch 1, bei dem man einen fünffach koordinierten Ruthenium- oder Osmium-Metallcarben-Metathesekatalysator mit einem neutralen Elektronendonorliganden in Kontakt bringt.

32. Verfahren zur Synthese eines sechsfach koordinierten Ruthenium- oder Osmium-Metathesekatalysators nach Anspruch 1, bei dem man in einem Ligandenaustausch den N-heterocyclischen Carbenliganden durch einen anderen neutralen Donorliganden in einem entsprechenden sechsfach koordinierten Ruthenium- oder Osmium-Komplex ersetzt.

33. Verfahren zur Polymerisation eines Olefins, bei dem man eine Verbindung der Formel: 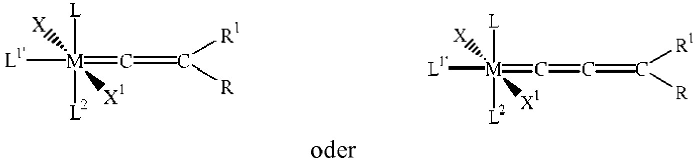 mit einer Verbindung der Formel: 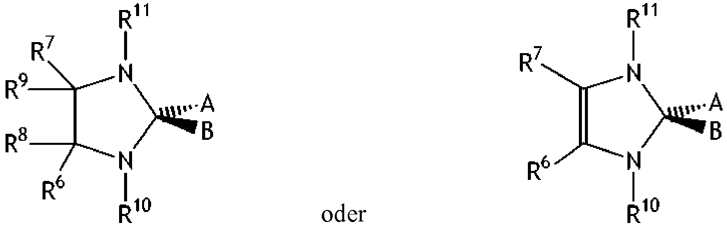 und einem cyclischen oder acyclischen Olefin in Kontakt bringt;
wobei
M für Ruthenium oder Osmium steht;
X und X¹ gleich oder verschieden sind und jeweils unabhängig voneinander für einen beliebigen anionischen Liganden stehen;
L, L^{1'} und L² gleich oder verschieden sind und jeweils unabhängig voneinander für einen beliebigen neutralen Elektronendonorliganden stehen;
R, R¹, R⁶, R⁷, R⁸, R⁹, R¹⁰ und R¹¹ gleich oder verschieden sind und jeweils unabhängig voneinander für Wasserstoff oder einen substituierten oder unsubstituierten Substituenten, der aus C₁-C₂₀-Alkyl, C₂-C₂₀-Alkenyl, C₂-C₂₀-Alkinyl, Aryl, C₁-C₂₀-Carboxylat, C₁-C₂₀-Alkoxy, C₂-C₂₀-Alkenyloxy, C₂-C₂₀-Alkinyloxy, Aryloxy, C₂-C₂₀-Alkoxycarbonyl, C₁-C₂₀-Alkylthio, C₁-C₂₀-Alkylsulfonyl und C₁-C₂₀-Alkylsulfinyl und Silyl ausgewählt ist, stehen; und
A für Wasserstoff, Si, Sn, Li, Na, MgX³ und Acyl steht, wobei X³ für ein beliebiges Halogen steht und
B aus CCl₃; CH₂SO₂Ph; C₆F₅, OR²¹ und N(R²²)(R²³) ausgewählt ist, wobei R²¹ aus Me, C₂H₅, i-C₃H₇, CH₂CMe₃, CMe₃, C₆H₁₁ (Cyclohexyl), CH₂Ph, CH₂-Norbornyl, CH₂-Norbornenyl, C₆H₅, 2,4,6-(CH₃)₃C₆H₂ (Mesityl), 2,6-i-Pr₂C₆H₂, 4-Me-C₆H₄ (Tolyl) oder 4-Cl-C₆H₄ ausgewählt ist; und wobei R²² und R²³ jeweils unabhängig voneinander aus Me, C₂H₅, i-C₃H₇, CH₂CMe₃, CMe₃, C₆H11 (Cyclohexyl), CH₂Ph, CH₂-Norbornyl, CH₂-Norbornenyl, C₆H₅, 2,4,6-(CH₃)₃C₆H₂ (Mesityl), 2,6-i-Pr₂C₆H₂, 4-Me-C₆H₄ (Tolyl) und 4-Cl-C₆H₄ ausgewählt sind.

34. Verfahren nach Anspruch 33, wobei
M für Ruthenium steht;
X und X¹ jeweils unabhängig voneinander aus Halogenid, CF₃CO₂, CH₃CO₂, CFH₂CO₂, (CH₃)₃CO, (CF₃)₂(CH₃)CO, (CF₃)(CH₃)₂CO, PhO, MeO, EtO, Tosylat, Mesylat oder Trifluormethansulfonat ausgewählt sind;
L für einen beliebigen neutralen Elektronendonorliganden steht;
L^{1'} und L² gleich oder verschieden sind und jeweils für ein substituiertes oder unsubstituiertes Heteroaren stehen;
R für Wasserstoff steht und R¹, R⁶, R⁷, R⁸, R⁹, R¹⁰ R¹¹ jeweils unabhängig voneinander aus Wasserstoff, C₁-C₂₀-Alkyl, C₂-C₂₀-Alkenyl und Aryl ausgewählt sind;
A für Wasserstoff steht und
B für CCl₃ steht.

35. Verfahren nach Anspruch 33, wobei es sich bei dem Olefin um ein substituiertes oder unsubstituiertes Norbornen oder ein norbornenartiges Monomer oder Derivat davon handelt.

36. Verfahren nach Anspruch 33, wobei es sich bei dem Olefin um substituiertes oder unsubstituiertes Dicyclopentadien handelt.

37. Verfahren nach Anspruch 33, wobei es sich bei dem Olefin um ein Gemisch aus einem oder mehreren substituierten oder unsubstituierten Norbornenen oder norbornenartigen Monomeren oder Derivaten davon handelt.

## Revendications

1. Composé de la formule 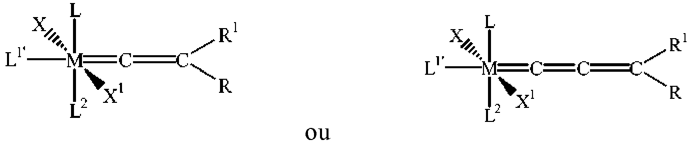 où
M est un ruthénium ou un osmium;
X et X¹ sont identiques ou différents et sont chacun indépendamment un ligand anionique quelconque;
L, L¹, et L² sont identiques ou différents et sont chacun indépendamment un ligand donneur d'électrons neutres quelconque, où au moins un est un Ligand carbène N-hétérocyclique;
R et R¹ sont identiques ou différents et sont chacun indépendamment un hydrogène ou un substituant substitué ou non substitué sélectionné parmi un alkyle C₁-C₂₀, alkényle C₂-C₂₀, alkynyle C₂-C₂₀, aryle, carboxylate C₁-C₂₀, alkoxy C₁-C₂₀, alkènyloxy C₂-C₂₀, alkynyloxy C₂-C₂₀, aryloxy, alkoxycarbonyle C₂-C₂₀, alkylthio C₁-C₂₀, alkylsulfonyle C₁-C₂₀, alkylsulfinyle C₁-C₂₀ et silyle.

2. Composé selon la revendication 1, où au moins un parmi le groupe de substituants R et R¹ est remplacé par un ou plusieurs groupements sélectionnés parmi alkyle C₁-C₁₀, alkoxy C₁-C₁₀ et aryle, et où le groupement est substitué ou non substitué.

3. Composé selon la revendication 2, où le groupement est remplacé par un ou plusieurs groupes sélectionnés parmi un halogène, un alkyle C₁-C₅, alkoxy C₁-C₅ et phényle.

4. Composé selon la revendication 1, où R est un hydrogène et R¹ est sélectionné parmi alkyle C₁-C₂₀, alkényle C₂-C₂₀ et aryle.

5. Composé selon la revendication 4, où R¹ est un phényle ou un vinyle.

6. Composé selon la revendication 1 où X et X¹ sont chacun indépendamment un hydrogèn, halogénure ou sélectionnés parmi un alkyle C₁-C₂₀, aryle, alkoxide C₁-C₂₀, aryloxide, alkyldikétonate C₃-C₂₀, aryldikétonate, carboxylate C₁-C₂₀, arylsulfonate, alkylsulfonate C₁-C₂₀, alkylthio C₁-C₂₀, alkylsulfonyle C₁-C₂₀ ou alkylsulfinyle C₁-C₂₀, où X et X¹ est chacun indépendamment substitué ou non substitué.

7. Composé selon la revendication 6, où au moins un de X et X¹ est remplacé par un ou plusieurs groupements sélectionnés parmi alkyle C₁-C₁₀, alkoxy C₁-C₁₀ et aryle, où le groupement est substitué ou non substitué.

8. Composé selon la revendication 7, où le groupement est remplacé par un ou plusieurs groupes sélectionnés parmi un halogène, alkyle C₁-C₅, alkoxy C₁-C₅ et phényle.

9. Composé selon la revendication 6, où X et X¹ sont chacun indépendamment sélectionnés parmi un halogénure, benzoate, carboxylate C₁-C₅, alkyle C₁-C₅, phénoxy, alkoxy C₁-C₅, alkylthio C₁-C₅, aryle et alkyle sulfonate C₁-C₅.

10. Composé selon la revendication 9, où X et X¹ sont chacun indépendamment sélectionnés parmi un halogénure, CF₃CO₂, CH₃CO₂, CFH₂CO₂, (CH₃)₃CO, (CF₃)₂(CH₃)CO, (CF₃)(CH₃)₂CO, PhO, MeO, EtO, tosylate, mésylate ou trifluorométhanesulfonate.

11. Composé selon la revendication 1, où, selon les exigences de la revendication 1, L, L^{1'} et L² sont chacun indépendamment sélectionnés parmi un ligand donneur d'électrons neutres monodentate, bidentate ou tétradentate.

12. Composé selon la revendication 11, où, selon les exigences de la revendication 1, L, L^{1'} et L² sont chacun indépendamment sélectionnés parmi phosphine, phosphine sulfonée, phosphite, phosphinite, phosphonite, arsine, stibine, éther, amine, amide, imine, sulfoxide, carboxyle, nitrosyle, pyridine et thioéther, et leurs dérivés et les ligands carbènes N-hétérocycliques.

13. Composé selon la revendication 1 , où both L¹ et L² sont des ligands carbènes N-hétérocycliques identiques ou différents.

14. Composé selon la revendication 1, où L est un ligand carbène N-hétérocyclique et L¹ et L² sont chacun des ligands hétérocycliques.

15. Composé selon la revendication 14, où au moins un de L^{1'} et L² est aromatique.

16. Composé selon la revendication 14, où L^{1'} et L² forment ensemble un ligand bidentate.

17. Composé selon la revendication 1, où le ligand carbène N-hétérocylcique est sélectionné parmi: 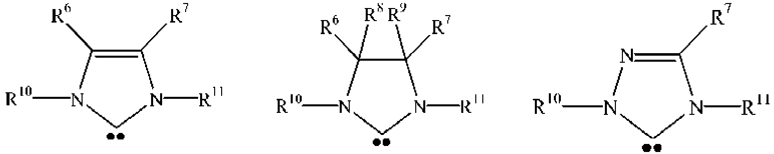 où R, R¹, R⁶, R⁷, R⁸, R⁹, R¹⁰ et R¹¹ sont chacun indépendamment un hydrogène ou un substituant substitué ou non substitué sélectionné parmi un alkyle C₁-C₂₀, alkényle C₂-C₂₀, alkynyle C₂-C₂₀, aryle, carboxylate C₁-C₂₀, alkoxy C₁-C₂₀, alkènyloxy C₂-C₂₀, alkynyloxy C₂-C₂₀, aryloxy, alkoxycarbonyle C₂-C₂₀, alkylthio C₁-C₂₀, alkylsulfonyle C₁-C₂₀, alkylsulfinyle C₁-C₂₀ et silyle.

18. Composé selon un composé quelconque de la revendication 1 où, selon les exigences de la revendication 1, au moins un de L^{1'} et L² est un hétéroarène non substitué ou substitué sélectionné parmi furane, thiophène, pyrrole, pyridine, bipyridine, picolylimine, gamma-pyrane, gamma-thiopyrane, phénanthroline, pyrimidine, bipyrimidine, pyrazine, indole, coumarone, thionaphthène, carbazole, dibenzofurane, dibenzothiophène, pyrazole, imidazole, benzimidazole, oxazole, thiazole, dithiazole, isoxazole, isothiazole, quinoline, bisquinoline, isoquinoline, bisisoquinoline, acridine, chromène, phénazine, phénoxazine, phénothiazine, triazine, thianthrène, purine, bisimidazole et bisoxazole.

19. Composé selon la revendication 18, où au moins un de L^{1'} et L² est une pyridine substitué ou non substituée ou bien un dérivé de pyridine substituée ou non substituée.

20. Composé selon la revendication 18, où L est un ligand imidazolidine et L^{1'} et L² sont tous deux une pyridine.

21. Composé selon la revendication 18, où l'hétéroarène substitué ou non substitué est sélectionné parmi: 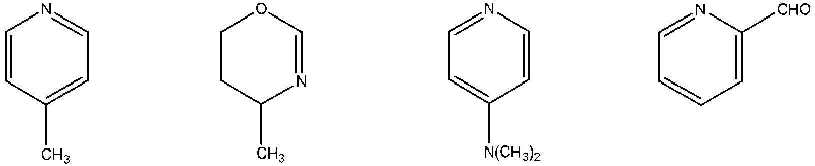 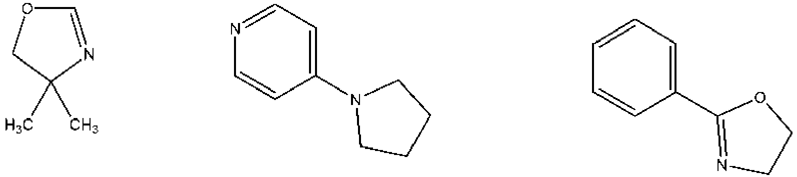 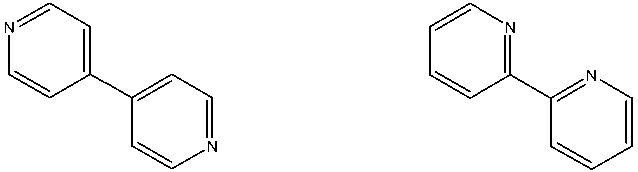 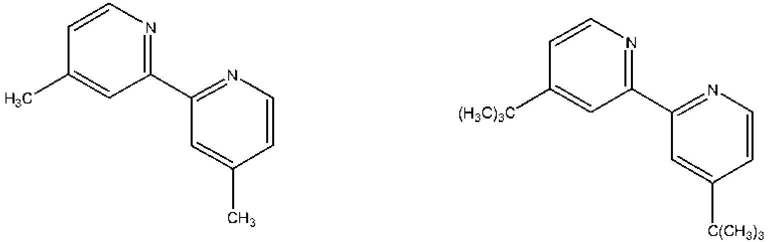 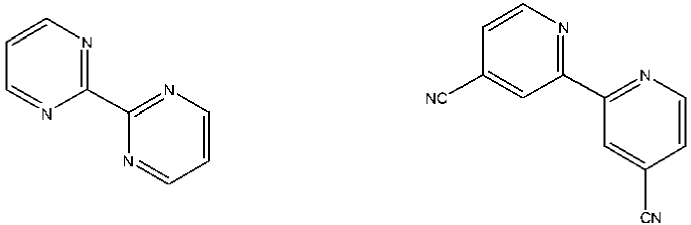 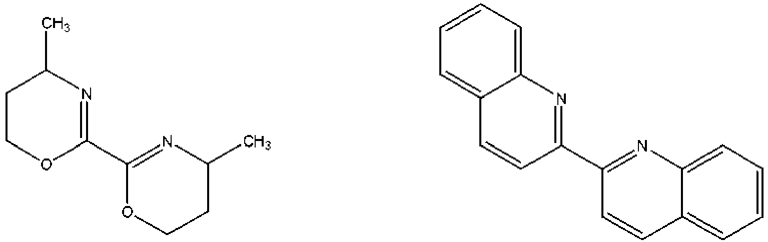 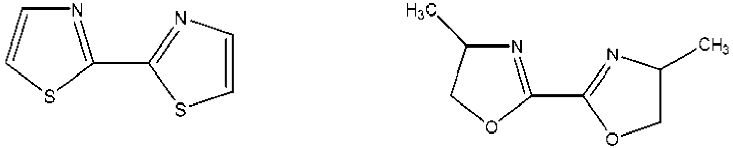 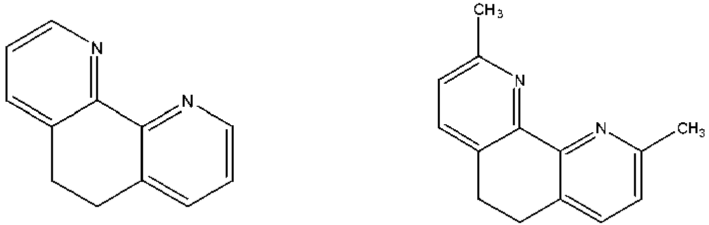 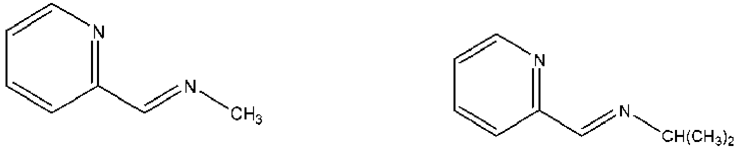 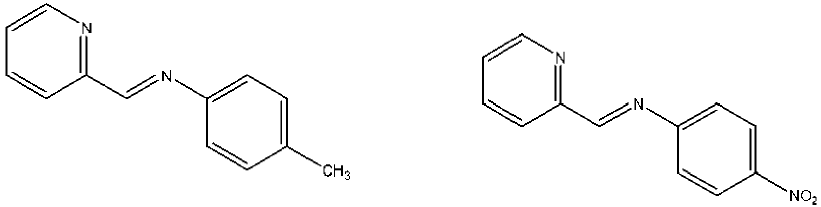 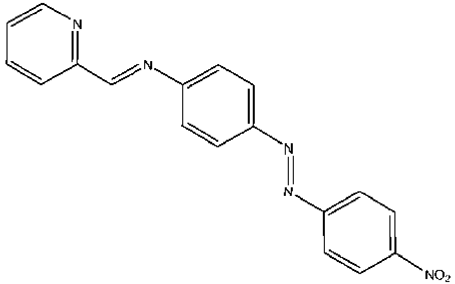 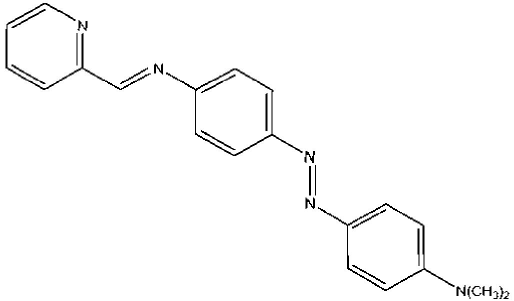 et

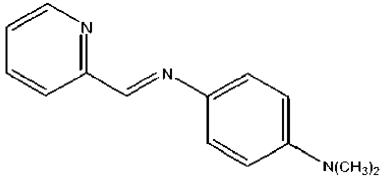

22. Composé selon l'un des composés quelconques de la revendication 1 selon les exigences de la revendications 1, au moins un de L^{1'} and L² est un hétérocycle non substitué ou substitué sélectionné parmi: 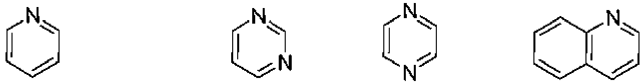 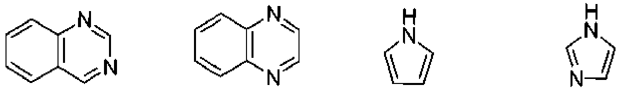 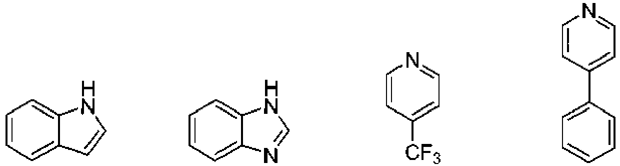 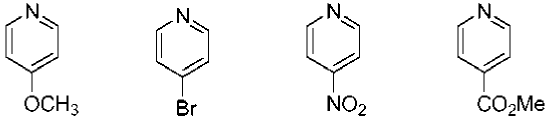 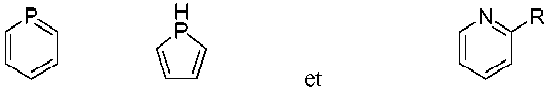 où R est sélectionné parmi un alkyle C₁-C₂₀, aryle, éther, amide, halide, nitro, ester, pyridyle.

23. Composé selon la revendication 1, où
M est un ruthénium;
X et X¹ sont chacun indépendamment sélectionnés parmi un halogénure, CF₃CO₂, CH₃CO₂, CFH₂CO₂, (CH₃)₃CO, (CF₃)₂(CH₃)CO, (CF₃)(CH₃)₂CO, PhO, MeO, EtO, tosylate, mesylate ou un trifluoroméethanesulfonate;
L est un ligand carbène N-hétérocyclique;
L^{1'} et L² sont identiques ou différents et sont chacun un hétéroarène substitué ou non substitué et où L^{1'} et L^{1'} peuvent être joints.
R est un hydrogène et R¹ est sélectionné parmi alkyle C₁-C₂₀, alkényle C₂-C₂₀ et aryle.

24. Composé selon la revendication 23, où X et X¹ sont chacun Cl, L est (IMesH₂), L^{1'} et L² sont chacun indépendamment une pyridine ou un dérivé de pyridine; R est un hydrogène et R¹ est un phényle ou vinyle.

25. Composé selon la revendication 1 sélectionné parmi: 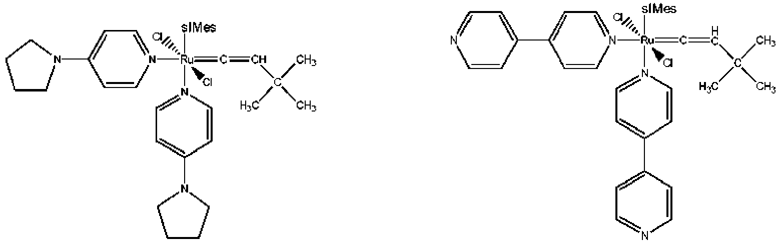 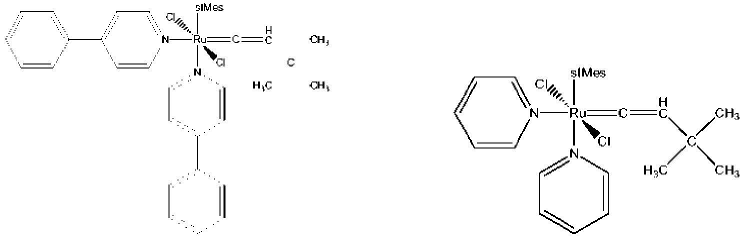 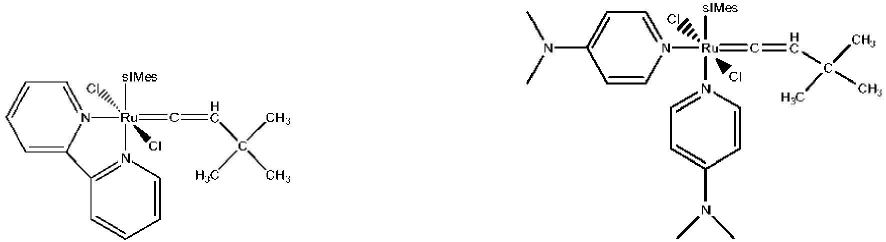 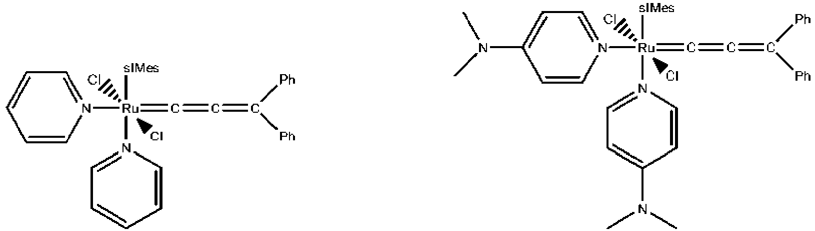 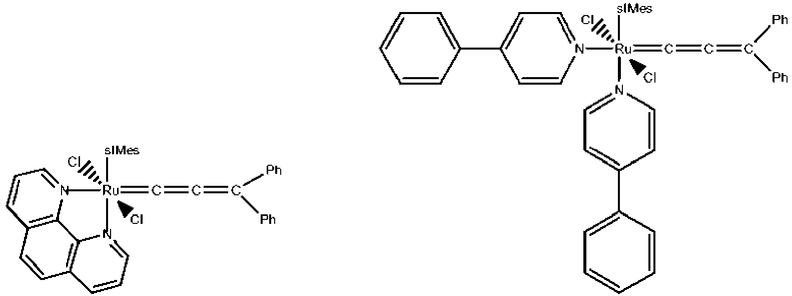 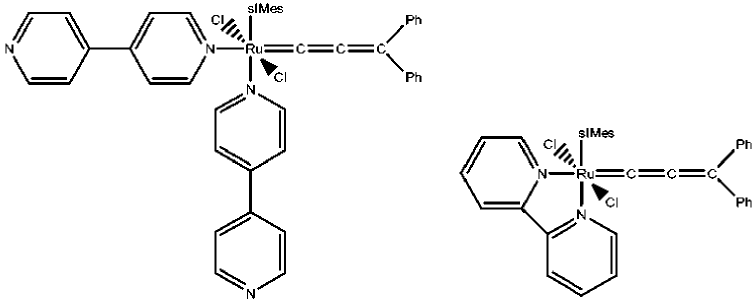 où sIMES ou IMesH₂ est

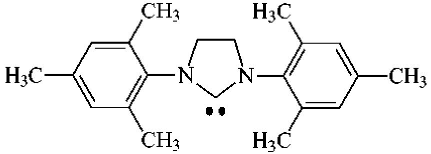

26. Composé selon la revendication 1, où
M est un ruthénium;
X et X¹ sont chacun indépendamment sélectionnés parmi un halogénure, CF₃CO₂, CH₃CO₂, CFH₂CO₂, (CH₃)₃CO, (CF₃)₂(CH₃)CO, (CF₃)(CH₃)₂CO, PhO, MeO, EtO, tosylate, mésylate or trifluorométhanesulfonate;
L est un ligand carbène N-hétérocyclique;
L^{1'} et L² sont identiques ou différents et sont chacun un hétéroarène substitué ou non substitué;
R est un hydrogène et R¹ est sélectionné parmi alkyle C₁-C₂₀ alkényle C₂-C₂₀ et aryle.

27. Composé selon la revendication 26, où X et X¹ sont chacun Cl, L est (IMesH₂), L^{1'} et L² sont chacun indépendamment une pyridine ou dérivé de pyridine; R est un hydrogène et R¹ est un phényle ou vinyle.

28. Composé selon la revendication 1, où
X et X¹ sont chacun indépendamment sélectionnés parmi un halogénure, CF₃CO₂, CH₃CO₂ CFH₂CO₂, (CH₃)₃CO, (CF₃)₂(CH₃)CO, (CF₃)(CH₃)₂CO, PhO, MeO, EtO, tosylate, mésylate or trifluorométhanesulfonate;
L est un ligand carbène N-hétérocyclique;
L^{1'} et L² sont identiques ou différents et sont chacun un hétéroarène substitué ou non substitué;
R est un hydrogène et R¹ est sélectionné parmi alkyle C₁-C₂₀, alkényle C₂-C₂₀ et aryle.

29. Procédé de métathèse d'une oléfine cyclique ou acyclique comprenant la mise en contact de l'oléfine avec un composé tel que défini dans l'une des revendications précédentes.

30. Procédé de polymérisation par métathèse à ouverture d'anneau d'une oléfine cyclique comprenant la mise en contact de l'oléfine avec un composé tel que défini dans la revendication 28.

31. Procédé de synthèse d'un catalyseur de métathèse hexacoordonné par ruthénium ou osmium selon la revendication 1 comprenant la mise en contact d'un catalyseur de métathèse de carbène de métal pentacoordonné par ruthénium ou osmium avec un ligand donneur d'électrons neutre.

32. Procédé de synthèse d'un catalyseur de métathèse hexacoordonné par ruthénium ou osmium selon la revendication 1, comprenant un échange de ligand dans lequel le ligand carbène N-hétérocyclique remplace un autre ligand donneur d'électrons neutre dans un complexe hexacoordonné par ruthénium ou osmium correspondant.

33. Procédé de polymérisation d'une oléfine comprenant la mise en contact avec un composé de la formule: 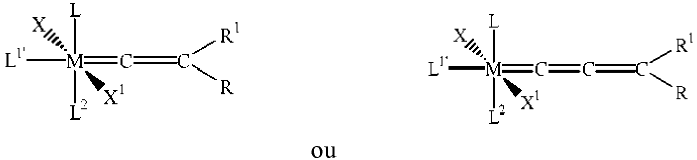 avec un compose de la formule: 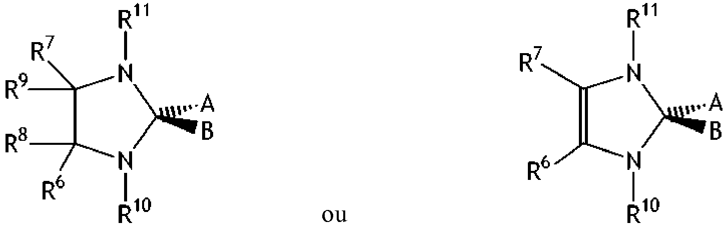 et une oléfine cyclique ou acyclique;
où
M est un ruthénium ou un osmium;
X et X¹ sont identiques ou différents et sont chacun indépendamment un ligand anionique quelconque; L, L^{1'}, et L² sont identiques ou différents et sont chacun indépendamment un ligand donneur d'électrons neutres quelconque;
R, R¹, R⁶, R⁷, R⁸, R⁹, R¹⁰ et R¹¹ sont identiques ou différents et sont chacun indépendamment un hydrogène ou un substituant substitué ou non substitué sélectionné parmi un alkyle C₁-C₂₀, alkényle C₂-C₂₀, alkynyle C₂-C₂₀, aryle, carboxylate C₁-C₂₀, alkoxy C₁-C₂₀, alkènyloxy C₂-C₂₀, alkynyloxy C₂-C₂₀, aryloxy, alkoxycarbonyle C₂-C₂₀, alkylthio C₁-C₂₀, alkylsulfonyle C₁-C₂₀ et alkylsulfinyle C₁-C₂₀ et silyle; et
A est un hydrogène, Si, Sn, Li, Na, MgX³ et acyl, où X³ est n'importe quel halogène et B est sélectionné parmi CCl₃; CH₂SO₂Ph; C₆F₅; OR²¹; et N(R²²)(R²³), où R²¹ est sélectionné parmi Me, C₂H₅, i-C₃H₇, CH₂CMe₃, CMe₃, C₆H₁₁ (cyclohexyle), CH₂Ph, CH₂norbomyle, CH₂norboményle, C₆H₅, 2,4,6-(CH₃)₃C₆H₂ (mésityle), 2,6-i-Pr₂C₆H₂, 4-Me- C₆H₄ (tolyle), 4-Cl-C₆H₄; et où R²² et R²³ sont chacun indépendamment sélectionnés parmi Me, C₂H₅, i-C₃H₇, CH₂CMe₃, CMe₃, C₆H₁₁ (cyclohexyle), CH₂Ph, CH₂norbomyle, CH₂norboményle, C₆H₅, 2,4,6-(CH₃)₃C₆H₂ (mésityle), 2,6-i-Pr₂C₆H₂, 4-Me-C₆H₄ (tolyle), et 4-Cl-C₆H₄.

34. Procédé selon revendication 33, où
M est un ruthénium;
X et X¹ sont chacun indépendamment sélectionnés parmi un halogénure, CF₃CO₂, CH₃CO₂, CFH₂CO₂, (CH₃)₃CO, (CF₃)₂(CH₃)CO, (CF₃)(CH₃)₂CO, PhO, MeO, EtO, tosylate, mésylate, ou trifluorométhanesulfonate;
L est un ligand donneur d'électrons neutres quelconque;
L^{1'} et L² sont identiques ou différents et sont chacun un hétéroarène substitué ou non substitué;
R est un hydrogène et R¹, R⁶, R⁷, R⁸, R⁹, R¹⁰, R¹¹ sont chacun indépendamment sélectionnés parmi un hydrogène, alkyle C₁-C₂₀, alkényle C₂-C₂₀ et aryle;
A est un hydrogène et
B est CCl₃.

35. Procédé selon revendication 33 où l'oléfine est un norbornène ou un monomère de type norbornè substitué ou non substitué ou un dérivé de ceux-ci.

36. Procédé selon revendication 33, où l'oléfine est un dicyclopentadiène substitué ou non substitué.

37. Procédé selon revendication 33, où l'oléfine est un mélange d'un ou plusieurs norbornènes ou monomères de type norbornène substitués ou non substitués ou dérivés de ceux-ci.
